(19) Europäisches Patentamt
European Patent Office
Office européen des brevets

(11) Numéro de publication: **0 072 756**
**B1**

(12) # FASCICULE DE BREVET EUROPEEN

(45) Date de publication du fascicule du brevet: **23.10.85**

(51) Int. Cl.⁴: **C 07 D 501/24**, C 07 D 498/04

(21) Numéro de dépôt: **82401532.5**

(22) Date de dépôt: **13.08.82**

(54) **Nouveaux dérivés de la céphalosporine et leur préparation.**

(30) Priorité: **17.08.81 FR 8115805**

(43) Date de publication de la demande: **23.02.83 Bulletin 83/8**

(45) Mention de la délivrance du brevet: **23.10.85 Bulletin 85/43**

(84) Etats contractants désignés: **AT BE CH DE FR GB IT LI LU NL SE**

(56) Documents cités:
**FR - A - 2 457 297**
**FR - A - 2 460 302**

**Le dossier contient des informations techniques présentées postérieurement au dépôt de la demande et ne figurant pas dans le présent fascicule.**

(73) Titulaire: **RHONE-POULENC SANTE, Les Miroirs 18 Avenue d'Alsace, F-92400 Courbevoie Cedex (FR)**

(72) Inventeur: **Farge, Daniel, 30 rue des Pins Sylvestres, F-94320 Thiais (FR)**
Inventeur: **Le Roy, Pierre, 2 allée des Cerisiers, F-94320 Thiais (FR)**
Inventeur: **Moutonnier, Claude, 3 rue Auguste Rodin, F-92350 Le Plessis-Robinson (FR)**
Inventeur: **Peyronel, Jean-François, 36 parc d'Ardenay, F-91120 Palaiseau (FR)**
Inventeur: **Plau, Bernard, 53 rue de Mesly, F-94000 Créteil (FR)**

(74) Mandataire: **Gaumont, Robert et al, RHONE-POULENC RECHERCHES Service Brevets Pharma 25, Quai Paul Doumer, F-92408 Courbevoie Cedex (FR)**

**EP 0 072 756 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention concerne de nouveaux dérivés de céphalosporine de formule générale:

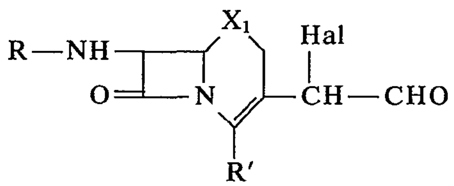

(I)

leurs formes isomères et leurs mélanges, et leur préparation.

Dans la formule générale (I),

– le symbole R′ représente un radical carboxy protégé
– le symbole Hal représente un atome d'halogène tel que le chlore, le brome et l'iode, et

A) le symbole $X_1$ représente un atome de soufre ou d'oxygène ou un radical sulfinyle et le symbole R représente un radical de formule générale:

$$R_1—\underset{\underset{\displaystyle R_2}{|}}{CH}—CO— \qquad (IIa)$$

[dans laquelle $R_1$ est un radical hétérocyclyle tel que thiényle, furyle ou dithiol-1,3 one-2 yle-4, ou un radical phényle, p.hydroxyphényle libre ou protégé ou phénoxy et $R_2$ est un atome d'hydrogène, ou $R_1$ est un radical phényle ou p.hydroxyphényle libre ou protégé et $R_2$ est un radical amino protégé] ou
un radical protecteur d'amino, facilement éliminable, ou bien

B) le symbole $X_1$ représente un atome de soufre et le symbole R représente un radical de formule générale:

$$R''CO— \qquad (IIb_1)$$

[dans laquelle R″ est choisi parmi les radicaux alcoyle contenant 1 à 7 atomes de carbone, cycloalcoyle, aryle, aralcoyle, hétéroaryle ou hétéroaralcoyle, qui peuvent être substitués par un ou plusieurs atomes d'halogènes ou radicaux alcoyle, alcoyloxy, alcoylthio, haloalcoyle, azido, cyano, amino protégé éventuellement substitué, carboxy protégé ou carboxyalcoyle protégé], ou un radical de formule générale:

$$R''—Y—(CH_2)_m—CO— \qquad (IIb_2)$$

[dans laquelle m est un nombre entier de 0 à 4, Y est un atome d'oxygène ou de soufre et R″ est défini comme ci-dessus], ou un radical de formule générale:

$$R''—\underset{\underset{\displaystyle R'''}{|}}{CH}—CO— \qquad (II\ b_3$$

[dans laquelle R″ est défini comme ci-dessus et R‴ est un atome d'halogène ou un radical hydroxy, hydroxy protégé, azido, acyloxy, amino protégé ou carboxy protégé], ou
R — NH représente un groupe sulfonamido.

Il est entendu que les radicaux et portions alcoyle et acyle cités ci-dessus ou ci-après sont droits ou ramifiés et contiennent (sauf mention spéciale) 1 à 4 atomes de carbone.

Les produits de formule générale (I), qui contiennent tous un radical — CH (Hal) — CHO en position-3, présentent des formes isomères; il est entendu que les épimères et leurs mélanges entrent dans le cadre de la présente invention.

Lorsque $R_2$ est un radical amino, ou lorsque R est un radical de formule générale (II $b_3$), le substituant en position-7 des produits de formule générale (I) existe sous les formes D et L; il est également entendu que les isomères qui en résultent ainsi que leurs mélanges entrent dans le cadre de la présente invention.

Le radical protecteur d'acide contenu par R′ est un radical facilement éliminable, sans altération du reste de la molécule. A titre d'exemple, R′ peut être protégé par un radical t.butyle, méthoxyméthyle, trichloro-2,2,2 éthyle, benzhydryle, p.nitrobenzyle ou p.méthoxybenzyle.

Lorsque $R_1$ contient un radical hydroxy protégé, ce dernier peut être protégé par tout groupement compatible avec la préparation et l'utilisation des produits de formule générale (I), par exemple trityle, tétrahydropyrannyle, alcoyloxycarbonyle (t.butoxycarbonyle), aryloxycarbonyle (benzyloxycarbonyle) ou p.méthoxybenzyle.

Lorsque le symbole $R_2$ représente un radical amino protégé, il peut être protégé par exemple par un groupement t.butoxycarbonyle, trichloro-2,2,2 éthoxcarbonyle, trityle, benzyle, benzyloxycarbonyle, formyle, trifluoracétyle, p.nitrobenzyloxycarbonyle, p.méthoxybenzyloxycarbonyle ou encore diphénylphosphinoyle, ou un radical tel que défini ci-après par la formule générale (III $a_8$) qui peuvent être introduits par application de la méthode décrite par A. Morimoto et coll., J. Chem. Soc. Perkin I 1109 (1980).

Lorsque le symbole R tel que défini en A) représente un radical protecteur facilement éliminable, il peut être par exemple choisi parmi:

1) benzhydryle ou trityle,
2) un radical acyle de formule générale:

$R_3CO—$ (III$a_1$)

dans laquelle $R_3$ représente

a) un atome d'hydrogène ou un radical alcoyle contenant 1 à 7 atomes de carbone ou méthyle substitué par 1 à 3 atomes d'halogène,
b) un radical phényle (pouvant être jusqu'à 3 fois substitué par des atomes d'halogène ou des radicaux hydroxy, nitro, cyano, trifluorométhyle, alcoyle ou alcoyloxy) ou un radical thiényle-2 ou -3,
c) un radical de formule générale:

$R_3'—Y—CH_2—$ (III$a_2$)

dans laquelle $R_3'$ est un radical phényle (pouvant être substitué par un atome d'halogène ou par un radical alcoyle, alcoyloxy ou hydroxy) et Y est un atome d'oxygène ou de soufre,

d) un radical arylalcoyle de formule générale:

$R_3''CH_2—$ (III$a_3$)

dans laquelle $R_3''$ est un radical phényle pouvant être jusqu'à 3 fois substitué par des radicaux hydroxy, alcoyle ou alcoyloxy, ou un radical hétérocyclyle tel que thiényle-2 ou -3 ou furyle-2 ou -3,

3) un radical amino-5 adipoyle dont les fonctions amine et acide sont protégées par des radicaux protecteurs tels que définis précédemment,
4) un radical de formule générale:

$R_4OCO—$ (III$a_4$)

dans laquelle $R_4$ est un radical alcoyle ramifié non substitué, un radical alcoyle droit ou ramifié portant un ou plusieurs substituants [tels que des atomes d'halogène ou des radicaux cyano, trialcoylsilyle, phényle ou phényle substitué (par un ou plusieurs atomes d'halogène ou radicaux alcoyle, alcoyloxy, nitro ou phényle)] ou un radical quinolyle,

5) un radical de formule générale:

$$Ar—\underset{(O)_n}{\underset{|}{S}}—$$ (III$a_5$) ou $ArSe—$ (III$a_6$)

dans lesquelle le reste Ar est un radical phényle éventuellement substitué par un ou plusieurs atomes d'halogène ou radicaux alcoyle ou nitro et n est égal à 0 ou 1,

6) ou bien R NH — peut être remplacé par un radical dialcoylaminométhylèneamino ou par un radical de formule générale:

$Ar'—CH{=}N—$ (III$a_7$)

dans laquelle Ar' est un radical phényle éventuellement substitué par un ou plusieurs radicaux tels que alcoyle, alcoyloxy, hydroxy ou nitro,

7) ou encore R est un radical diphénylphosphinoyle ou un radical de formule générale:

$$(ZO)_2\underset{O}{\underset{\downarrow}{P}}—$$ (III$a_8$)

dans laquelle Z est alcoyle, trichloro-2,2,2 éthyle, phényle ou benzyle, ces deux derniers étant éventuellement substitués par un atome d'halogène ou par un radical alcoyle, alcoyloxy ou nitro, ou les symboles Z forment ensemble un radical alcoylène contenant 2 ou 3 atomes de carbone.

Comme exemples de radicaux protecteurs R pouvent être utilisés, on peut citer les radicaux suivants:

formyle, acétyle, trichloracétyle, phenylacétyle,
phénoxyacétyle, benzoyle,
t.butoxycarbonyle,
chloro-2 diméthyl-1,1 éthoxycarbonyle,
trichloro-2,2,2 éthoxycarbonyle,
trichloro-2,2,2 diméthyl-1,1 éthoxycarbonyle,
cyano-2 diméthyl-1,1 éthoxycarbonyle,
triméthylsilyl-2 éthoxycarbonyle,
benzyloxycarbonyle,
p.méthoxybenzyloxycarbonyle,
diméthoxy-3,5 benzyloxycarbonyle,
p.nitrobenzyloxycarbonyle,
diphénylméthoxycarbonyle,
(biphényl-4)-2 isopropyloxycarbonyle,
quinolyl-8 oxycarbonyle,
o.nitrophénylthio,
p.nitropénylthio,
diméthoxyphosphoryle,
diéthoxyphosphoryle,
diphénoxyphosphoryle,
dibenzyloxyphosphoryle.

Comme exemples de radicaux méthyléneamino définis précédemment en 6), on peut citer:

diméthylaminométhylèneamino,
diméthoxy-3,4 benzylidèneamino,
nitro-4 benzylidèneamino.

A titre d'exemple, le symbole R″ défini précédement peut représenter notamment: benzyle, p.hydroxybenzyle, amino-4 carboxy-4 butyle dont les fonctions amine et acide sont protégées, méthyle, éthyle, n.amyle, heptyle, nitro-3 (ou -4) benzyle, phénéthyle, méthoxy-2phényle, diméthoxy-2,6 phényle, p. (ou o.) aminobenzyle, protégés, p.méthoxybenzyle, naphtyl-1 méthyle, isothiazolyl-3 méthyle, isothiazolyl-4 (ou -5) méthyle, pyridyl-4 méthyle, isoxazolyl-5 méthyle, benzofuranylméthyle, indolyl-2 méthyle, (méthyl-3 imidazolyl)-1 méthyle, (méthyl-5 thiényl)-2 (ou -3) méthyle, (méthoxy-5 thiényl)-2 (ou -3) méthyle, (chloro-4 thiényl)-2 (ou -3) méthyle, (thiadiazol-1,2,5 yl-3) méthyle, (méthoxy-4 thiadiazol-1,2,5 yl-3) méthyle ou tétrazolylméthyle.

Lorsque R″ contient un radical amino ou carboxy protégés, ces derniers peuvent être protégés par tout groupement protecteur compatible avec les réactions mises en oeuvre pour la préparation et l'utilisation des produits et notamment: lorsque R″ contient un radical amino, par un groupement protecteur défini pour $R_2$ et lorsque R″ contient un radical carboxy, par un groupement tel que défini précédemment pour R′.

Lorsque le symbole R représente un radical de formule générale ($II\,b_2$), à titre d'exemple il peut représenter phénoxyacétyle, dichloro-3,4 phénylthioacétyle, pyridyl-4 thioacétyle, thiazolyl-2 thioacétyle ou pyrimidinyl-2 thioacétyle.

Lorsque le symbole R représente un radical de formule générale ($II\,b_3$), à titre d'exemple le groupement —CR″R″ peut représenter $\alpha$-hydroxybenzyle, $\alpha$-amino chloro-3 hydroxy-4 benzyle ou $\alpha$-amino thiényl-3 méthyle.

Lorsque R NH— représente un groupe sulfonamido, il peut être à titre d'exemple éthylsulfonamido, benzylsulfonamido, chloro-4 phénylsulfonamido ou méthoxy-4 phénylsulfonamido.

Selon l'invention, les dérivés de la céphalosporine de formule générale (I) pour lesquels $X_1$ est autre que le groupe sulfinyle peuvent être préparés par action d'un agent d'halogénation sur une énamine de formule générale:

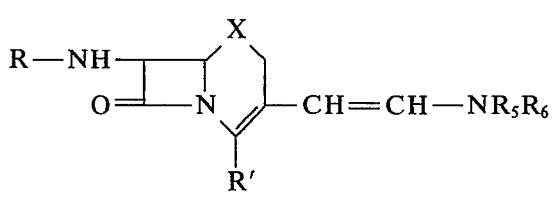

(IV)

[dans laquelle R et R' sont définis comme précédemment pour la formule générale (I), $R_5$ et $R_6$, identiques ou différents, représentent des radicaux alcoyle (éventuellement substitués par un radical alcoyloxy ou dialcoylamino) ou phényle, ou forment ensemble avec l'atome d'azote auquel ils rattachés, un hétérocycle à 5 ou 6 chaînons contenant éventuellement un autre hétéroatome choisi parmi l'azote, l'oxygène ou le soufre et éventuellement substitué par un radical alcoyle et X est un atome d'oxygène ou de soufre], suivie de l'hydrolyse du produit formé.

Par exemple on met en oeuvre une énamie de formule générale (IV) dans laquelle $R_5$ et $R_6$ sont chacun un radical méthyle.

Parmi les agents d'halogénation peuvent être cités: les halogènes, les N-haloamides [par exemple N-bromo (ou N-chloro) succinimide, N-bromo (ou N-chloro) acétamide, dibromohydantoïne], les hypohalogénites d'alcoyle (par exemple hypochlorite d'éthyle ou de t.butyle, hypobromite de t.butyle).

On opère généralement l'halogénation dans un solvant organique tel qu'un éther (par exemple tétrahydrofuranne, dioxanne), un solvant chloré (par exemple chlorure de méthylène, chloroforme) un ester (par exemple acétate d'éthyle), un alcool (par exemple méthanol, éthanol), un amide (par exemple diméthylformamide, diméthylacétamide), un nitrile (par exemple acétonitrile) ou une cétone (par exemple acétone), ou dans un mélange de tels solvants, à une température comprise entre −70 et 0°C.

L'hydrolyse s'effectue à une température comprise entre −70 et 20°C.

Selon l'invention, les produits de formule générale (I) dans laquelle $X_1$ est un radical sulfinyle, peuvent être obtenus par oxydation d'un produit de formule générale (I) dans laquelle $X_1$ est un atome de soufre.

On opère notamment dans les conditions décrites dans la demande de brevet allemand 2 637 176, notamment en présence d'acide, m.chloroperbenzoïque dans un solvant organique tel que le dichlorométhane.

Les énamines de formule générale (IV) peuvent être préparées par application de la méthode décrite dans la brevet belge 883 416, à partir de dérivés de céphalosporines de formule générale:

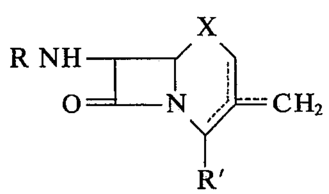

(V)

dans laquelle X, R et R' ont une définition correspondante.

Les dérivés de céphalosporine de formule générale (CV) dans laquelle X est un atome de soufre peuvent être obtenus comme décrit dans le brevet belge 883 416 ou, lorsque R est un radical facilement éliminable tel que défini précédemment en 7), par application de la méthode décrite par A. Morimoto et coll., J.C.S. Perkin I, 1109 (1980), à partir de l'halogénure correspondant R—Hal [qui peut être lui-même obtenu selon l'une des méthod es décrites par K. Sasse, Methoden der Organischen Chemie, Vol. 12, part. 2, page 274, Houben Weyl, Georg Thieme Verlag, Stuttgart (1964)].

Les oxacéphalosporines de formule générale (V) peuvent être obtenues selon les méthodes décrites dans la littérature, par exemple:

- dans les brevets belges 863 998 et 848 288,
- dans le brevet américain 4 108 992 ou
- par Y. Hamashima et coll., Tet. Let. 4943 (1979), et
- par C. L. Branch et coll., J. C. S. Perkin I, 2268 (1979)
- ou à partir de l'oxacéphalosporine de formule

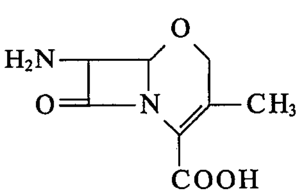

(VI)

par analogie avec les méthodes employées en chimie des céphalosporines, et décrites par exemple par

S. Seki et coll., tet. Lett., 33, 2915 (1977),
R. R. Chauvette et coll., J. Org. Chem., 38 (17), 2994 (1973),
J. C. Sheehan et coll., J. Amer. Chem. Soc. 80, 1156 (1958),
E. H. Flynn, Cephalosporins and Penicillins, Ac. Press (1972),
L. Moroder et coll., Hoppe Seyler's Z. Physiol. Chem. 357 1651 (1976),
J. Ugi et coll., Angew. Chem. Int. Ed. Engl. 17 (5), 361 (1978),

L. Zervas et coll., J. Amer. Chem. Soc. 85, 3660 (1963),
J. F. Fitt, J. Org. Chem., 42 (15), 2639 (1977),
A. Morimoto et coll., J. Chem. Soc. Perkin I, 1109 (1980),
dans le brevet belge 788 885 ou dans Helv. Chim. Acta, 51, 924 (1968),

- lorsque R représente diphénylméthoxycarbonyle: par action de l'azidoformiate correspondant en milieu hydroorganique, en présence d'un bicarbonate alcalin,
- lorsque R représente quinolyl-8 oxycarbonyle: par action du carbonate correspondant en milieu hydroorganique basique,
- lorsque R—NH— est remplacé par nitro-4 benzylidèneamino ou diméthoxy-3,4 benzylidèneamino: selon la méthode décrite par. R.A. Firestone, Tetrahedron Lett., 375 (1972).

Les dérivés de la céphalosporine selon l'invention sont utiles comme intermédiaires pour la préparation de thiazolyl-3 céphalosporines de formule générale:

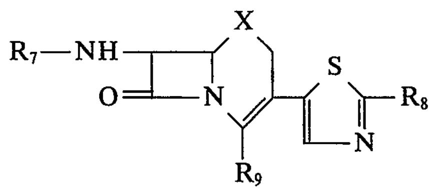

(VII)

dans laquelle

$A_1$) le symbole X représente un atome d'oxygène ou de soufre, le symbole $R_7$ représente un radical de formule générale (IIa) dans laquelle $R_1$ et $R_2$ sont définis comme précédemment à l'exception de contenir un groupement hydroxy ou amino protégé.
le symbole $R_8$ représente un atome d'hydrogène, un radical phényle (éventuellement substitué par un radical acylamino), alcoylthio, alcoylamino, dialcoylamino, anilino, acylamino, benzoylamino ou thénoylamino (ces 3 derniers radicaux étant éventuellement substitués sur l'atome d'azote par un radical alcoyle ou phényle), un radical alcoyloxycarbonylamino, dialcoylaminoéthylamino, dialcoylaminoéthylèneamino, ou alcoylidènehydrazo, un radical acétamido substitué par un radical amino, amino-2 éthylthio ou L amino-2 carboxy-2 éthylthio, ou un radical de structure $-AR'_8$ dans lequel A représente un radical bivalent choisi parmi $-CH_2-$, —NH— ou —NHCO— et $R'_8$ représente un radical méthyl-1 pyridinio-3 (ou -4), benzoylméthyl-1 pyridino-3 (ou -4) ou carboxyméthyl-1 pyridino-3 (ou -4),
le symbole $R_9$ représente un radical carboxy ou représente un radical carboxylato lorsque $R'_8$ est un radical pyridinio substitué,
ou bien

$B_1$) le symbole X représente un atome de soufre, le symbole $R_7$ prend l'une des significations données précédemment pour R en B), étant etendu que les groupements amino, carboxy or hydroxy que contiennent certains radicaux sont à l'état libre, le symbole $R_8$ représente un atome d'hydrogène ou un radical alcoyle, alcoyloxycarbonyl, acylamino ou dialcoylamino, et le symbole $R_9$ représente un radical carboxy.

Les produits de formule générale (VII) peuvent être obtenus à partir des produits de formule générale (I) en opérant comme suit:

I/ Lorsque $R_8$ est autre qu'un radical $-AR'_8$,

1° — on fait agir un produit de formule générale:

$$R_8—CS—NH_2 \qquad \text{(VIII)}$$

dans alquelle $R_8$ est défini comme ci-dessus, étant entendu que lorsqu'il contient un radical amino, ce dernier est protégé, sur un dérivé de la céphalosporine de formule générale (I) [dans laquelle R représente soit un radical de formule générale (II), soit prend une des significations données précédemment en B), et $X_1$, Hal et R' sont définis comme précédemment dans la formule générale (I)], puis éventuellement on fait agir un agent de déshydratation, le cas échéant, on réduit le sulfoxyde obtenu, et on élimine les groupements protecteurs.

On opère généralement en milieu organique ou hydroorganique, par exemple dans des solvants (ou des mélanges de solvants) tels que des alcools (méthanol, éthanol), des éthers (tétrahydrofuranne, dioxanne), des cétones (acétone), des nitriles (acétonitrile), des amides secondaires (diméthylformamide, diméthylacétamide), des esters (acétate d'éthyle) ou des acides (acide acétique, acide formique), en présence ou non d'une base (soude, potasse, carbonates, carbonates acides de métaux

alcalins, sels d'acides carboxyliques et de métaux alcalins, amines tertiaires), à une température comprise entre —50°C et la témperature de reflux du mélange réactionnel.

Il est parfois préférable d'introduire un agent de déshydratation. C'est le cas notamment lorsque $R_8$ représente un radical phènyle ou phényle substitué.

Parmi les agents de déshydratation utilisables, on peut citer: les halogénures d'acides sulfoniques [par exemple chlorure de tosyle, chlorure de méthanesulfonyle ou un halogénure du type R $SO_2Cl$ dans lequel R est alcoyle, trifluoro (ou trichloro) méthyle ou phényle éventuellement substitué par halogène ou nitro], les halogénures de phosphoryle (par exemple oxychlorure de phosphore) ou le chlorure de sulfonyle, soit dans un solvent basique [pyridine, amide (par exemple diméthylformamide, diméthylacétamide, hexaméthylphosphortriamide)] soit dans un solvent chlore (par exemple chloroforme, chlorure de méthylène), un éther (par exemple tétrahydrofuranne), un ester, une cétone, un nitrile, ou un solvant aromatique, en présence d'une amine tertiaire (par exemple pyridine, quinoléine, triéthylamine).

La réduction du sulfoxyde s'effectue, le cas échéant, selon les méthodes décrites dans la demande de brevet allemand 2 637 176.

L'élimination des groupements protecteurs d'acide peut s'effecteur par exemple:

- lorsqu'il s'agit d'un groupement t.butyle, p.méthoxybenzyle ou benzhydryle: par traitement en milieu acide, dans les conditions décrites ci-après pour l'élimination du radical trityle protecteur d'amino. Dans le cas du radical benzhydryle, on peut opérer en présence d'anisole ou par traitement par le chlorure d'aluminium dans les conditions décrites par T. Tsuji et coll., Tet. Lett., 30, 2793 (1979);
- lorsqu'il s'agit d'un groupement méthoxyméthyle: par traitement en milieu acide dilué:
- lorsqu'il s'agit d'un groupement trichloro-2,2,2 éthyle ou p.nitrobenzyle: par réduction (notamment traitement par le zinc dans l'acide acétique, ou lorsqu'il s'agit du groupement p.nitrobenzyle par hydrogénolyse).

L'élimination des groupements protecteurs d'amine peut s'effectuer par exemple:

- lorsqu'il s'agit d'un radical t.butoxycarbonyle, trityle, p.méthoxybenzyloxycarbonyle ou formyle: par traitement en milieu acide. De préference on utilise l'acide trifluoracétique en opérant à une température comprise entre 0 et 20°C, ou bien on utilise l'acide formique, phosphorique ou polyphosphorique anhydres ou aqueux à une température comprise entre 20 et 60°C, ou encore l'acide paratoluènesulfonique ou méthanesulfonique dans l'acétone ou l'acétonitrile, à une température comprise entre 20°C et la température de reflux du mélange réactionnel. Dans ces conditions le produit de formule générale (I) peut être obtenu sous forme de trifluoracétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique;
- lorsqu'il s'agit d'un radical trichloro-2,2,2 éthoxycarbonyle ou p.nitrobenzyloxycarbonyle ou d'un radical de formule générale ($IIIa_8$) dans laquelle Z est trichloro-2,2,2 éthyle ou nitrobenzyle: par réduction (notamment traitement par le zinc dans l'acide acétique);
- lorsqu'il s'agit d'un radical chloracétyle ou trichloracétyle: par application de la méthode décrite dans le brevet français publié sous le n° 2 243 199;
- lorsqu'il s'agit d'un radical benzyle ou benzyloxycarbonyle: par hydrogénation catalytique;
- lorsqu'il s'agit d'un radical trifluoracétyle: par traitement en milieu basique;
- lorsqu'il s'agit d'un radical de formule générale ($IIIa_8$): selon la méthode décrite dans le brevet belge 833 619;
- lorsqu'il s'agit d'un radical diphénylphosphinoyle: selon la méthode décrite par P. Haake et coll., J. Am. Chem. Soc., 95, 8073 (1973).

L'élimination des groupements protecteurs du radical hydroxy s'effectue le cas écheant:

- lorsqu'il s'agit de groupement trityle, tétrahydropyrannyle ou méthoxybenzyle: par acidolyse, par exemple par l'acide trifluoracétique, l'acide formique aqueux ou non, ou l'acide paratoluènesulfonique;
- lorsqu'il s'agit des groupements alcoyloxycarbonyle ou aryloxycarbonyle: selon les méthodes décrites dans le brevet belge 871 213.

2° — On peut aussi faire agir un produit de formule générale (VIII) tel que défini ci-dessus sur un dérivé de la céphalosporine de formule générale (I) dans laquelle R représente un radical protecteur et X, Hal et R' sont définis comme précédemment, puis éventuellement un agent de déshydratation, pour préparer un produit de formule générale:

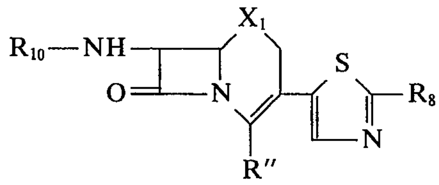

(IX)

dans laquelle $X_1$ et $R_8$ sont définis comme précédemment, R″ est défini comme R′ précédemment ou représente un radical carboxy et $R_{10}$ représente un radical protecteur d'amino, facilement éliminable, tel que défini précédemment.

La réaction s'effectue dans les conditions décrites précédemment en 1°—.

Le cas échéant, l'élimination du radical protecteur d'acide s'effectue dans les conditions décrites précédemment.

On élimine alors le radical protecteur d'amino $R_{10}$ ou le cas échéant on élimine simultanément le radical protecteur $R_{10}$ et le radical protecteur contenu par R′ dans le produit de formule générale (IX), pour préparer une amino-7 céphalosporine (ou oxacéphalosporine) de formule générale:

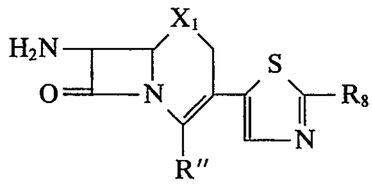

(X)

dans laquelle $R_8$ et $X_1$ sont définis comme précédemment et R″ est défini comme R′ ou représente un radical carboxy.

L'élimination du radical protecteur $R_{10}$ s'effectue par toute méthode connue pour libérer une fonction amine sans toucher au reste de la molécule.

On opère notamment dans les conditions décrites dans le brevet belge 883 415ou décrites ci-avant en 1°.

Puis on acyle de dérivé d'amino-7 céphalosporine de formule générale (X) au moyen d'un acide représenté par la formule générale:

$$R_7—OH \qquad (XI)$$

[dans laquelle $R_7$ est défini comme précédemment et dont, le cas échéant, la fonction amine et/ou la fonction acide sont préalablement protégées], ou d'un dérivé réactif de cet acide, et le cas échéant réduit le sulfoxyde obtenu puis on élimine les radicaux protecteurs.

Lorsque $R_7$ contient un radical hydroxy, ce dernier peut être libre ou protégé par un radical protecteur tel que défini précédemment.

Lorsque $R_7$ contient un radical amino, celui-ci est protégé par un radical tel que défini précédemment pour $R_2$.

Lorsque $R_7$ contient une fonction acide, cette dernière est protégée par un radical tel que défini précédemment.

Lorsque l'on utilise le produit de formule générale (XI) sous sa forme acide, on effectue la condensation sur l'amino-7 céphalosporine de formule générale (X) dans laquelle R″ est un radical protecteur, en présence d'un agent de condensation tel qu'un carbodiimide (par exemple dicyclohexylcarbodiimide), le NN′-carbonyldiimidazole ou l'éthoxy-2 éthoxycarbonyl-1 dihydro-1,2 quinoléine, à une température comprise entre −20 et 40°C, puis on élimine les groupements protecteurs.

Lorsque l'on veut utiliser un dérivé réactif de l'acide de formule générale (XI), il est possible de mettre en oeuvre l'anhydride, un anhydride mixte, ou un ester réactif de formule générale:

$$R_7—OZ_1 \qquad (XII)$$

dans laquelle, $R_7$ étant défini comme précédemment, $Z_1$ représente un radical succinimido, benzotriazolyl-1, nitro-4 phényle, dinitro-2,4 phényle, pentachlorophényle ou phtalimido, étant entendu que lorsque $R_7$ contient un radical amino, la fonction amine de tous ces dérivés réactifs est préalablement protégée.

Il est également avantageux d'utiliser un halogénure d'acide. Dans ce dernier cas, lorsque $R_7$ contient un groupement amino, on peut faire réagir le chlorhydrate du chlorure d'acide ce qui évite dans ce cas particulier la protection préalable du radical amino.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone

(par exemple acétone) ou dans des mélanges des solvants ci-dessus, en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine) ou en présence d'un agent de silylation tel que le bistriméthylsilylacétamide, ou bien dans un milieu hydroorganique en présence d'un agent alcalin de condensation tel que le bicarbonate de sodium, et l'on opère à une température comprise entre −40 et 40°C, puis on remplace les groupements protecteurs par des atomes d'hydrogène.

Lorsque l'on met en oeuvre un ester réactif de formule générale (XII), on opère généralement en présence d'une amine tertiaire (par exemple triéthylamine) dans un solvant organique tel que le diméthylformamide à une température comprise entre 0 et 40°C, puis on remplace le ou les groupements protecteurs par des atomes d'hydrogène.

L'élimination des radicaux protecteurs s'effectue dans les conditions décrites précédemment.

Les produits de formule générale (VIII) et (XIV) décrits ci-après, peuvent être préparés par action d'ammoniac sur l'isothiocyanate ou le dithiocarbamate correspondant, ou par action de sulfure d'hydrogène sur le nitrile correspondant, ou plus précisément selon les méthodes citées ci-après:

— lorsque $R_8$ est un atome d'hydrogène: selon la méthode décrite par A. W. Hofmann, Chem. Ber. 11, 340 (1878),
— lorsque $R_8$ est un radical acylaminophényle: par application de la méthode de L. Stephesen et coll., J. Chem. Soc. (C), 861 (1969),
— lorsque $R_8$ représente un radical alcoylthio: par action du dithiocarbamate d'ammonium sur l'halogénure d'alcoyle convenable,
— lorsque $R_8$ représente dialcoylamino: par application de la méthode de Mameli, Ann. Chimica, 46, 545 (1956),
— lorsque $R_8$ représente acylamino: selon la méthode décrite par M. L. Moore et F. S. Crossley, J. Am. Chem. Soc., 62, 3274 (1940),
— lorsque $R_8$ ou $R_8''$ représente benzoylamino, thénoylamino nicotinoylamino ou isonicotinoylamino: selon la méthode de W. H. Pike, Chem. Ber., 6, 755 (1873),
— lorsque $R_8$ représente acylamino, benzoylamino ou thénoylamino substitués sur l'atome d'azote par un radical alcoyle: par analogie avec la méthode décrite par Kurzer, J. Chem. Soc., 1957, 4461 à partir de N-cyanoacétamides, de N-cyanobenzamides ou de N-cyanothénoylamides obtenus respectivement par application de la méthode de R. Huffmann et F. C. Schaefer, J. Org. Chem., 28, 1816 (1963) ou de K. Hartke et E. Palou, Chem. Ber., 99 (10) 3155 (1966),
— lorsque $R_8$ est un radical acylamino, benzoylamino ou thénoylamino substitués sur l'atome d'azote par un radical phényle: selon la méthode décrite par P. K. Srivastava, Ind. J. Chem., 7 (4), 323 (1969),
— lorsque $R_8$ est un radical alcoyloxycarbonylamino: par application de la méthode décrite par R. E. Doran, J. Chem. Soc., 69, 331 (1896),
— lorsque $R_8$ est un radical dialcoylaminoéthylamino: selon la méthode décrite dans la demande de brevet allemand 2 738 711,
— lorsque $R_8$ est un radical dialcoylaminométhylèneamino: par application de la méthode décrite par H. Bredereck et coll., Ber., 97, 61 (1964),
— lorsque $R_8$ est alcoylidènehydrazo: par action de la thiosemicarbazide sur la cétone ou l'aldéhyde convenable.

II/ Lorsque l'on veut obtenir un produit de formule générale (VII) dans laquelle
a) $R_8$ est acylamino, benzoylamino ou thénoylamino éventuellement substitués sur l'atome d'azote, alcoyloxycarbonylamino, dialcoylaminométhylèneamino ou un radical acétamido substitué,
b) $R_8$ est un radical de structure $-AR_8'$ ou
c) $R_8$ est un radical acétamido substitué par un radical amino-2 éthylthio ou L amino-2 carboxy-2 éthylthio,

1°) il est également possible de préparer un produit de formule générale:

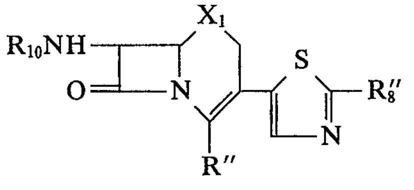

(XIII)

dans laquelle $R_{10}$, R″ et $X_1$ sont définis comme précédemment et $R_8''$ est un radical amino, alcoylamino ou phénylamino, un radical de structure

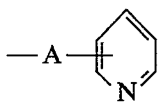

dans laquelle A qui est défini comme por la formule générale (VII) est substitué en position -3 ou -4 du noyau pyridyle, ou un radical halogénoacétamido (tel que chloracétamido, bromacétamido ou iodoacétamido), à partir d'un produit de formule générale (I) dans laquelle R est un radical protecteur d'amino,

— soit par action d'un produit de formule générale

$R_8''$ CS $NH_2$ (XIV)

(dans laquelle $R_8''$ est défini comme ci-dessus à l'exception de représenter un radical halogénoacétamido), puis éventuellement un agent de déshydratation, et le cas échéant réduction du sulfoxyde obtenu et éventuellement élimination du radical protecteur d'acide, dans les conditions définies précédemment en I/ 1°),

— soit lorsque $R_8''$ est un radical halogénoacétamido, à partir d'un dérivé de céphalosporine de formule générale (XIII) dans laquelle $R_8''$ est amino, par action d'un acide halogénoacétique ou d'un de ses dérivés, en opérant dans les conditions d'acylation définies précédemment en I/ 2°).

On traite alors le dérivé de céphalosporine de formule générale (XIII) par analogie avec la méthode décrite en I/ 2°) pour la préparation d'un produit de formule générale (VII) par l'intermédiaire de dérivés de céphalosporine de formule générales (IX) et (X), de manière à obtenir une thiazolylcéphalosporine de formule générale:

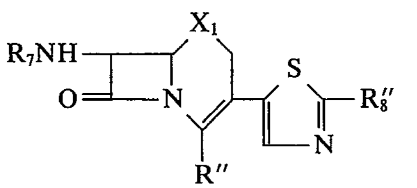

(XV)

dans laquelle R'', $R_8''$ et $X_1$ sont définis comme précédemment et $R_7$ est défini comme précédemment étant entendu que si $R_7$ contient un radical amino, celui-ci est protégé et si $R_8''$ représente un radical amino, ce dernier est de préférence protégé.

Les conditions de réalisation et d'élimination des radicaux protecteurs sont identiques à celles décrites antérieurement.

Le produit de formule générale (VII) est obtenu par application de l'un des procédés suivants, selon la signification de $R_8$

— lorsque l'on veut obtenir un produit de formule générale (VII) pour lequel $R_8$ est défini comme précédemment en a) ou représente un radical de structure —$AR_8'$ dans laquelle A est un radical bivalent de structure —NHCO—, on transforme un dérivé de céphalosporine de formule générale (XV) dans laquelle $R_8''$ est un radical amino, alcoylamino ou phénylamino, par toute méthode connue pour former une fonction amide, carbamate ou amidine sans toucher au reste de la molécule, puis le cas échéant réduit le sulfoxyde obtenu et élimine les groupements protecteurs,

— lorsque l'on veut obtenir un produit de formule générale (VII) dans laquelle $R_8$ est défini comme précédemment en b), on fait agir un halogénure de méthyle, de benzoylméthyle ou de carboxyméthyle (dont la fonction acide est libre ou préalablement protégée), sur un dérivé de céphalosporine de formule générale (XV) dans laquelle $R_8''$ est un radical de structure

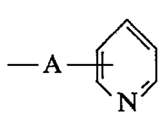

dans laquelle A est défini comme précédemment, puis le cas échéant réduit le sulfoxyde obtenu et élimine le cas échéant les groupements protecteurs.

La réaction s'effectue généralement dans un solvant organique tel qu'un amide (diméthylformamide, hexaméthylphosphorotriamide ou diméthylacétamide), un nitrile (acétonitrile par exemple), une cétone (acétone par exemple) ou un dérivé nitré (nitrométhane ou nitrobenzène par exemple), ou dans un mélange de tels solvants à une température comprise entre 0 et 80°C;

— lorsque l'on veut obtenir un produit de formule générale (VII) dans laquelle $R_8$ est défini comme précédemment en c), on fait agir la cystéamine ou la cystéine dont la fonction amine et le cas

échéant acide sont préalablement protégés, sur un dérivé de céphalosporine de formule générale (XV) dans laquelle $R_8''$ est un radical halogénoacétamido, puis le cas échéant réduit le sulfoxyde obtenu et élimine les radicaux protecteurs.

La réaction s'effectue généralement dans un solvant organique par exemple un amide (diméthylformamide), un nitrile (acétonitrile), ou un mélange de tels solvants, à une température comprise entre −20°C et la température de reflux du mélange réactionnel. De préférence on opère en présence d'une base organique tertiaire par exemple la diisopropyléthylamine, la diéthylphénylamine ou la triéthylamine.

Le cas échéant la réduction du sulfoxyde s'effectue selon les méthodes décrites dans la demande de brevet allemand 2 637 176. 2°) il est également possible de préparer directement un dérivé de céphalosporine de formule générale (XV) dans laquelle $R_8''$ est défini comme précédemment à l'exception de représenter un radical halogénoacétamido, par action d'un produit de formule générale (XIV) dans laquelle $R_8''$ est défini comme ci-dessus, sur un produit de formule générale (I) [dans laquelle R représente soit un radical de formule générale (IIa), soit prend une des significations données précédemment en B)] par analogie avec la méthode décrite précédemment pour la préparation d'un produit de formule générale (XIII) dans laquelle $R_8''$ est défini comme ci-dessus.

Il est également possible de préparer un produit de formule générale (XV) dans laquelle $R_8''$ est un radical halogénoacétamido, à partir des produits de formule générale (XV) ci-dessus, par analogie avec la méthode décrite précédemment pour la préparation des dérivés de la céphalosporine de formule générale (XIII).

Les produits de formule générale (VII) peuvent alors être obtenus à partir des dérivés de la céphalosporine de formule générale (XV), dans les conditions décrites précédemment.

Les produits de formule générale (IX), (X), (XIII) et (XV) dans laquelle $X_1$ est un radical sulfinyle peuvent éventuellement être obtenus par oxydation des produits de formules générales (IX), (X), (XIII) et (XV) dans laquelles $X_1$ est un atome de soufre, selon les méthodes décrites dans la demande debrevet allemand 2 637 176.

Les nouveaux produits selon la présente invention et les produits de formule générale (VII) peuvent être éventuellement purifiés par des méthodes physiques telles que la cristallisation, la chromatographie, ou l'ultrafiltration.

Les dérivés de la céphalosporine de formule générale (VII) dans laquelle les radicaux sont définis en $A_1$) et leurs sels pharmaceutiquement acceptables sont des agents antibactériens à spectre étroit particulièrement intéressants, qui manifestent une activité remarquable in vitro et in vivo sur les germes gram-positifs, particulièrement sur staphylocoques et surtout sur streptocoques D.

In vitro, les produits de formule générale (VII) se sont montrés actifs à une concentration comprise entre 0,03 et 8 $\mu g/cm^3$ sur des souches de staphylocoques sensibles à la pénicilline G (Staphylococcus aureus Smith), à une concentration comprise entre 0,00006 et 0,03 $\mu g/cm^3$ sur Streptococcus pyogenes Dig 7 et Streptococcus pneumoniae TIL et surtout à des concentrations comprises entre 4 et 60 $\mu g/cm^3$ sur Streptococcus faecium ATCC 9790.

In vivo les produits de formule générale (VII) se sont montrés actifs sur les infections expérimentales de la souris à Staphylococcus aureus Smith à une doese comprise entre 0,01 et 5 mg/kg par jour, par voie sous-cutanée.

Par ailleurs, la $DL_{50}$ des produits de formule générale (VII) est comprise entre 0,5 g/kg et des doses supérieures à 2,5 g/kg par voie sous-cutanée chez la souris.

Les dérivés de la céphalosporine de formule générale (VII) dans laquelle les radicaux sont définis en $B_1$) ont éte décrits dans la demande de brevet français 2 206 085 comme agents antibactériens à spectre large, actifs sur les germes à gram-positifs et surtout gram-négatifs. Cette demande française décrit la préparation des dérivés de céphalosporine par synthese totale. La synthèse de ces produits par l'intermédiaire des produits selon l'invention est considérablement plus facile.

D'un intérêt particulier sont les produits de formule générale (I) dans laquelle
le symbole X représente un atome d'oxygène ou de soufre,
le symbole Hal représente un atome de chlore, de brome ou d'iode,
le symbole R' représente un radical carboxy protégé par un groupement benzhydryle ou p.nitrobenzyle,
le symbole R représente soit un radical de formule générale (II a) dans laquelle $R_1$ est phényle ou phénoxy et $R_2$ est un atome d'hydrogène, ou, lorsque $R_1$ est phényle, un radical amino protégé, soit un radical protecteur d'amino choisi parmi t.butoxycarbonyle, trityle, ou un radical de formule générale (III $a_8$) dans laquelle Z est un radical alcoyle, ou bien
B/ le symbole X représente un atome de soufre et
le symbole R représente un radical de formule générale (II $b_1$) dans laquelle R″ est un radical aralcoyle, ou R représente un radical de formule générale (II $b_2$) dans laquelle m égale 0 ou 1, Y est un atome d'oxygène ou de soufre et R″ est alcoyle contenant 1 à 7 atomes de carbone, aryle ou aralcoyle qui peuvent être substitués par un ou deux halogènes, ou R est un radical de formule générale (II $b_3$) dans laquelle R″ est aryle et R‴ est amino protégé.

Les exemples suivants donnés à titre non limitatif illustrent la présente invention.

Exemple 1

A une solution refroidie à −55°C de 2 g benzhydryloxycarbonyl-2 t.butoxycarbonylamino-7 (diméthylamino-2 vinyl)-3 oxo-8 thia-5 aza-1 bicyclo[4.2.0] octène-2 (isomère E) dans 11 $cm^3$ de tétrahydrofurane sec on ajoute goutte à goutte, en 5 minutes, une solution de 0,2 $cm^3$ de brome dans 2 $cm^3$ de chlorure de méthylène sec. Le mélange réactionnel est agité pendant 1 heure à −60°C puis versé dans un mélange de 200 $cm^3$ d'acétate d'éthyle et 200 $cm^3$ de eua glacée. La couche organique est lavée par 100 $cm^3$ de solution demi-saturée de bicarbonate de sodium puis par 100 $cm^3$ d'eau et 100 $cm^3$ de solution demi-saturée de chlorure de sodium et séchée sur sulfate de sodium en présence de noir décolorant. Après filtration, le solvant est évaporé sous pression réduite (30 mm de mercure; 4 kPa) à 30°C. Le résidu est tepris par 50 $cm^3$ d'oxyde d'isopropyle que l'on évapore sous pression réduite (30 mm de mercure; 4 kPa) à 30°C. On obtient 1,8 g de benzhydryloxycarbonyl-2 (bromo-1 oxo-2 éthyl)-3 t.butoxycarbonylamino-7 oxo-8 thia-5 aza-1 bicyclo[4.2.0] octène-2 sous la forme d'un solide beige clair (mélange des deux épimères du bromoaldéhyde).

Spectre infra-rouge ($CHBr_3$), bandes caractéristiques ($cm^{-1}$) 3420, 1790, 1725, 1505, 1455, 1390, 1370, 1245, 1225, 760, 745.

Spectre de RMN du proton ($CDCl_3$, 350 MHz, $\delta$ en ppm, J en Hz)

épimère A

1,42 (s, 9H, $(CH_3)_3C-$); 3,71 et 3,55 (AB, J = 17,5, 2H, $-SCH_2-$); 5,06 (d, J = 4, 1H, H en 6); 5,22 (d, J = 9, 1H, −NH−); 5,65 (dd, J = 4 et 9, 1H, H en 7); 6,01 (s, 1H, $-C\underline{H}Br-$); 6,99 (s, 1H, $-CHAr_2$); 7,3 à 7,5 (m, 10H, aromatiques); 9,31 (s, 1H, −CHO).

épimère B

1,42 (s, 9H, $(CH_3)_3C-$); 3,35 et 3,65 (AB, J = 17,5, 2H, $-SCH_2-$); 5,01 (d, J = 4, 1H, H en 6); 5,29 (d, J = 9, 1H, −NH−); 5,72 (dd, J = 4 et 9, 1H, H en 7); 6,00 (s, 1H, $-C\underline{H}Br-$); 6,92 (s, 1H, $-CHAr_2$); 7,3 à 7,5 (m, 10H, aromatiques); 9,30 (s, 1H, −CHO).

Le benzhydryloxycarbonyl-2 t.butoxycarbonylamino-7 (diméthylamino-2 vinyl)-3 oxo-8 thia-5 aza-1 bicyclo[4.2.0] octène-2 (isomère E) peut être préparé comme décrit dans le brevet belge 883 415.

Exemple 2

En opérant de façon similaire à l'exemple 1 mais en traitant une solution de 21,4 g de benzhydryloxycarbonyl-2 t.butoxycarbonylamino-7 (diméthylamino-2 vinyl)-3- oxo-8 thia-5 aza-1 bicyclo[4.2.0] octène-2 (forme E) dans 100 $cm^3$ de tétrahydrofuranne sec à −60°C par 40 $cm^3$ d'une solution chlorométhylénique de chlore à 10% (poids/volume), on obtient 21,6 g de benzhydryloxycarbonyl-2 t.butoxycarbonylamino-7 (chloro-1 oxo-2 éthyl)-3 oxo-8 thia-5 aza-1 bicyclo[4.2.0] octène-2 (mélange des deux épimères du chloroaldéhyde).

Spectre infra-rouge ($CHBr_3$), bandes caractéristiques ($cm^{-1}$) 3420, 1785, 1720, 1505, 1450, 1390, 1365, 775, 740.

Spectre de RMN du proton (350 MHz, $CDCl_3$, $\delta$ en ppm, J en Hz)

épimère A

1,44 (s, 9H, $(CH_3)_3C-$); 3,46 et 3,60 (AB, J = 18, 2H, $-SCH_2-$); 5,06 (d, J = 5, 1H, H en 6); 5,24 (d, J = 9, 1H, −NH−); 5,67 (dd, J = 5 et 9, 1H, H en 7); 5,90 (s, 1H, −CHCl−); 6,96 (s, 1H, $-CHAr_2$); 7,20 à 7,60 (m, 10H, aromatiques); 9,38 (s, 1H, −CHO).

épimère B

1,44 (s, 9H, $(CH_3)_3C-$); 3,23 et 3,63 (AB, J = 18, 2H, $-SCH_2-$); 5,0 (d, J = 5, 1H, H en 6); 5,28 (d, J = 9, 1H, −NH−); 5,71 (dd, J = 5 et 9, 1H, H en 7); 5,94 (s, 1H, −CHCl−); 6,91 (s, 1H, $-CHAr_2$); 7,20 à 7,60 (m, 10H, aromatiques); 9,45 (s, 1H, −CHO).

Exemple 3

On ajoute 0,08 $cm^3$ d'une solution 0,5 M de brome dans du dichlorométhane à une solution refroidie à −70°C de 0,02 g de (diméthylamino-2 vinyl)-3 (nitro-4 benzyloxycarbonyl)-2 oxo-8 phénylacétamido-7 thia-5 aza-1 bicyclo[4.2.0] octène-2 et agite pendant 5 minutes à −70°C. Le mélange réactionnel est dilué par 20 $cm^3$ d'acétate d'éthyle et lavé par 20 $cm^3$ d'eau distillée et par 20 $cm^3$ de solution saturée de chlorure de sodium. La solution organique est séchée sur sulfate de magnésium

et concentrée sous pression réduite (30 mm de mercure; 4 kPa) à 30°C. On obtient 0,02 g d'une laque brun clair constituée essentiellement du mélange des deux épimères (bromoaldéhyde) du (bromo-1 oxo-2 éthyl)-3 (nitro-4 benzyloxycarbonyl)-2 oxo-8 phénylacétamido-7 thia-5 aza-1 bicyclo[4.2.0] octène-2 en proportions égales.

Spectre de RMN du proton (350 MHz, $CDCl_3$, $\delta$ en ppm, J en Hz)
3,33 et 3,51 (2D, 1H du $-S-CH_2-$ pour les deux isomères);

3,57 à 3,77 (Mt, l'autre –H du $-S-CH_2-$ et $Ar-CH_2-CO-N\langle$ pour les deux isoméres);

4,98 et 5,04 (2D, J ≃ 4,5, –H en 6);
5,29 et 5,38 + 5,30 et 5,39(4D, J ≃ 12, $-COO-CH_2-Ar$ des deux isomères);
5,88 et 5,93 (2DD, J ≃ 4,5 et 9, –H en 7 des deux isomères);

6,09 et 6,21 (2S, >CHBr des deux isomères); 7,2 à 7,4 (aromatiques + –CONH– en 7 es deux isomères);

7,56 (D, J ≃ 7,5, H aromatiques en méta du $NO_2$ des deux isomères);
8,23 (D, J ≃ 7,5, –H aromatiques en ortho du $NO_2$ des deux isomères);

9,4 et 9,44 (2S, $-C{\lesssim}^{O}_{H}$ des deux isomères).

## Exemple 4

On traite 1,4 g de (diméthylamino-2 vinyl)-3 oxo-8 (nitro-4 benzyloxycarbonyl)-2 phénoxyacétamido-7 thia-5 aza-1 bicyclo[4.2.0] octène-2 (isomère E) purifié, par 0,132 $cm^3$ de brome en opérant selon le mode opératoire décrit dans l'exemple 1 et on obtient 1,4 g de (bromo-1 oxo-2 éthyl)-3 oxo-8 (nitro-4 benzyloxycarbonyl)-2 phénoxyacétamido-7 thia-5 aza-1 bicyclo[4.2.0] octène-2 (mélange des épimères du bromoaldéhyde dans les proportions 50/50) sous la forme de meringue beige.

Spectre RMN du proton (350 MHz, $CDCl_3$, $\delta$ en ppm, J en Hz)
3,39 et 3,68 + 3,57 et 3,78 (2 séries de 2D, J ≃ 18, 2H, $-S-CH_2-$);
4,57 (S, 2H, $-O-CH_2-CO-$ des deux isomères); 5,05 et 5, 11 (2D, J ≃ 5, 1H, –H en 6);
5,32 et 5,41 + 5,33 et 5,42 (2 séries de 2D, J ≃ 13, 2H, $-COOCH_2-$);
5,96 et 6,02 (2DD, J ≃ 5 et 9, 1H, –H en 7);

6,11 et 6,22 (2S, 1H, >CH Br); 6,93 (D, J ≃ 7,5, 2H, aromatiques en ortho du phénoxy);

7,05 (T, J ≃ 7,5,1H, aromatiques en para du phénoxy);
7,33 (T, J ≃ 7,5 + D, J ≃ 9, (3H), aromatiques en méta du phénoxy + –CO–NH–);
7,59 et 7,60 (2D, J ≃ 7,5, 2H, aromatiques en méta nitro);

8,23 (D, J ≃ 7,5 2H, aromatiques en ortho du nitro); 9,43 et 9,46 (2S, 1H, $-C{\lesssim}^{O}_{H}$).

## Exemple 5

A une solution refroidie à –78°C de 7,14 g de benzhydryloxycarbonyl-2 (diméthylamino-2 vinyl)-3 oxo-8 tritylamino-7 oxa-5 aza-1 bicyclo[4.2.0] octène-2, isomère E, dans 50 $cm^3$ de tétrahydrofuranne sec, on ajoute en 10 minutes 1,72 g de brome en solution dans 7 $cm^3$ de chlorure de méthylène sec; on agite pendant 10 minutes à –78°C puis on laisse remonter la température jusqu'a –40°C et ajoute 0,39 $cm^3$ d'eau distillée. Après 10 minutes, on prélève une fraction aliquote de 5 $cm^3$ du mélange réactionnel, que l'on dilue dans 10 $cm^3$ d'acétate d'éthyle. Cette phase organique est lavée par 5 $cm^3$ d'eau distillée, puis par 5 $cm^3$ d'une solution demi-saturée de chlorure de sodium. Elle est alors séchée sur sulfate de sodium anhydre et concentrée à sec sous pression réduite (30 mm de mercure; 4 kPa) à 20°C. Le résidu (0,61 g) est chromatographié sur une colonne (hauteur: 21 cm; diamètre: 1,8 cm) de gel de silice (0,04–0,06 mm) en éluant sous 40 kPa par un mélange cyclohexane-acétate d'éthyle 75–25 (en volumes) et en recueillant des fractions de 25 $cm^3$. Les fractions 3 et 4 contenant le mélange de épimères de benzhydryloxycarbonyl-2 (bromo-1 oxo-2 éthyl)-3 oxo-8 tritylamino-7 oxa-5 aza-1 bicyclo[4.2.0] octène-2 sont rassemblées et concentrées à sec sous pression réduite (30 mm de mercure; 4 kPa) à 20°C. On obtient 0,03 g de mélange des deux bromoaldéhydes, sous la forme d'une meringue marron.

Position des pics caractéristiques du spectre de RMN du proton (350 MHz, $CDCl_3$, $\delta$ en ppm, J en Hz)

6,22 et 6,41 (s, 2H, >CHBr); 9,27 (s, 2H, $-C\underline{H}O$).

Le benzhydryloxycarbonyl-2 (diméthylamino-2 vinyl)-3 oxo-8 tritylamino-7 oxa-5 aza-1 bicyclo[4.2.0] octène-2, forme E peut être obtenu de la manière suivante:

On chauffe à 80°C, sous azote, une solution de 4,25 g de benzhydryloxycarbonyl-2 méthyl-3 oxo-8 tritylamino-7 oxa-5 aza-1 bicyclo[4.2.0] octène-2 dans 20 $cm^3$ de diméthylformamide. On ajoute, goutte à goutte pendant 7 minutes, dans la solution maintenue sous agitation à 80°C, 1,55 $cm^3$ de t.butoxy bis-diméthylaminométhane. L'addition terminée, le mélange est conservé sous agitation à 80°C pendant 17 minutes. La solution est diluée par 150 $cm^3$ d'acétate d'éthyle, la phase organique est lavée par 3 fois 60 $cm^3$ d'eau distillée et 60 $cm^3$ d'une solution demi-saturée de chlorure de sodium, séchée sur sulfate de sodium, filtrée et concentrée à sec à 30°C sous pression réduite (20 mm de mercure; 2,7 kPa). Le résidu est trituré dans 150 $cm^3$ d'éther éthylique, la suspension obtenu est filtrée et le filtrat est concentré à sec à 30°C sous pression réduite (20 mm de mercure; 2,7 kPa). On obtient 3,14 g d'un produit brut constitué principalement de benzhydryloxycarbonyl-2 (diméthylamino-2 vinyl)-3 oxo-8 tritylamino-7 oxa-5 aza-1 bicyclo[4.2.0] octène-2, formule E utilisable sans purification supplémentaire.

Spectre infra-rouge (KBr), bandes caractéristiques ($cm^{-1}$) 1780, 1660, 1615, 1490, 1450, 745, 700.

Spectre de RMN du proton (350 MHz, $CDCl_3$, $\delta$ en ppm, J en Hz)

2,77 (s, 6H, $-N(CH_3)_2$; 3,71 (d, J = 3,5, 1H, H en 6); 4,12 et 4,53 (2d, J = 17, 2H, $-CH_2-O-$); 4,26 (mf, 1H, H en 7); 6,24 et 6,40 (2d, J = 13, 2H, $-CH=CH-$); 6,81 (s, 1H, $-COO-CH(C_6H_5)_2$).

7,74 g de benzhydryloxycarbonyl-2 méthyl-3 oxo-8 tritylamino-7 oxa-5 aza-1 bicyclo[4.2.0] octène-2 sont préparés selon un schéma de synthèse décrit dans le brevet américain 4 108 992 dans lequel on remplace le glyoxylate de t.butyle par le glyoxylate de benzhydryle préparé selon le brevet français 1 495 047.

L'oxacéphalosporine attendue est obtenue sous la forme d'un solide blanc à partir de 13,2 g de tritylamino-3 (propyne-2 yloxy)-4 oxo-2 azétidine.

Spectre infra-rouge (KBr), bandes caractéristiques ($cm^{-1}$) 3340, 1780, 1715, 1620, 1595, 1585, 1490, 1450, 1220, 745, 700.

Spectre de RMN du proton (350 MHz, $CDCl_3$, $\delta$ en ppm, J en Hz)

1,90 (s, 3H, $-CH_3$); 3,75 (d, J = 3,5, 1H, H en 6); 3,87 et 4,08 (2d, J = 18, 2H, $-CH_2-O-$); 4,30 (d, J = 3,5, 1H, H en 7); 6,85 (s, 1H, $-COO-\underline{H}C(C_6H_5)_2$); 7,15 à 7,4 (mt, 26H, aromatiques et $-\underline{H}N-C(C_6H_5)_3$).

## Exemple 6

Selon la mode opératoire de l'exemple 1, on traite 2,3 g de benzhydryloxycarbonyl-2 (diméthylamino-2 vinyl)-3 diéthoxyphosphorylamino-7 oxo-8 thia-5 aza-1 bicyclo[4.2.0] octène-2 (isomère E) dans 12 $cm^3$ de tétrahydrofuranne sec par une solution de 0,2 $cm^3$ de brome dans 0,8 $cm^3$ de dichlorométhane puis on verse le mélange réactionnel dans un mélange de 40 $cm^3$ d'acétate d'éthyle et 100 $cm^3$ d'eau glacée. La couche organique est lavée par 3 fois 50 $cm^3$ d'eau glacée puis séchée sur sulfate de magnésium. Après évaporation du solvent sous pression réduite (30 mm de mercure; 4 kPa) à 30°C on obtient 2,49 g de benzhydryloxycarbonyl-2 (bromo-1 oxo-2 éthyl)-3 diéthoxyphosphorylamino-7 oxo-8 thia-5 aza-1 bicyclo[4.2.0] octène-2 (mèlange des bromoaldéhydes diastéréoisomères) sous la forme d'une meringue beige.

Spectre infra-rouge ($CHBr_3$), bandes caractéristiques ($cm^{-1}$) 3380, 2730, 1785, 1720, 1490, 1245, 1020, 975, 760, 740.

Spectre RMN du proton (350 MHz, $CDCl_3$, $\delta$ en ppm, J en Hz) mélange de deux diastéréoisomères dans les proportions 55/45 (A/B)

1,30 à 1,40 (Mt, $\simeq$ 6H, $-CH_3$ de A et B);

3,36 et 3,68 + 3,53 et 3,74 (2 séries de 2D, J $\simeq$ 18, $-CH_2S-$ de A et B);

3,50 et 3,56 $\left(2T, J \simeq 11, >\underset{\underset{O}{\|}}{P}-NH- \text{ de A et B}\right)$;

4 à 4,2 (Mt, $\simeq$ 4H, $-O-CH_2-$ de A et B);

5,01 et 5,06 (2D, J $\simeq$ 5, $-H$ en 6 de A et B); 5,16 et 5,24 (2DT, J $\simeq$ 5 et 11, 2H, $-H$ en 7 de A et B);

5,97 (s, 1H, $>CH-Br$ de A et B); 6,90 (s, $-COOC\underline{H}(C_6H_5)_2$ de B); 6,97 (s, $-COO-C\underline{H}(C_6H_5)_2$ de A);

7,25 à 7,45 (Mt, aromatiques de A et B); 9,30 $\left(s, -C\begin{smallmatrix}\nearrow O\\ \searrow H\end{smallmatrix} \text{ de B}\right)$; 9,32 $\left(s, -C\begin{smallmatrix}\nearrow O\\ \searrow H\end{smallmatrix} \text{ de A}\right)$.

Le benzhydryloxycarbonyl-2 (diméthylamino-2 vinyl)-3diéthoxyphosphorylamino-7 oxo-8 thia-5 aza-1 bicyclo[4.2.0] octène-2 (isomère E) peut être préparé de la manière suivante:

A une solution de 10 g benzhydryloxycarbonyl-2 diéthoxyphosphorylamino-7 méthyl-3 oxo-8 thia-5 aza-1 bicyclo[4.2.0] octène-2 dans 100 $cm^3$ de N,N-diméthylformamide sec à 80°C on ajoute 6 $cm^3$

de t.butoxy bisdiméthylaminométhane. Après 12 minutes à 80°C le mélange réactionnel est versé dans 400 $cm^3$ d'acétate d'éthyle et lavé 5 fois par 250 $cm^3$ d'eau distillée. Après séchage sur sulfate de magnésium et évaporation du solvant sous pression réduite (30 mm de mercure; 4 kPa) à 30°C on obtient 10,5 g de benzhydryloxycarbonyl-2 (diméthylamino-2 vinyl)-3 diéthoxyphosphorylamino-7 oxo-8 thia-5 aza-1 bicyclo[4.2.0] octène-2 (isomère E) sous la forme d'un solide cristallisé orange. Ce produit est purifié par cristallisation dans 30 $cm^3$ d'acétate d'éthyle et on obtient 5,3 g de benzhydryloxycarbonyl-2 (diméthylamino-2 vinyl)-3 diéthoxyphosphorylamino-7 oxo-8 thia-5 aza-1 bicyclo[4.2.0] octène-2 (isomère E) sous la forme d'une poudre cristalline jaune (F. = 193–194°C).

Spectre infra-rouge ($CHBr_3$), bandes caractéristiques ($cm^{-1}$) 3370, 3320, 2800, 1760, 1680, 1610, 1530, 1230, 1010, 970, 750.

Spectre RMN du proton (350 MHz, $CDCl_3$, $\delta$ en ppm, J en Hz)

1,35 et 1,40 (2T, J $\simeq$ 7, 6H, $-CH_3$); 2,90 (s, 6H, $-N(CH_3)_2$); 3,08 et 3,18 (2D, J $\simeq$ 15, 2H, $-S-CH_2-$);

3,84 (T, J $\simeq$ 10, 1H, $>P(=O)-NH-$); 4,15 et 4,22 (2Q, J $\simeq$ 7, 4H, $-OCH_2$;

4,81 (DT, J $\simeq$ 4,5 et 10, 1H, –H en 7); 5,08 (D, J $\simeq$ 4,5, 1H, –H en 6);

6,53 et 6,78 (2D, J $\simeq$ 14, 2H, $-CH=CH-N<$); 6,88 (s, 1H, $-COO-\underline{CH}(C_6H_5)_2$);

7,20 à 7,50 (Mt, 10H, aromatiques).

Le benzhydryloxycarbonyl-2 diéthoxyphosphorylamino-7 méthyl-3 oxo-8 thia-5 aza-1 bicyclo[4.2.0] octène-2 peut être préparé selon la méthode de A. Morimoto et al., J. C. S. Perkin I, 1109 (1980).

## Exemple 7

En opérant selon le mode opératoire de l'exemple 2, on traite par 18,4 $cm^3$ d'une solution à 5% de chlore dans le dichlorométhane, une solution refroide à −70°C de 7,3 g de benzhydryloxycarbonyl-2 diéthoxyphosphorylamino-7 (diméthylamino-2 vinyl)-3 oxo-8 thia-5 aza-1 bicyclo[4.2.0] octène-2 (isomère E) dans 36 $cm^3$ de tétrahydrofuranne sec. On obtient 7 g de benzhydryloxycarbonyl-2 (chloro-1 oxo-2 éthyl)-3 diéthoxyphosphorylamino-7 oxo-8 thia-5 aza-1 bicyclo[4.2.0] octène-2 (mélange des diastéréoisomères du chloroaldéhyde) sous la forme d'une meringue orangée.

Spectre infra-rouge ($CHBr_3$), bandes caractéristiques ($cm^{-1}$) 3380, 2730, 1785, 1720, 1490, 1245, 1020, 975, 760, 740.

Spectre RMN du proton (350 MHz, $CDCl_3$, $\delta$ en ppm, J en Hz) mélange de deux diastéréoisomères dans les proportions 55 : 45 (A et B)

1,30 à 1,40 (Mt, $\simeq$ 6H, $-CH_3$ de A et B);

3,23 et 3,64 + 3,51 (2D, J $\simeq$ 18 + AB limite, J $\simeq$ 18, 2H, $-CH_2-S-$ de A et B);

3,54 (T, J $\simeq$ 10, 1H, $>P(=O)-NH-$ de A et B); 4,03 à 4,17 (Mt, $\simeq$ 4H, $-O-CH_2$ de A et B);

4,98 et 5,05 (2D, J $\simeq$ 5, 1H, –H en 6 de A et B); 5,17 et 5,25 (2DT, J $\simeq$ 10 et 5, H en 7 de A et B);

5,87 (s, $>CH-Cl$ de B); 5,91 (s, $>CH-Cl$ de A); 6,90 (s, $-COO-\underline{CH}(C_6H_5)_2$ de B);

6,95 (s, $-COO\underline{CH}\ (C_6H_5)_2$ de A); 7,25 à 7,50 (Mt, aromatiques de A et B);

9,38 (s, $-C(=O)H$ de A); 9,42 (s, $-C(=O)H$ de B).

## Exemple 8

A une solution de 2,67 g de benzhydryloxycarbonyl-2 t.butoxycarbonylamino-7 (diméthylamino-2 vinyl)-3 oxo-8 thia-5 aza-1 bicyclo[4.2.0] octène-2 (isomère E) dans 30 $cm^3$ de tétrahydrofuranne refroidie à −20°C on ajoute en 25 minutes une solution de 0,89 g de N-bromosuccinimide dans 20 $cm^3$ de tétrahydrofuranne. Le mélange réactionnel est agité pendant 30 minutes à −20°C puis concentré à sec sous pression réduite (30 mm de mercure; 4 kPa) à 30°C.

Le résidu obtenu est chromatographié sur une colonne (hauteur: 22 cm, diamètre: 4,4 cm) de gel de silice (0,04–0,06 mm) en éluant sous une pression de 0,4 bar par un mélange de cyclohexane et d'acétate d'éthyle (60/40 en volumes) et en recueillant des fractions de 50 $cm^3$. Les fractions 3 à 6 contenant le produit pur sont réunies et concentrées à sec sous pression réduite (30 mm de mercure, 4 kPa) à 30°C. On obtient 1.3 g de benzhydryloxycarbonyl-2 (bromo-1 oxo-2 éthyl)-3 t.butoxycarbonylamino-7 oxo-8 thia-5 aza-1 bicyclo[4.2.0] octène-2 (mélange d'épimères du bromoaldéhyde) sous

la forme d'une meringue orangée dont les spectres infra-rouge et de RMN du proton sont identiques à ceux du produit décrit dans l'exemple 1.

Les exemples suivants illustrent la préparation et l'utilisation des produits selon l'invention, sans qu'il soit nécessaire d'isoler le produit de formule générale (I) pour le mettre en oeuvre dans une nouvelle phase réactionelle.

Exemple 9

A une solution refroidie à −55°C de 5,35 g de benzhydryloxycarbonyl-2 t.butoxycarbonylamino-7 (diméthylamino-2 vinyl)-3 oxo-8 thia-5 aza-1 bicyclo[4.2.0] octène-2 forme E dans 20 cm$^3$ de tétrahydrofuranne sec on ajoute en 5 minutes une suspension de 2,54 g d'iode dans 20 cm$^3$ de chlorure de méthylène sec. Le mélange réactionnel est agité pendant 2 heures en laissant progressivement remonter la température à 0°C puis traite par 0,36 cm$^3$ d'eau distillée et agite pendant 2 heures à +5°C. A 25 cm$^3$ de ce mélange réactionnel contenant en solution le mélange des épimères du benzhydryloxycarbonyl-2 t.butoxycarbonylamino-7 (iodo-1 oxo-2 éthyl)-3 oxo-8 thia-5 aza-1 bicyclo[4.2.0] octène-2, on ajoute une solution de 0,57 g de thiourée dans un mélange de 5 cm$^3$ de tétrahydrofuranne et de 2 cm$^3$ d'eau puis on laisse agiter pendant 16 heures à 25°C. On ajoute 0,57 g de thiourée et agite encore pendant 3 heures à 25°C puis on dilue par 150 cm$^3$ d'acétate d'éthyle. La phase organique est lavée successivement par 100 cm$^3$ d'eau distillée, 100 cm$^3$ de solution saturée de bicarbonate de sodium, 100 cm$^3$ d'eau distillée et 100 cm$^3$ de solution demi-saturée de chlorure de sodium. Après séchage sur sulfate de magnésium le solvant est évaporé sous pression réduite (30 mm de mercure; 4 kPa) à 30°C. Le résidu est chromatographié sur une colonne (hauteur: 30 cm; diamètre: 2 cm) de silice (0,04—0,06 mm) en éluant sous 0,4 bar (40 kPa) par un mélange de cyclohexane et d'acétate d'éthyle 45—55 (en volumes). Après évaporation des solvants on recueille 0,08 g d'(amino-2 thiazolyl-5)-3 benzhydryloxycarbonyl-2 t.butoxycarbonylamino-7 oxo-8 thia-5 aza-1 bicyclo[4.2.0] octène-2 sous la forme d'une meringue orangée dont les caractéristiques sont les suivantes:

Spectre infra-rouge ($CHBr_3$), bandes caractéristiques (cm$^{-1}$) 3480, 3420, 3380, 1780, 1720, 1600, 1495, 1455, 1390, 1370, 1240, 1220, 755, 740.

Spectre RMN du proton (350 MHz, $CDCl_3$, $\delta$ en ppm, J en Hz)
1,47 (s, 9H, $-C(CH_3)_3$); 3,46 et 3,61 (2d, J = 18, 2H, $-S-CH_2-$); 5,01 (d, J = 4,5, 1H, −H en 6); 5,27 (mf, 2H, $-NH_2$); 5,57 (d, J = 9, 1H, −CO—NH—); 5,64 (dd, J = 9 et 4,5, 1H, −H en 7); 6,85 (s, 1H, $-COO-C\underline{H}(C_6H_5)_2$) 6,94 (s, 1H, −H thiazole); 7,10 à 7,50 (mt, 10H, aromatiques).

Exemple 10

A une solution refroidie à −60°C de 11,3 g de benzhydryloxycarbonyl-2 [(D) $\alpha$-t.butoxycarbonylaminophénylacétamido]-7 (diméthylamino-2 vinyl)-3 oxo-8 thia-5 aza-1 bicyclo[4.2.0] octène-2, isomère E, dans 80 cm$^3$ de tétrahydrofuranne sec on ajoute en 10 minutes 2,69 g de brome en solution dans 5 cm$^3$ de chlorure de méthylène sec. On agite pendant 45 minutes en laissant remonter la température jusqu'à −15°C puis ajoute un mélange de 0,6 cm$^3$ d'eau distillée et de 3 cm$^3$ de tétrahydrofuranne. Après 30 minutes à −15°C on ajoute au mélange réactionnel, constitué d'une solution du mélange des bromaldéhydes épimèrs du benzhydryloxycarbonyl-2 (bromo-1 oxo-2 éthyl)-3 [(D) $\alpha$-t.butoxycarbonylaminophénylacétamido]-7 oxo-8 thia-5 aza-1 bicyclo[4.2.0] octène-2 une solution de 1.93 g de thiourée dans un mélange de 18 cm$^3$ de tétrahydrofuranne et 4 cm$^3$ d'eau. Le mélange réactionnel est agité pendant 4 heures entre 5 et 20°C puis dilué par 500 cm$^3$ d'acétate d'éthyle et lavé par 300 cm$^3$ de solution demi-saturée de bicarbonate de sodium, 300 cm$^3$ d'eau et 300 cm$^3$ de solution demi saturée de chlorure de sodium. Le résidu obtenu après séchage de la solution sur sulfate de sodium et évaporation sous pression réduite (30 mm de mercure) à 40°C est chromatographié sur une colonne (hauteur: 30 cm; diamètre: 5 cm) de silice (0,04—0,06 mm) en éluant sous 0,4 bar (40 kPa) par un mélange de cyclohexane et d'acétate d'éthyle 35—65 (en volumes) et en recueillant des fractions de 100 cm$^3$. Les fractions 18 à 31 contenant le produit pur sont rassemblées et concentrées à sec sous pression réduite (30 mm de mercure; 4 kPa) à 40°C. On obtient 2,08 g d'(amino-2 thiazolyl-5)-3 benzhydryloxycarbonyl-2 [(D) $\alpha$-t.butoxycarbonylaminophénylacétamido]-7 oxo-8 thia-5 aza-1 bicyclo[4.2.0] octène-2 sous la forme d'une meringue orangée.

Spectre infra-rouge ($CHBr_3$), bandes caractéristiques (cm$^{-1}$) 3480, 3390, 3200, 1785, 1715, 1695, 1600, 1495, 1455, 1390, 1370, 1220, 760, 740.

Spectre de RMN du proton (350 MHz, $CDCl_3$, $\delta$ en ppm, J en Hz)
1,43 (s, 9H, $-C(CH_3)_3$); 3,36 et 3,55 (2d, J = 18, 2H, $-S-CH_2-$); 4,94 (s large, 2H, $-NH_2$); 4,98 (d, J = 5, 1H, −H en 6); 5,24 (mf, 1H, $>N-\underset{|}{C}H-CO-N<$); 5,67 (d, J = 6, 1H, $-NH-\underset{\underset{O}{\|}}{C}-O-$); 5,83 (dd, J = 5 et 9, 1H, −H en 7); 6,73 (mf, 1H, −CO—NH—); 6,83 (s, 1H, $-COO-C\underline{H}(C_6H_5)_2$); 6,94 (s, 1H, −H thiazole); 7,10 à 7,50 (mt, 15H, aromatiques).

## Exemple 11

Une solution de 14 g de benzhydryloxycarbonyl-2 (diméthylamino-2 vinyl)-3 oxo-8 phénoxyacétamido-7 thia-5 aza-1 bicyclo[4.2.0] octène-2 (isomère E) dans 70 $cm^3$ de tétrahydrofuranne sec à −55°C est traitée par une solution de 1,32 $cm^3$ de brome dans 8 $cm^3$ de chlorure de méthylène sec (addition en 5 minutes). Le mélange réactionnel est agité pendant 1 heure à −55°C puis réchauffé à −30°C et additionné de 0,9 $cm^3$ d'eau distillée. On maintient pendant 2 heures à −30°C et on obtient une solution du mélange des bromaldéhydes épimères du benzhydryloxycarbonyl-2 (bromo-1 oxo-2 éthyl)-3 oxo-8 phénoxyacétamido-7 thia-5 aza-1 bicyclo[4.2.0] octène-2 que l'on traite par une solution de 2,25 g de thiourée dans un mélange de 20 $cm^3$ de tétrahydrofuranne et 6 $cm^3$ d'eau distillée. Après addition du réactif on laisse réchauffer le mélange réactionnel et on agite pendant 16 heures à 20°C, puis dilue par 350 $cm^3$ d'acétate d'éthyle. On lave par 300 $cm^3$ de solution saturée de bicarbonate de sodium puis par 3 fois 200 $cm^3$ d'eau distillée et par 200 $cm^3$ de solution demi-saturée de chlorure de sodium, sèche sur sulfate de magnésium filtre et concentre sous pression réduite (30 mm de mercure; 4 kPa) à 30°C. Le résidu est chromatographié sur une colonne (hauteur: 50 cm; diamètre: 4 cm) de gel de silice (0,2—0,06 mm) en éluant par de mélanges de cyclohexane et d'acétate d'éthyle: 60—40 (2 litres); 40—60 (2 litres); 30—70 (1 litre); 20—80 (1 litre) (proportions en volumes) et en recueillant des fractions de 300 $cm^3$. Les fractions 7 à 10 contenant le produit pur sont réunies et évaporées à sec sous pression réduite (30 mm de mercure; 4 kPa) à 30°C. On obtient 5,67 g d'(amino-2 thiazolyl-5)-3 benzhydryloxycarbonyl-2 oxo-8 phénoxyacétamido-7 thia-5 aza-1 bicyclo[4.2.0] octène-2 sous la forme d'une meringue jaune clair.

Spectre infra-rouge ($CHBr_3$), bandes caractéristiques ($cm^{-1}$) 3480, 3390, 1780, 1725, 1690, 1600, 1455, 1225, 750.

Spectre de RMN du proton (350 MHz, $CDCl_3$, $\delta$ en ppm, J en Hz)

3,43 et 3,53 (2d, J = 18, 2H, $-S-CH_2-$); 4,56 (s, 2H, $-O-CH_2-CO-N\langle$);

5,05 (d, J = 5, 1H, −H en 6); 5,22 (mf, 2H, $-NH_2$); 5,87 (dd, J = 9 et 5, 1H, −H en 7);
6,84 (s, 1H, $-COO-\underline{CH}(C_6H_5)_2$); 6,92 (s, 1H, −H thiazole);
6,91 (d, J = 7,5, 2H, −H aromatiques en ortho du phénoxy);
7,02 (t, J = 7,5, 1H, −H aromatique en para du phénoxy); 7,15 à 7,45 (mt, 12H, aromatiques);
7,51 (d, J = 9, 1H, −CO—NH—).

## Exemple 12

A une solution refroidie à −60°C de 0,5 g de benzhydryloxycarbonyl-2 (dichloro-3,4 phénylthio)acétamido-7 (diméthylamino-2 vinyl)-3 oxo-8 thia-5 aza-1 bicyclo[4.2.0] octène-2 (isomère E) dans 10 $cm^3$ de tétrahydrofuranne sec, on ajoute 0,043 $cm^3$ de brome, agite à −60°C pendant 70 minutes puis verse le mélange réactionnel dans un mélange de 60 $cm^3$ d'acétate d'éthyle et 60 $cm^3$ d'eau distillée. La phase aqueuse est lavée 3 fois par 60 $cm^3$ d'eau distillée, puis par 10 $cm^3$ de solution saturée de chlorure de sodium, séchée sur sulfate de magnésium et concentrée à sec sous pression réduite (30 mm de mercure; 4 kPa) à 30°C, pour donner 0,52 g de benzhydryloxycarbonyl-2 (bromo-1 oxo-2 éthyl)-3 (dichloro-3,4 phénylthio)acétamido-7 oxo-8 thia-5 aza-1 bicyclo[4.2.0] octène-2 (mélange des deux diastéréoisomères au niveau du substituant en -3) sous la forme d'une meringue beige clair.

Spectre de RMN du proton (350 MHz, $CDCl_3$, $\delta$ en ppm, J en Hz)
3,32 et 3,50 (2d, J = 18, 2H, 1H de chaque $-S-CH_2-$ des deux diastéréoisomères);
3,55 à 3,75 (mt, 3H, 1H de chaque $-S-CH_2-$ et $-S-CH_2-CO-N=$ des deux diastéréoisomères);
4,99 et 5,04 (2d, J = 5, 1H, −H en 6 des deux diastéréoisomères);
5,82 et 5,89 (2dd, J = 9 et 5, 1H, −H en 7 des deux diastéréoisomères);

5,97 et 6,01 (2s, 1H, $\rangle$CH Br des deux diastéréoisomères);

6,90 et 6,97 (2s, 1H, $-COO-\underline{CH}(C_6H_5)_2$ des deux diastéréoisomères);
7,17 et 7,18 (2dd, J = 8 et 2, 1H, −H aromatiques en 6 des deux diastéréoisomères);

7,20 à 7,5 (mt, aromatiques); 9,29 et 9,30 $\left(2s, 1H, -C\begin{smallmatrix}{\nearrow}O\\ {\searrow}H\end{smallmatrix} \text{ des deux diastéréoisomères}\right)$.

Le benzhydryloxycarbonyl-2 (dichloro-3,4 phénylthio) acétamido-7 (diméthylamino-2 vinyl)-3 oxo-8 thia-5 aza-1 bicyclo[4.2.0] octène-2 isomère E peut être obtenu de la manière suivante:

A une solution de 4,2 g de benzhydryloxycarbonyl-2 (dichloro-3,4 phénylthio) acétamido-7 méthyl-3 oxo-8 thia-5 aza-1 bicyclo[4.2.0] octène-2 dans 40 $cm^3$ de N,N diméthylformamide sec à 80°C, on ajoute 2,17 $cm^3$ de t.butoxybisdiméthylaminométhane puis agite le mélange réactionnel pendant 15 minutes à 80°C, avant de le verser dans un mélange de 500 $cm^3$ d'acétate d'éthyle et de 150 $cm^3$ d'eau distillée. La phase organique est lavée 3 fois par 150 $cm^3$ d'eau distillée puis par 50 $cm^3$ de

solution saturée de chlorure de sodium. Le résidu obtenu après concentration à sec sous pression réduite (30 mm de mercure; 4 kPa) à 40°C, est chromatographié sur une colonne (hauteur: 32 cm, diamètre: 6 cm) de gel de silice (0,04—0,063 mm) en éluant sous une pression de 0,5 bar (50 kPa) par un mélange de cyclohexane et d'acétate d'éthyle (40/60 en volumes), en recueillant des fractions de 100 $cm^3$. Les fractions 10 à 14 sont réunies et concentrées à sec sous pression réduite (30 mm de mercure; 4 kPa) à 30°C pour donner 0,51 g de benzhydryloxycarbonyl-2 (dichloro-3,4 phénylthio) acétamido-7 (diméthylamino-2 vinyl)-3 oxo-8 thia-5 aza-1 bicyclo[4.2.0] octène-2 isomère E sous la forme d'une meringue jaune.

Spectre de RMN du proton (250 MHz, $CDCl_3$, $\delta$ en ppm, J en Hz)
2,79 et 3,08 (2d, J = 15, 2H, $-S-CH_2-$); 2,85 (s, 6H, $-N(CH_3)_2$); 3,76 (s, 2H, $-CH_2-CO-N=$);
5,07 (d, J = 4,5, 1H, −H en 6); 5,4 (dd, J = 8 et 4,5, 1H, −H en 7);
6,34 et 6,48 (2d, J = 13,5, 2H, −CH=CH−); 6,82 (s, 1H, $-COO-\underline{C}\underline{H}(C_6H_5)_2$);
7,15 à 7,55 (mt, aromatiques); 8,0 (d, J = 8, 1H, −CO−NH−).

Le benzhydryloxycarbonyl-2 (dichloro-3,4 phénylthio) acétamido-7 méthyl-3 oxo-8 thia-5 aza-1 bicyclo[4.2.0] octène-2 peut être préparé par action du chlorure de l'acide dichloro-3,4 phénylthioacétique sur l'amino-7 benzhydryloxycarbonyl-2 méthyl-3 oxo-8 thia-5 aza-1 bicyclo[4.2.0] octène-2.

Spectre de RMN du proton (250 MHz, $CDCl_3$, $\delta$ en ppm, J en Hz)
2,09 (s, 3H, $-CH_3$); 3,13 et 3,40 (2d, J = 18, 2H, $-SCH_2-$);
3,60 et 3,71 (2d, J = 16, 2H, $-SCH_2CO-$); 4,29 (d, J = 5, 1H, H en 6);
5,74 (dd, J = 5 et 9, 1H, H en 7); 6,94 (s, 1H, $-COOC\underline{H}<$); 7,16 (dd, J = 2 et 8,5, H en 6 du phényle);
7,20 à 7,45 (mf, 13H, benzhydryle + −CONH− +H en 5 et en 2 du phényle).

## Exemple 13

On ajoute goutte à goutte en 30 minutes une solution de 4,31 g d'acide métachloroperbenzoïque à 85% dans 50 $cm^3$ de dichlorométhan à une solution refroidie entre 0 et −5°C de 14,69 g de benzhydryloxycarbonyl-2 (bromo-1 oxo-2 éthyl)-3 t.butoxycarbonylamino-7 oxo-8 thia-5 aza-1 bicyclo[4.2.0] octène-2 (mélange des diastéréoisomères an niveau du substituant en -3) dans 150 $cm^3$ de dichlorométhane. Après 30 minutes à −5°C le mélange réactionnel est versé sur un mélange de 100 $cm^3$ de solution demi-saturée de bicarbonate de sodium et de 200 $cm^3$ de dichlorométhane. La phase organique est lavée par 150 $cm^3$ d'eau distillée et 150 $cm^3$ de solution demi-saturée de chlorure de sodium puis séchée sur sulfate de sodium et concentrée à sec sous pression réduite (30 mm de mercure; 4 kPa) à 20°C pour donner 13,35 g den benzhydryloxycarbonyl-2 (bromo-1 oxo-2 éthyl)-3 t.butoxycarbonylamino-7 oxo-8 oxyde-5 thia-5 aza-1 bicyclo[4.2.0] octène-2 (mélange des diastéréoisomères au niveau du substituant en -3) brut sous la forme d'une meringue brun clair. Le produit brut obtenu est partagé en 2 lots qui sont chromatographiés sur une colonne (hauteur: 30 cm, diamètre: 5 cm) de gel de silice (0,04—0,063 mm) en éluant sous une pression de 0,4 bar (40 kPa) par un mélange de cyclohexane et d'acétate d'éthyle (40/60 en volumes) et en recueillant des fractions de 125 $cm^3$. Les fractions 7 à 10 des deux chromatographies sont réunies et concentrées à sec sous pression réduite (30 mm de mercure; 4 kPa) à 20°C pour donner 2,43 g de benzhydryloxycarbonyl-2 (bromo-1 oxo-2 éthyl)-3 t.butoxycarbonylamino-7 oxo-8 oxyde-5 thia-5 aza1 bicyclo[4.2.0] octène-2 (mélange des diastéréoisomères au niveau du substituant en -3) sous la forme d'une meringue jaune.

Spectre infra-rouge ($CHCl_3$), bandes caractéristiques ($cm^{-1}$) 3420, 1805, 1720, 1505, 1453, 1395, 1370, 1160, 1050.

Spectre de RMN du proton (350 MHz, $CDCl_3$, $\delta$ en ppm, J en Hz)
1,47 (s, 9H, $-C(CH_3)_3$ des deux diastéréoisomères);
3,27 et 3,42 (2d, J = 18, 2H, 1H de $-SCH_2-$ de chacun des deux diastéréoisomères);
3,83 et 3,98 (2d, J = 18, 2H, 1H de $-SCH_2-$ de chacun des deux diastéréoisomères);
4,50 et 4,60 (2d, J = 4, 1H, −H en 6 des deux diastéréoisomères);
5,70 à 5,97 (mt, 2H, −H en 7 et −CONH− des deux diastéréoisomères);
6,25 et 6,29 (2s, 1H, $>CH\,Br$ des deux diastéréoisomères);
6,94 et 6,98 (2s, 1H, $-COO-\underline{C}\underline{H}(C_6H_5)_2$ des deux diastéréoisomères);
7,20 à 7,50 (mt, aromatiques des deux diastéréoisomères);
9,27 et 9,29 $\left(2s, 1H, -C\langle^{H}_{O}\right.$ des deux diastéréoisomères$\left.\right)$.

## Exemple 14

Par oxydation de 53 g de benzhydryloxycarbonyl-2 t.butoxycarbonylamino-7 (chloro-1 oxo-2 éthyl)-3 oxo-8 thia-5 aza-1 bicyclo[4.2.0] octène-2 (mélange des diastéréoisomères) par 19 g d'acide m.chlorperbenzoïque à 90% dans 700 $cm^3$ de dichlorométhane en opérant selon le mode opératoire de l'exemple 13 on obtient 44,8 g de benzhydryloxycarbonyl-2 t.butoxycarbonylamino-7 (chloro-1 oxo-2 éthyl)-3 oxo-8 oxyde-5 thia-5 aza-1 bicyclo[4.2.0] octène-2 (mélange des diastéréoisomères) brut sous la forme d'une meringue brune.

Rf = 0,24—0,4 (chromatoplaque de gel de silice éluée par un mélange de cyclohexane et d'acétate de éthyle 35/85 en volumes).

## Exemple de reference 1

Le produit de l'exemple 1 peut être utilisé de la manière suivante:

A une solution refroidie à 3°C de 5,87 g de mélange des épimères du benzhydryloxycarbonyl-2 t.butoxycarbonylamino-7 (bromo-1 oxo-2 éthyl)-3 oxo-8 thia-5 aza-1 bicyclo[4.2.0] octène-2 dans 58,7 $cm^3$ de tétrahydrofuranne sec on ajoute en 5 minutes une solution de 1,8 g d'acétylthiourée dans 20 $cm^3$ de tétrahydrofuranne sec. Le mélange réactionnel est ensuite agité pendant 4 heures 30 minutes à 25°C puis dilué par 150 $cm^3$ d'acétate d'éthyle et 150 $cm^3$ de solution saturée de bicarbonate de sodium. La couche organique est lavée par 2 fois 100 $cm^3$ d'eau distillée puis par 100 $cm^3$ de solution saturée de chlorure de sodium et séchée. Après évaporation du solvant sous pression réduite (30 mm de mercure; 4 kPa) à 30°C le résidu est chromatographié sur une colonne (hauteur: 30 cm; diamètre: 6 cm) de silice (0,04—0,06 mm) en éluant sous une pression de 0,4 bar (40 kPa) par 1600 $cm^3$ de mélange de cyclohexane et d'acétate d'éthyle 70—30 (en volumes) puis par 2000 $cm^3$ de mélange de cyclohexane et d'acétate d'éthyle 30—70 (en volumes) et en recueillant des fractions de 100 $cm^3$. Les fractions 27 à 33 contenant le produit pur sont rassemblées et concentrées à sec. On obtient 0,54 g d'(acétamido-2 thiazolyl-5)-3 benzhydryloxycarbonyl-2 t.butoxycarbonylamino-7 oxo-8 thia-5 aza-1 bicyclo[4.2.0] octène-2 sous la forme d'une meringue jaune:

Rf = 0,45; chromatoplaque de gel de silice, éluant: cyclohexane-acétate d'éthyle 25—75 (en volumes).

Spectre infra-rouge ($CHBr_3$), bandes caractéristiques ($cm^{-1}$) 3400, 3270, 3220, 3180, 1790, 1730, 1700, 1545, 1510, 1495, 1455, 1370, 1230, 1165, 760, 745, 700.

Spectre de RMN du proton (350 MHz, DMSO $d_6$, $\delta$ en ppm, J en Hz)
1,42 (s, 9H, $(CH_3)_3C-$); 2,14 (s, 3H, $CH_3CO-$); 3,76 et 3,88 (AB, J = 18, 2H, $-SCH_2-$);
5,17 (d, J = 4, 1H, H en 6); 5,59 (dd, J = 4 et 9, 1H, H en 7); 6,81 (s, 1H, $-CHAr_2$);
7 à 7,4 (m, 11H, $H_4$ thiazole et aromatiques); 8,04 (d, J = 9, 1H, $-CONH-C_7$);
12,05 (s, 1H, $-N\underline{H}-COCH_3$).

4,6 g d'(acétamido-2 thiazolyl-5)-3 benzhydryloxycarbonyl-2 t.butoxycarbonylamino-7 oxo-8 thia-5 aza-1 bicyclo[4.2.0] octène-2 sont dissous dans 190 $cm^3$ d'acétonitrile à 35°C et traités par 2,9 g d'acide p.toluènesulfonique (monohydrate) en solution dans 20 $cm^3$ d'acétonitrile pendant 3 heures à 35°C, 16 heures à 25°C puis encore 6 heures à 35°C après addition de 0,95 g d'acide p.toluènesulfonique (monohydrate). Le mélange réactionnel est concentré partiellement sous pression réduite (30 mm de mercure; 4 kPa) à 30°C puis dilué par 200 $cm^3$ de chlorure de méthylène et 200 $cm^3$ de solution saturée de bicarbonate de sodium. La couche aqueuse est extraite par 100 $cm^3$ de chlorure de méthylène et les solutions organiques rassemblées sont lavées par 200 $cm^3$ d'eau puis séchées et concentrées sous pression réduite (30 mm de mercure; 4 kPa) à 30°C. Le résidu est séché sous pression réduite (10 mm de mercure; 1,3 kPa) à 25°C. On obtient 3,3 g d'(acétamido-2 thiazolyl-5)-3 amino-7 benzhydryloxycarbonyl-2 oxo-8 thia-5 aza-1 bicyclo[4.2.0] octène-2 brut sous la forme d'une poudre beige.

Spectre infra-rouge ($CHBr_3$), bandes caractéristiques ($cm^{-1}$) 3440, 3400, 3340, 3260, 3160, 1780, 1720, 1695, 1670, 1560, 1515, 1495, 1450, 1370, 1220, 760.

Spectre de RMN du proton (250 MHz, $CDCl_3$, $\delta$ en ppm, J en Hz)
2,15 (s, 3H, $-CO-CH_3$); 3,57 et 3,74 (2d, J = 18, 2H, $-S-CH_2$); 4,84 (d, J = 4,5, 1H, —H en 7);
5,05 (d, J = 4,5, 1H, —H en 6); 6,92 (s, 1H, $-COO-C\underline{H}(C_6H_5)_2$); 7,03 (s, 1H, —H thiazole);
7,05 à 7,50 (mt, aromatiques); 11,45 (mf, 1H, $-NH-\overline{CO}-$).

A une solution de 2,27 g d'(acétamido-2 thiazolyl-5)-3 amino-7 benzhydryloxycarbonyl-2 oxo-8 thia-5 aza-1 bicyclo[4.2.0] octène-2 dans 27,5 $cm^3$ de tétrahydrofuranne sec à 2°C on ajoute en 5 minutes une solution de 0,55 $cm^3$ de chlorure de (thiényl-2) acétyle dans 5 $cm^3$ de tétrahydrofuranne sec puis, après 30 minutes, une solution de 0,63 $cm^3$ de triéthylamine dans 5 $cm^3$ de tétrahydrofuranne sec. Après 1 heure de réaction à 2°C le mélange réactionnel est filtré et le filtrat est dilué dans un mélange de 200 $cm^3$ d'acétate d'éthyle et 200 $cm^3$ de solution demi-saturée de bicarbonate de sodium. La couche organique est lavée par 100 $cm^3$ d'eau puis 100 $cm^3$ de solution saturée de chlorure de sodium, séchée sur sulfate de magnésium et concentrée à sec sous pression réduite (30 mm de mercure; 4 kPa) à 40°C. Le résidu est chromatographié sur une colonne (hauteur: 30 cm; diamètre:

5 cm) de silice (0,04—0,06 mm) en éluant sous 0,4 bar (40 kPa) par une mélange de cyclohexane et d'acétate d'éthyle 30—70 (en volumes) et en recueillant des fractions de 50 $cm^3$. Les fractions 15 à 22 contenant le produit pur sont rassemblées et concentrées sous pression réduite (30 mm de mercure; 4 kPa) à 40°C. On obtient 0,92 g d'(acétamido-2 thiazolyl-5)-3 benzhydryloxycarbonyl-2 oxo-8 (thiényl-2 acétamido)-7 thia-5 aza-1 bicyclo[4.2.0] octène-2 sous la forme d'une poudre jaune.

Spectre infra-rouge (KBr), bandes caractéristiques ($cm^{-1}$) 3280, 3200, 2500, 1785, 1770, 1725, 1695, 1670, 1650, 1550, 1530, 1495, 1450, 1220, 690.

Spectre de RMN du proton (350 MHz, $CDCl_3$, $\delta$ en ppm, J en Hz)
2,09 (s, 3H, $CH_3CO-$); 3,47 (AB limite, 2H, $-SCH_2-$); 3,84 (s, 2H, $ArC\underline{H}_2CO-$); 5,04 (d, J = 4,5, 1H, H en 6); 5,88 (dd, J = 4,5 et 9, 1H, H en 7); 6,83 (s, 1H, $-OC\underline{H}Ar_2$); 6,9 à 7,0 (m, 2H, H en 3 et 4 du Thiophène); 7,13 (s, 1H, H en 4 du thiazole); 7,10 à 7,45 (m, 16H, aromatiques, H en 5 du thiophène et $-CON\underline{H}-C_7$).

Une solution de 0,85 g d'(acétamido-2 thiazolyl-5)-3 benzhydryloxycarbonyl-2 oxo-8 (thiényl-2 acétamido)-7 thia-5 aza-1 bicyclo[4.2.0] octène-2 dans 20 $cm^3$ d'acide formique est agitée pendant 30 minutes à 50°C puis concentrée à sec sous pression réduite (10 mm de mercure; 1,3 kPa) à 40°C. Le résidu est repris dans 50 $cm^3$ d'éthanol, agité pendant 5 minutes à 50°C puis le solvant est évaporé à sec sous pression réduite (30 mm de mercure; 4 kPa) à 40°C. Le résidu est repris dans 100 $cm^3$ d'éthanol; on agite pendant 10 minutes à 50°C puis essore le solide, qui est lavé par 3 fois 15 $cm^3$ d'éthanol et 3 fois 10 $cm^3$ d'éther éthylique. Après séchage, on obtient 0,53 g d'(acétamido-2 thiazolyl-5)-3 carboxy-2 oxo-8 (thiényl-2 acétamido)-7 thia-5 aza-1 bicyclo[4.2.0] octène-2 sous la forme d'une puodre jaune clair.

Spectre infra-rouge (KBr), bandes caractéristiques ($cm^{-1}$) 3260, 3200, 3100 à 2200, 1780, 1685, 1650, 1545, 1370, 700.

Spectre de RMN du proton (350 MHz, DMSO $d_6$, $\delta$ en ppm, J en Hz)
2,15 (s, 3H, $-CO-CH_3$); 3,74 et 3,80 (2d, J = 14, 2H, $-CO-CH_2-$); 3,76 et 3,88 (2d, J = 18, 2H, $-CH_2-S-$); 5,16 (d, J = 5, 1H, —H en 6); 5,72 (dd, J = 5 et 9, 1H, —H en 7); 6,90 à 7 (mt, 2H, =CH—CH= thiophène); 7,35 (dd, J = 4 et 1, 1H, =CH—S— thiophène); 7,50 (s, 1H, —H thiazole); 9,16 (d, J = 9, 1H, —CO—NH—); 13,13 (s large, 1H, $-N\underline{H}-CO-CH_3$).

## Exemple de reference 2

On acyle 3,4 g d'(acétamido-2 thiazolyl-5)-3 amino-7 benzhydryloxycarbonyl-2 oxo-8 thia-5 aza-1 bicyclo[4.2.0] octène-2 (obtenu comme décrit dans l'exemple de référence 1) par 1,95 g de chlorure de (dithiol-1,3 one-2 yl-4) acéthyle dans 75 $cm^3$ de tétrahydrofuranne sec en présence de 1,4 $cm^3$ de triéthylamine en opérant selon la mode opératoire décrit dans l'exemple de référence 1. Le produit brut est chromatographié sur une colonne (hauteur: 30 cm; diamètre: 6 cm) de gel de silice (0,04—0,06 mm) en éluant sous une pression de 0,4 bar (40 kPa) par un mélange de cyclohexane et d'acétate d'éthyle 20—80 (en volumes) en recueillant des fractions de 100 $cm^3$. Les fractions 11 à 20 sont réunies et concentrées à sec sous pression réduite (30 mm de mercure; 4 kPa) à 30°C. On obtient 1,87 g du produit attendu que l'on cristallise dans 90 $cm^3$ d'acétonitrile. On obtient 0,8 g d'(acétamido-2 thiazolyl-5)-3 benzhydryloxycarbonyl-2 (dithiol-1,3 one-2 yl-4 acétamido)-7 oxo-8 thia-5 aza-1 bicyclo[4.2.0] octène-2 sous la forme de cristaux blancs.

Spectre infra-rouge (KBr), bandes caractéristiques ($cm^{-1}$) 3280, 1780, 1730, 1695, 1640, 1545, 1225, 700.

Spectre de RMN du proton (350 MHz, DMSO $d_6$, $\delta$ en ppm, J en Hz)

2,14 (s, 3H, $-CO-CH_3$); 3,67 (s, 2H, $-CH_2-CO-N\langle$); 3,79 et 3,92 (2d, J = 18, 2H, $-CH_2-S-$);

5,23 (d, J = 4,5, 1H, —H en 6); 5,8 (dd, J = 4,5 et 9, 1H, —H en 7); 6,83 (s, 1H, $-COOC\underline{H}(C_6H_5)_2$); 7,01 (s, 1H, —S—CH=); 7,03 à 7,4 (mt, 11H, aromatiques et —H thiazole); 9,3 (d, J = 9, 1H, —CO—NH—); 12,06 (s, 1H, $-N\underline{H}-CO-CH_3$).

En opérant selon le mode opératoire décrit dans l'exemple de référence 1 mais à partir de 0,8 g d'(acétamido-2 thiazolyl-5)-3 benzhydryloxycarbonyl-2 (dithiol-1,3 one-2 yl-4 acétamido)-7 oxo-8 thia-5 aza-1 bicyclo[4.2.0] octène-2, on obtient 0,2 g d'(acétamido-2 thiazolyl-5)-3 carboxy-2 (dithiol-1,3 one-2 yl-4 acétamido)-7 oxo-8 thia-5 aza-1 bicyclo[4.2.0] octène-2 sous la forme d'un solide beige.

Spectre infra-rouge (KBr), bandes caractéristiques ($cm^{-1}$) 3270, 3100 à 2100, 1785, 1685, 1640, 1545.

Spectre de RMN du proton (350 MHz, DMSO $d_6$, $\delta$ en ppm, J en Hz)

2,14 (s, 3H, $-CO-CH_3$); 3,65 (s, 2H, $-CH_2-CO-N\langle$); 3,77 et 3,91 (2d, J = 18, 2H, $-CH_2-S-$);

5,18 (d, J = 5, 1H, —H en 6); 5,68 (dd, J = 5 et 9, 1H, —H en 7); 7,01 (s, 1H, —S—CH=);

7,52 (s, 1H, H, thiazole); 9,27 (d, J = 9, 1H, —CO—NH—);
12,15 (s large, 1H, —N$\underline{H}$—CO—$CH_3$).

Exemple de reference 3

Le produit de l'exemple 1 peut être utilisé de la manière suivante:

On agite à 20°C pendant 1 heure 20 une solution de 5,87 g de benzhydryloxycarbonyl-2 (bromo-1 oxo-2 éthyl)-3 t.butoxycarbonylamino-7 oxo-8 thia-5 aza-1 bicyclo[4.2.0] octène-2 (mélange des épimères du bromoaldéhyde) et de 1,5 g de thiobenzamide dans 60 $cm^3$ de tétrahydrofuranne sec puis on ajoute au mélange réactionnel refroidi à 2°C 0,85 $cm^3$ de chlorure de méthanesulfonyle puis en 5 minutes 5,6 $cm^3$ de triéthylamine. Après 2 heures d'agitation à 2°C le mélange réactionnel est versé dans un mélange de 100 $cm^3$ d'acétate d'éthyle et de 50 $cm^3$ d'acide chlorhydrique 1N. La phase organique est lavée par 100 $cm^3$ de solution saturée de bicarbonate de sodium puis par 100 $cm^3$ de solution saturée de chlorure de sodium, séchée sur sulfate de magnésium et concentrée à sec sous pression réduite (30 mm de mercure; 4 kPa) à 30°C. Le résidu est chromatographié sur une colonne (hauteur: 25 cm; diamètre: 5 cm) de gel de silice (0,04—0,06 mm) en éluant sous une pression de 0,4 bar (40 kPa) par un mélange de cyclohexane et d'acétate d'éthyle 80—20 (en volumes) et en recueillant des fractions de 125 $cm^3$. Les fractions 17 à 22 contenant le produit pur sont réunies et concentrées à sec sous pression réduite (30 mm de mercure; 4 kPa) à 30°C. On obtient 1 g de benzhydryloxycarbonyl-2 t.butoxycarbonylamino-7 oxo-8 (phényl-2 thiazolyl-5)-3 thia-5 aza-1 bicyclo[4.2.0] octène-2 sous la forme d'une meringue jaune.

Spectre infra-rouge ($CHBr_3$), bandes caractéristiques ($cm^{-1}$) 3420, 1790, 1720, 1595, 1495, 1450, 1500, 1420, 1390, 1370, 1235, 760, 740.

Spectre de RMN du proton (350 MHz, $CDCl_3$, $\delta$ en ppm, J en Hz)
1,48 (s, 9H, —C($CH_3$)$_3$); 3,62 et 3,78 (2d, J = 18, 2H, —$CH_2$—S—); 5,10 (d, J = 5, 1H, —H en 6);
5,29 (d, J = 9, 1H, —CO—NH—); 5,73 (dd, J = 5 et 9, 1H, —H en 7); 6,96 (s, 1H, —COOC$\underline{H}$($C_6H_5$)$_2$);
7 à 7,5 (mt, aromatiques); 7,54 (s, 1H, —H thiazole);
7,77 (mt, 2H, —H aromatiques du phényle en ortho du thiazole).

On traite 5,7 g de benzhydryloxycarbonyl-2 t.butoxycarbonylamino-7 oxo-8 (phényl-2 thiazolyl-5)-3 thia-5 aza-1 bicyclo[4.2.0] octène-2 par 57 $cm^3$ d'acide trifluoroacétique pendant 20 minutes à 20°C puis concentre le mélange réactionnel à sec sous pression réduite (0,2 mm de mercure; 0,03 kPa) à 30°C. Le résidu est repris par 100 $cm^3$ d'acétate d'éthyle que l'on évapore sous pression réduite (30 mm de mercure; 4 kPa) à 30°C. Le résidu est trituré avec 100 $cm^3$ d'oxyde d'isopropyle. Le solide est essoré, lavé par 3 fois 50 $cm^3$ d'oxyde d'isopropyle et séché sous pression réduite (0,2 mm de mercure; 0,03 kPa) à 20°C. On obtient 3,3 g d'amino-7 carboxy-2 oxo-8 (phényl-2 thiazolyl-5)-3 thia-5 aza-1 bicyclo[4.2.0] octène-2 sous la forme d'un solide ocre.

Spectre infra-rouge (KBr), bandes caractéristiques ($cm^{-1}$) 3300 à 2000, 1785, 1615, 1400, 760, 690.

Spectre de RMN du proton (350 MHz, DMSO $d_6$, $\delta$ en ppm, J en Hz)
3,82 et 3,92 (d, J = 18, 2H, —S—$Ch_2$); 4,92 (d, J = 4,5, 1H, —H en 7); 5,13 (d, J = 4,5 1H, —H en 6);
7,40 à 7,60 (mt, —H aromatiques du benzène en méta et en para du thiazole);
7,85 à 8 (mt, —H aromatiques de benzène en ortho du thiazole et —H thiazole).

Une solution de 3,3 g d'amino-7 carboxy-2 oxo-8 (phényl-2 thiazolyl-5)-3 thia-5 aza-1 bicyclo[4.2.0] octène-2 dans un mélange de 35 $cm^3$ d'eau distillée et 24 $cm^3$ d'acétone contenant 2 g de bicarbonate de sodium est refroidie à —10°C puis traitée par une solution de 0,86 $cm^3$ de chlorure de (thiényl-2) acétyle dans 10 $cm^3$ d'acétone que l'on ajoute goutte à goutte en 8 minutes. Le mélange réactionnel est agité pendant 30 minutes à —10°C puis 3 heures à 20°C. L'acétone est évaporée sous pression réduite (30 mm de mercure; 4 kPa) à 30°C et l'on ajoute 150 $cm^3$ d'acétate d'éthyle et 100 $cm^3$ de solution saturée de bicarbonate de sodium. La couche aqueuse est extraite par 100 $cm^3$ d'acétate d'éthyle puis traitée par 1 g de noir décolorant et 5 g d'adjuvant de filtration (poudre de diatomées) et filtrée sur adjuvant de filtration en rinçant par 2 fois 50 $cm^3$ d'eau distillée. Les filtrats réunis sont acidifiés à pH = 2 par de l'acide chlorhydrique 4N. Le précipité est essoré, lavé par 50 $cm^3$ d'acetate d'éthyle puis trituré avec 100 $cm^3$ d'oxyde d'isopropyle et séché. On obtient 0,8 g de carboxy-2 oxo-8 (phényl-2 thiazolyl-5)-3 (thiényl-2 acétamido)-7 thia-5 aza-1 bicyclo[4.2.0] octène-2 sous la forme d'un solide de couleur crème.

Spectre infra-rouge (KBr), bandes caractéristiques ($cm^{-1}$) 3290, 2700—2200, 1780, 1730, 1690, 1530, 1450, 690.

Spectre de RMN du proton (350 MHz, DMSO $d_6$, $\delta$ en ppm, J en Hz)

3,74 et 3,82 (2d, J = 14, 2H, —$CH_2$—CO—N$\langle$); 3,86 et 3,96 (2d, J = 18, 2H, —S—$CH_2$—);

5,23 (d, J = 4,5, 1H, —H en 6); 5,77 (dd, J = 4,5 et 9, 1H, —H en 7);
6,90 à 7 (mt, 2H, =CH—CH= du thiophène); 7,35 (dd, J = 4,5 et 1, 1H, =CH—S— du thiophène);
7,45 à 7,55 (mt, 3H, —H aromatiques de benzène en méta et en para du thiazole);

7,92 (d, J = 8, 2H, —H aromatiques de benzène en ortho du thiazole); 7,93 (s, 1H, —H du thiazole); 9,21 (d, J = 9, 1H, —CONH—).

On dissout 0,336 g de carboxy-2 oxo-8 (phényl-2 thiazolyl-5)-3 (thiényl-2 acétamido)-7 thia-5 aza-1 bicyclo [4.2.0] octène-2 dans une solution de 0,05 g de bicarbonate de sodium dans 10 $cm^3$ d'eau. Après lavage par 25 $cm^3$ d'acétate d'éthyle la phase aqueuse est filtrée puis lyophilisée. On obtient 0,32 g de sel de sodium du carboxy-2 oxo-8 (phényl-2 thiazolyl-5)-3 (thiényl-2 acétamido)-7 thia-5 aza-1 bicyclo [4.2.0] octène-2 sous la forme d'un lyophilisat beige.

Spectre infra-rouge (KBr), bandes caractéristiques ($cm^{-1}$) 3280, 1760, 1665, 1605, 1545, 1495, 1450, 1395, 690.

Spectre de RMN du proton (DMSO $d_6$, $\delta$ en ppm, J en Hz)
3,68 et 3,81 (2d, J = 16, 2H, $-SCH_2-$); 3,77 (s, 2H, $-CH_2CO-$); 5,08 (d, J = 5, 1H, H en 6); 5,54 (dd, J = 5 et 8, 1H, H en 7); 6,9 à 7 (mt, 2H, $H_3$ et $H_4$ du thiényle); 7,36 (dd, J = 1 et 5, 1H, $H_5$ du thiènyle); 7,4 à 7,5 (mt, 3H, $H_3$, $H_4$ et $H_5$ du phényle); 7,85 (d, J = 7,5, 2H, $H_2$ et $H_6$ du phényle); 7,90 (s, 1H, H en 4 du thiazole); 9,15 (d, J = 9, 1H, —CONH—).

## Exemple de reference 4

Le produit de l'exemple 1 peut être utilisé de la manière suivante:

Une solution de 2,34 g benzhydryloxycarbonyl-2 (bromo-1 oxo-2 éthyl)-3 t.butoxycarbonylamino-7 oxo-8 thia-5 aza-1bicyclo [4.2.0] octène-2(mélange des bromoaldéhydes epimères) et de 0,3 g de thioformamide dans 23 $cm^3$ de tétrahydrofuranne sec est agitée pendant 3 heures à 20°C puis chauffée à 50°C pendant 90 minutes. Le mélange réactionnel est dilué par 160 $cm^3$ d'acétate d'éthyle et lavé par 100 $cm^3$ de solution saturée de bicarbonate de sodium puis par 100 $cm^3$ de solution demi-saturée de chlorure de sodium. Après séchage de la couche organique sur sulfate de magnésium et évaporation du solvant sous pression réduite (30 mm de mercure; 4 kPa) à 30°C, on soumet le résidu obtenu à une chromatographie sur une colonne (hauteur: 30 cm; diamètre: 2,5 cm) de gel de silice (0,04—0,06 mm) en éluant sous une pression de 0,8 bar (80 kPa) par un mélange de cyclohexane et d'acétate d'ethyle 70—30 (en volumes) et en recueillant des fractions de 50 $cm^3$. Les fractions 16 à 25 contenant le produit pur sont réunies et concentrées à sec sous pression réduite (30 mm de mercure; 4 kPa) à 30°C et le résidu est trituré dans 50 $cm^3$ d'oxyde d'isopropyle. Après filtration et séchage, on obtient 0,85 g de benzhydryloxycarbonyl-2 t.butoxycarbonylamino-7 oxo-8 (thiazolyl-5)-3 thia-5 aza-1 bicyclo [4.2.0] octène-2 sous la forme d'un solide de couleur crème.

Spectre infra-rouge ($CHBr_3$), bandes caractéristiques ($cm^{-1}$) 3420, 1790, 1725, 1505, 1500, 1455, 1390, 1370, 870, 760, 740.

Spectre de RMN du proton (350 MHz, $CDCl_3$, $\delta$ en ppm, J en Hz)
1,49 (s, 9H, $-C(CH_3)_3$); 3,56 et 3,74 (2d, J = 18, 2H, $-S-CH_2-$); 5,09 (d, J = 4,5, 1H, —H en 6); 5,32 (d, J = 9, 1H, —CO—NH—); 5,72 (dd, J = 9 et 4,5, 1H, —H en 7); 6,91 (s, 1H, $-COOC\underline{H}(C_6H_5)_2$); 7 à 7,4 (mt, 10H, aromatiques); 7,58 (s, 1H, H, du thiazole); 8,58 (s, 1H, $H_2$ du thiazole).

En operant selon la mode opératoire décrit dans l'exemple de référence 1 mais à partir de 3,85 g de benzhydryloxycarbonyl-2 t.butoxycarbonylamino-7 oxo-8(thiazolyl-5)-3 thia-5 aza-1 bicyclo [4.2.0] octène-2, on prépare le dérivé amino-7 par action de 3,8 $cm^3$ d'acide méthanesulfonique dans 38 $cm^3$ d'acétonitrile puis on acyle l'amino-7 benzhydryloxycarbonyl-2 oxo-8(thiazolyl-5)-3 thia-5 aza-1 bicyclo [4.2.0] octène-2 brut obtenu par 0,86 $cm^3$ de chlorure de (thiényl-2) acétyle dans 65 $cm^3$ de tétrahydrofuranne sec en présence de 0,98 $cm^3$ de triéthylamine. Après chromatographie sur une colonne (hauteur: 30 cm; diamètre = 4 cm) de gel de silice (0,04—0,06 mm) en éluant sous une pression de 0,4 bar (40 kPa) par un mélange de cyclohexane et d'acétate d'éthyle 30—70 (en volumes), et concentration à sec sous pression réduite des fractions 11 a 20 (de 40 $cm^3$ chacune) contenant le produit pur, on obtient 2,67 g de benzhydryloxycarbonyl-2 oxo-8 (thiazolyl-5)-3 (thiényl-2 acétamido)-7 thia-5 aza-1 bicyclo [4.2.0] octène-2 sous la forme d'une meringue de couleur crème.

Spectre infra-rouge ($CHCl_3$), bandes caractéristiques ($cm^{-1}$) 3400, 1790, 1730, 1690, 1505, 695.

Spectre de RMN du proton (350 MHz, $CDCl_3$, $\delta$ en ppm, J en Hz)

3,51 et 3,71 (2d, J = 18, 2H, $-S-CH_2$); 3,88 (s, 2H, $-CH_2CO-N\langle$);

5,09 (d, J = 5, 1H, —H en 6); 5,94 (dd, J = 5 et 9, 1H, —H en 7); 6,43 (d, J = 9, 1H, —CO—NH—); 6,90 (, 1H, $-COO-C\underline{H}(C_6H_5)_2$); 6,98 à 7,05 (mt, $H_3$ et $H_4$ du thiophène); 7,05 à 7,45 (mt, 11H, aromatiques et $H_5$ du thiophène); 7,55 (s, 1H, $H_4$ du thiazole); 8,59 (s, 1H, $H_2$ du thiazole).

En opérant selon le mode opératoire décrit dans l'exemple de référence 1 mais à partir de 2,55 g de benzhydryloxycarbonyl-2 oxo-8 (thiazolyl-5)-3 (thiényl-2 acétamido)-7 thia-5 aza-1 bicyclo [4.2.0] octène-2, on obtient 1,03 g de carboxy-2 oxo-8 (thiazolyl-5)-3 (thiényl-2 acétamido)-7 thia-5 aza-1 bicyclo [4.2.0] octène-2 sous la forme d'un solide beige.

Spectre infra-rouge (KBr), bandes caractéristiques ($cm^{-1}$) 3260, 3150–2200, 1780, 1655, 1540, 1220, 700.

Spectre de RMN du proton (350 MHz, DMSO $d_6$, $\delta$ en ppm, J en Hz)

3,80 et 3,87 (2 AB limite, 4H, $-CH_2-CO-N<$ et $-CH_2-S-$); 5,20 (d, J = 4,5, 1H, –H en 6);

5,76 (dd, J = 9 et 4,5, 1H, –H en 7); 6,90 à 7 (mt, 2H, $H_3$ et $H_4$ du thiophène);
7,35 (dd, J = 5 et 1, 1H, H en 5 du thiophène); 7,91 (s, 1H, $H_4$ du thiazole);
9,09 (s, 1H, H en 2 du thiazole); 9,19 (d, J = 9, 1H, –CO–NH–); 13,65 (mf, 1H, –COOH).

## Exemple de reference 5

Selon le mode opératoire décrit dans l'exemple de référence 3 on traite 5,2 g de benzhydryloxycarbonyl-2 t.butoxycarbonylamino-7 oxo-8 (thiazolyl-5)-3 thia-5 aza-1 bicyclo[4.2.0] octène-2 (obtenu comme décrit dans l'exemple de référence 4) par 80 $cm^3$ d'acide trifluoroacétique. On obtient 4 g de trifluoroacétate d'amino-7 carboxy-2 oxo-8 (thiazolyl-5)-3 thia-5 aza-1 bicyclo[4.2.0] octène-2 sous la forme d'un solide de couleur crème.

Spectre infra-rouge (KBr), bandes caractéristiques ($cm^{-1}$) 3300–2200, 1785, 1680, 1620, 1205, 1180, 1140, 800 725.

Dans une suspension refroidie à −10°C de 2,53 g de (D)N-t.butoxycarbonyl (hydroxy-4 phényl) glycine et de 1,33 $cm^3$ de triéthylamine dans 40 $cm^3$ de tétrahydrofuranne on ajoute 1,3 $cm^3$ de chloroformiate d'isobutyle puis on agite le mélange réactionnel à une température comprise entre −10 et −15°C pendant 1 heure avant d'ajouter en 10 minutes une solution de 3,8 g de trifluoroacétate d'amino-7 carboxy-2 oxo-8 (thiazolyl-5)-3 thia-5 aza-1 bicyclo[4.2.0] octène-2 et de 4 $cm^3$ de triéthylamine dans un mélange de 20 $cm^3$ de tétrahydrofuranne et 20 $cm^3$ d'eau distillée. Le mélange réactionnel est agité pendant 1 heure à température comprise entre 0 et 5°C puis 2 heures à 20°C. Après évaporation du tétrahydrofuranne sous pression réduite (30 mm de mercure; 4 kPa) à 30°C on ajoute 50 $cm^3$ de solution saturée de bicarbonate de sodium et lave la phase aqueuse par 100 $cm^3$ d'acétate d'éthyle avant de l'acidifier à pH 2 par de l'acide chlorhydrique 4N, puis de l'extraire par 2 fois 100 $cm^3$ d'acétate de éthyle. La phase organique est lavée par 100 $cm^3$ d'eau distillée et par 100 $cm^3$ de solution saturée de chlorure de sodium puis séchée et concentrée à sec sous pression réduite (30 mm de mercure; 4 kPa) à 30°C. On obtient 1,2 g de [(D)$\alpha$-t.butoxycarbonylamino (hydroxy-4 phényl) acétamido]-7 carboxy-2 oxo-8 (thiazolyl-5)-3 thia-5 aza-1 bicyclo[4.2.0] octène-2 brut sous la forme d'une meringue jaune.

[Rf = 0,35; chromatoplaque de gel de silice, éluant: mélange acétate d'éthyle, acétone, eau, acide formique (50–10–5–5 en volumes)]

que l'on purifie par conversion en son ester de benzhydryle de la manière suivante:

On redissout le produit brut dans 25 $cm^3$ d'acétonitrile pour l'estérifier par 0,4 g de diphényldiazométhane pendant 1 heure à 20°C; après concentration à 10 $cm^3$ sous pression réduite 30 mm de mercure; 4 kPa) à 30°C on reprend dans 100 $cm^3$ d'acétate d'éthyle et lave par 50 $cm^3$ d'acide chlorhydrique 4N puis par 50 $cm^3$ de solution saturée de bicarbonate de sodium et 2 fois par 50 $cm^3$ de solution saturée de chlorure de sodium. Après séchage sur sulfate de magnésium on concentre à sec sous pression réduite (30 mm de mercure; 4 kPa) à 30°C puis on chromatographie sur une colonne (hauteur: 20 cm, diamètre: 2 cm) de gel de silice (0,04–0,06 mm) en éluant sous une pression de 0,4 bar (40 kPa) par une mélange de cyclohexane et d'acétate d'éthyle (50–50 en volumes) et en recueillant des fractions de 50 $cm^3$. Les fractions 11 à 24 contenant le produit pur sont réunies et concentrées à sec sous pression réduite (30 mm de mercure; 4 kPa) à 30°C. On obtient 0,16 g de benzhydryloxycarbonyl-2 [(D)$\alpha$-t.butoxycarbonylamino (hydroxy-4 phényl) acétamido]-7 oxo-8 (thiazolyl-5)-3 thia-5 aza-1 bicyclo[4.2.0] octène-2 sous la forme d'un solide jaune amorphe.

Spectre infra-rouge ($CHBr_3$), bandes caractéristiques ($cm^{-1}$) 3560, 3400, 3330, 1785, 1720, 1690, 1610, 1595, 1490, 1450, 1390, 1365, 1220, 755.

Spectre de RMN du proton (350 MHz, $CDCl_3$, $\delta$ en ppm, J en Hz)

1,45 (s, 9H, $-C(CH_3)_3$); 3,43 et 3,63 (2d, J = 18, 2H, $-S-CH_2$); 5,03 (d, J = 5, 1H, –H en 6);

5,13 $\left(\text{mf, 1H, } Ar-\underset{\underset{N<}{|}}{CH}-CON<\right)$; 5,60 (mf, 1H, –NH–COO–);

5,87 (dd, J = 5 et 9 et mf, 2H, –H en 7 et –OH); 6,62 (d, J = 9, 1H, –CO–NH–);
6,77 (d, J = 8, 2H, –H aromatiques en ortho de –OH); 6,90 (s, 1H, $-COO-\underline{CH}(C_6H_5)_2$);
7 à 7,4 (mt, aromatiques);
7,52 (s, 1H, =CH–N = du thiazole); 8,56 (s, 1H, –N=CH–S– du thiazole).

Selon le mode opératoire décrit ci-après à l'exemple de référence 10, on traite 0,16 g de benzhydryloxycarbonyl-2 [(D)$\alpha$-t.butoxycarbonylamino (hydroxy-4 phényl) acétamido]-7 oxo-8 (thiazolyl-5)-3 thia-5 aza-1 bicyclo[4.2.0] octène-2 par 5 $cm^3$ d'acide trifluoroacétique; on obtient 0,082 g de

trifluoroacétate de [(D)$\alpha$-amino (hydroxy-4 phényl) acétamido]-7 carboxy-2 oxo-8 (thiazolyl-5)-3 thia-5 aza-1 bicyclo [4.2.0] octène-2 sous la forme d'un solide de couleur crème.

Spectre infra-rouge (KBr), bandes caractéristiques ($cm^{-1}$) 3300—2200, 1775, 1675, 1610, 1515, 1200, 1135, 840, 800, 720.

Spectre de RMN du proton (350 MHz, DMSO $d_6$, $\delta$ en ppm, J en Hz)

3,73 et 3,82 (2d, J = 18, 2H, $-CH_2-S-$); 4,90 (s, 1H, 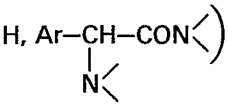);

5,15 (s, J = 5, 1H, —H en 6); 5,84 (dd, J = 5 et 9, 1H, —H en 7);
6,80 (s, J = 8, 2H, —H aromatiques en ortho du —OH);
7,30 (d, J = 8, 2H, —H aromatiques en méta de —OH); 7,88 (s, 1H, =CH—N= du thiazole);
8,60 (mf, 3H, $-NH_3^{\oplus}$); 9,06 (s, 1H, —N=CH—S—); 9,53 (d, J = 9, 1H, —CO—NH—);
9,80 (mf, 1H, —OH); 13,25 (mf très étalé, —COOH).

En opérant selon le mode opératoire de l'exemple de référence 10 à partir de 3,9 g de trifluoroacétate de [(D)$\alpha$-amino (hydroxy-4 phényl) acétamido]-7 carboxy-2 oxo-8 (thiazolyl-5)-3 thia-5 aza-1 bicyclo [4.2.0] octène-2 et par traitement par 25 $cm^3$ de résine Amberlite IR-45 (sous forme $OH^-$) dans 100 $cm^3$ d'eau distillée puis filtration et lyophilisation on obtient 1,6 g de [(D)$\alpha$-amino (hydroxy-4 phenyl) acétamido]-7 carboxy-2 oxo-8 (thiazolyl-5)-3 thia-5 aza-1 bicyclo [4.2.0] octène-2 (sel interne) sous forme d'un lyophilisat blanc.

Spectre infra-rouge (KBr), bandes caractéristiques ($cm^{-1}$) 3300—2200, 1765, 1690, 1610, 1520, 1390, 1250, 840.

Le spectre de RMN du proton ($CF_3CO_2D$) est identique à celui du trifluoroacétate pris dans les mêmes conditions.

## Exemple de reference 6

Le produit de l'exemple 2 peut être utilisé de la manière suivante:

A une solution de 10,3 g de benzhydryloxycarbonyl-2 t.butoxycarbonylamino-7 (chloro-1 oxo-2 éthyl)-3 oxo-8 thia-5 aza-1 bicyclo [4.2.0] octène-2 (mèlange des épimères du chloraldéhyde) dans un mélange de 100 $cm^3$ de tétrahydrofuranne et de 40 $cm^3$ d'éthanol on ajoute 3,34 g de N-phényl-thiourée puis on chauffe à la température de reflux du mélange réactionnel pendant 135 minutes. On dilue par 250 $cm^3$ d'acétate d'éthyle et 500 $cm^3$ de solution demi-saturée de bicarbonate de sodium. La couche organique est lavée par 250 $cm^3$ de solution demisaturée de chlorure de sodium puis séchée sur sulfate de magnésium et concentrée à sec sous pression réduite (30 mm de mercure; 4 kPa) à 30°C. Le résidu obtenu est chromatographié sur une colonne (hauteur: 30 cm; diamètre: 7 cm) de gel de silice (0,04—0,06 mm) en éluant sous une pression de 0,7 bar (70 kPa) par und mélange de cyclohexane et d'acétate d'éthyle 70—30 (en volumes). Après avoir recueilli 1,5 litre d'éluat on recueille des fractions de 100 $cm^3$ , réunit les fractions 20 et 21 et concentre à sec sous pression réduite (30 mm de mercure; 4 kPa) à 30°C. Le résidu est trituré avec 30 $cm^3$ d'oxyde d'isopropyle. On obtient 2,7 g d'(anilino-2 thiazolyl-5)-3 benzhydryloxycarbonyl-2 t.butoxycarbonylamino-7 oxo-8 thia-5 aza-1 bicyclo [4.2.0] octène-2 sous la forme d'un solide jaune.

Spectre infra-rouge ($CHBr_3$), bandes caractéristiques ($cm^{-1}$) 3420, 1785, 1720, 1600, 1540, 1500, 1495, 1455, 1390, 1370, 750.

Spectre de RMN du proton (350 MHz, $CDCl_3$, $\delta$ en ppm, J en Hz)
1,47 (s, 9H, $-C(CH_3)_3$); 3,52 et 3,67 (2d, J = 18, 2H, $-S-CH_2-$); 5,03 (d, J = 4,5, 1H, —H en 6); 5,41 (d, J = 9, 1H, —CO—NH —); 5,66 (dd, J = 4,5 et 9, 1H, —H en 7); 6,95 (s, 1H, $-COO-\underline{C}H(C_6H_5)_2$); 7,05 (s, 1H, —H thiazole); 7,05 à 7,4 (mt, 15H, aromatiques); 8,91 (s, large, 1H, —NH—).

On traite 2,3 g d'(anilino-2 thiazolyl-5)-3 benzhydryloxycarbonyl-2 t.butoxycarbonylamino-7 oxo-8 thia-5 aza-1 bicyclo [4.2.0] octène-2 par 2,3 $cm^3$ d'acide méthanesulfonique dans 23 $cm^3$ d'acétonitrile selon le mode opératoire décrit dans l'exemple de référence. On obtient 1,85 g d'amino-7 (anilino-2 thiazolyl-5)-3 benzhydryloxycarbonyl-2 oxo-8 thia-5 aza-1 bicyclo [4.2.0] octène-2 brut que l'on acyle par 0,445 $cm^3$ de chlorure de (thiényl-2) acétyle dans 25 $cm^3$ de tétrahydrofuranne en présence de 0,505 $cm^3$ de triéthylamine en opérant selon le mode opératoire décrit dans l'exemple de référence 1. Le produit brut obtenu est chromatographié sur une colonne (hauteur: 30 cm; diamètre: 2,5 cm) de gel de silice (0,04—0,06 mm) en éluant sous une pression de 1,2 bar 120 kPa) par 3 litres de mélange de cyclohexane et d'acétate d'éthyle 70—30 (en volumes) en recueillant des fractions de 50 $cm^3$. Les fractions 20 à 50 sont réunies et concentrées à sec. Après trituration avec de l'éther isopropylique on obtient 0,9 g d'(anilino-2 thiazolyl-5)-3 benzhydryloxycarbonyl-2 oxo-8 (thiényl-2 acétamido)-7 thia-5 aza-1 bicyclo [4.2.0] octène-2 sous la forme d'un solide jaune.

Spectre infra-rouge (KBr), bandes caractéristiques ($cm^{-1}$) 3380, 1785, 1720, 1675, 1600, 1530, 1500, 1455, 750, 700, 620, 605.

Spectre de RMN du proton (350 MHz, $CDCl_3$, $\delta$ en ppm, J en Hz)

3,51 (AB limite, 2 H, $-S-CH_2-$); 3,84 (s, 2 H, $-CH_2-CO-N\langle$); 5,04 (d, J = 4,5 1 H, $-H$ en 6);

5,84 (dd, J = 4,5 et 9, 1 H, $-H$ en 7); 6,92 (s, 1 H, $-COO-C\underline{H}(C_6H_5)_2$);
6,95 à 7,03 (mt, 2 H, $=CH-CH=$ du thiophène);
7,05 à 7,40 (mt, 18 H, aromatiques, $-H$ thiazole, $=CH-S-$ du thiophène et $-CO-NH-$);
8,65 (mf, 1 H, $-NH-$).

On traite 0,9 g d'(anilino-2 thiazolyl-5)-3 benzhydryloxycarbonyl-2 oxo-8 (thiényl-2 acétamido)-7 thia-5 aza-1 bicyclo[4.2.0] octène-2 par 9 $cm^3$ d'acide formique selon le mode opératoire décrit dans l'exemple de référence 1 et obtient 0,4 g d'(anilino-2 thiazolyl-5)-3 carboxy-2 oxo-8 (thiényl-2 acétamido)-7 thia-5 aza-1 bicyclo[4.2.0] octène-2 sous la forme d'un solide jaune.

Spectre infra-rouge (KBr), bandes caractéristiques ($cm^{-1}$) 3400, 3280, 3150—2000, 1770, 1665, 1625, 1605, 1530, 1500, 1455, 1400, 750, 700.

Spectre de RMN du proton (350 MHz, DMSO $d_6$, $\delta$ en ppm, J en Hz)

3,79 (AB limite, 2 H, $-CH_2-CO-N\langle$); 3,76 et 3,87 (2d, J = 18, 2 H, $-S-CH_2$);

5,16 (d, J = 5, 1 H, $-H$ en 6); 5,68 (dd, J = 5 et 9, 1 H, $-H$ en 7);
6,85 à 7 (mt, 2 H, $=CH-CH=$ du thiophène);
7,20 à 7,4 (mt, 5 H, $-H$ aromatiques en méta et para de l'anilino, $=CH-S-$ du thiophène et $-H$ du thiazole);
7,58 (d, J = 7,5, 2 H, $-H$ aromatiques en ortho de l'anilion); 9,15 (d, J = 9, 1 H, $-CO-NH-$);
10,27 (s, large, 1 H, $-NH-$).

## Exemple de reference 7

Le produit de l'exemple 5 peut être mis en oeuvre de la manière suivante:

Au mélange réactionnel de l'exemple 5, maintenu à −20°C, on ajoute en 10 minutes une solution de 1,23 g de thiourée dans un mélange de 8 $cm^3$ tétrahydrofuranne et 1,6 $cm^3$ d'eau, puis on laisse remonter la température à 20°C en agitant pendant 30 minutes. Le mélange est alors transféré dans une ampoule à décanter contenant 300 $cm^3$ d'acétate d'éthyle, lavé par 250 $cm^3$ d'une solution demi-saturée de bicarbonate de sodium, 250 $cm^3$ d'eau et 250 $cm^3$ de solution saturée de chlorure de sodium. La phase organique est alors séchée sur sulfate de sodium anhydre; on filtre et le filtrat est concentré à sec sous pression réduite (30 mm de mercure; 4 kPa) à 40°C. Le résidu (6,48 g) est chromatographié sur gel de silice (0,04—0,06 mm) en éluant sous 40 kPa par une mélange cyclohexane-acétate d'éthyle 30—70 (en volumes) et en recueillant des fractions de 60 $cm^3$. Les fractions 12 à 21 contenant le produit sont réunies et l'éluant est évaporé sous pression réduite (30 mm de mercure; 4 kPa) à 40°C. On obtient 0,65 g d'(amino-2 thiazolyl-5)-3 benzhydryloxycarbonyl-2 oxo-8 tritylamino-7 oxa-5 aza-1 bicyclo[4.2.0] octène-2 sous la forme d'une meringue marron clair.

Spectre infra-rouge (KBr), bandes caractéristiques ($cm^{-1}$) 1790, 1725, 1490, 1220, 745, 700.

Spectre de RMN du proton (350 MHz, $CDCl_3$, $\delta$ en ppm, J en Hz)
3,12 (d, J = 10, 1 H, $-N\underline{H}-C(C_6H_5)_3$); 3,85 (d, J = 3,5 1 H, $-H$ en 6);
4,05 et 4,50 (2d, J = 18, 2 H, $-CH_2-O-$); 4,39 (dd, J = 3,5 et 10, 1 H, $-H$ en 7);
4,88 (mf, 2 H, $-NH_2$); 6,91 (s, $COOC\underline{H}(C_6H_5)_2$, $-H$ du thiazole); 7,10 à 7,65 (mt, aromatiques).

A une solution refroidie à 0°C de 0,80 g d'(amino-2 thiazolyl-5)-3 benzhydryloxycarbonyl-2 oxo-8 tritylamino-7 oxa-5 aza-1 bicyclo[4.2.0] octène-2 dans 10 $cm^3$ de tétrahydrofuranne sec, on ajoute en 3 minutes une solution de 0,11 g de chlorure d'acétyle dans 2 $cm^3$ de tétrahydrofuranne sec, puis une solution de 0,18 $cm^3$ de triéthylamine dans 2 $cm^3$ de tétrahydrofuranne sec. Le mélange est agité pendant 30 minutes à 0°C, puis filtré sur poudre de diatomées. Le filtrat est dilué par 50 $cm^3$ d'acétate d'éthyle et lavé par 50 $cm^3$ d'eau distillée, 50 $cm^3$ d'une solution demi-saturée de bicarbonate de sodium et 500 $cm^3$ de solution saturée de chlorure de sodium. La phase organique est séchée sur sulfate de sodium anhydre; on filtre, lave l'insoluble par 10 $cm^3$ d'acétate d'éthyle, et la solution organique est concentrée sous pression redùite (30 mm de mercure; 4 kPa). Le résidu (0,89 g) est chromatographié sur une colonne de silice (0,04—0,06 mm) (hauteur: 24 cm; diamètre: 2,2 cm) en éluant par un mélange cyclohexane-acétate d'éthyle 40—60 (en volumes) sous 0,4 kPa et en recueillant des fractions de 60 $cm^3$. Les fractions 5 à 11 sont réunies et le solvant est évaporé sous pression réduite (30 mm de mercure; 4 kPa) à 40°C. On obtient 0,55 g d'acétylamino-2 thiazolyl-5)-3 benzhydryloxycarbonyl-2 oxo-8 tritylamino-7 oxo-5 aza-1 bicyclo[4.2.0] octène-2 sous la forme d'une meringue marron clair.

Spectre infra-rouge (KBr), bandes caractéristiques ($cm^{-1}$) 3340, 1790, 1725, 1700, 1540, 1210.

Spectre de RMN du proton (350 MHz, $CDCl_3$, $\delta$ en ppm, J en Hz)

2,16 (s, 3 H, $\rangle N-CO-CH_3$); 3,14 (d, J = 10, 1 H, $-N\underline{H}-C(C_6H_5)_3$); 3,89 (d, J = 3,5, 1 H, $-H$ en 6);

4,05 et 4,48 (d, J = 18, 2H, $-CH_2-O$); 4,43 (dd, J = 10 et 3,5, 1H, –H en 7); 6,86 (s, 1H, $-COOC\underline{H}(C_6H_5)_2$; 7,10 (s, 1H, –H du thiazole); 7,10 à 7,65 (mt, aromatiques); 10,93 (mf, 1H, $-N\underline{H}-CO-CH_3$).

On porte au reflux pendant 3 heures une solution de 0,54 g d'(acétylamino-2 thiazolyl-5)-3 benzhydryloxycarbonyl-2 oxo-8 tritylamino-7 oxo-5 aza-1 bicyclo[4.2.0] octène-2 et de 0,14 g d'acide paratoluènesulfonique monohydraté dans 5 $cm^3$ d'acétone. Le mélange réactionnel est dilué par 10 $cm^3$ d'acétate d'éthyle. La solution organique est lavée par 10 $cm^3$ d'une solution demi-saturée de bicarbonate de sodium, puis 10 $cm^3$ de solution saturée de chlorure de sodium, puis séchée sur sulfate de sodium anhydre. On filtre, lave l'insoluble par 2 fois 5 $cm^3$ d'acétate d'éthyle et la solution organique est concentrée à sec sous pression réduite (3 mm de mercure; 0,4 kPa) à 40°C. Le résidu (0,58 g) est une meringue marron contenant principalement l'amino-7 (acétylamino-2 thiazolyl-5)-3 benzhydryloxycarbonyl-2 oxo-8 oxa-5 aza-1 bicyclo[4.2.0] octène-2 [Rf = 0,11 (chromatoplaque de gel de silice; éluant cyclohexane-acétate d'éthyle 20—80)]. A une solution refroide à 0°C de 0,58 g du produit précédent, dissous dans 5 $cm^3$ de tétrahydrofuranne sec, on ajoute goutte à goutte pendant 2 minutes 0,117 g de chlorure de thiényl-2 acétyle en solution dans 1 $cm^3$ de tétrahydrofuranne. La solution marron obtenu est laissée sous agitation à 0°C pendant 5 minutes, puis on ajoute goutte à goutte 0,073 g de triéthylamine en solution dans 1 $cm^3$ de tétrahydrofuranne sec. On laisse sous agitation pendant 1 heure 15 minutes à 0°C. Le mélange est alors filtré et le filtrat est lavé par 25 $cm^3$ d'une solution demi-saturée de bicarbonate de sodium, 25 $cm^3$ d'eau distillée et 25 $cm^3$ de solution saturée de chlorure de sodium. La phase organique est séchée sur sulfate de sodium anhydre; on filtre, puis lave l'insoluble par 2 fois 5 $cm^3$ d'acétate d'éthyle. La solution organique est concentrée à sec sous pression déduite (30 mm de mercure; 4 kPa) à 40°C et le résidu est chromatographié sur gel de silice (0,2—0,06 mm) (diamètre de la colonne: 1,4 cm; hauteur de silice: 15 cm) en éluant par des mélanges cyclohexane-acétate d'ethyle 50—50 (500 $cm^3$), 40—60 (200 $cm^3$, 30—70 (200 $cm^3$) et en recueillant des fractions de 15 $cm^3$. Les fractions 40 à 49 sont réunies et le solvant est évaporé sous pression réduite. On obtient 0,1 g d'acétylamino-2 thiazolyl-5)-3 benzhydryloxycarbonyl-2 oxo-8 (thiényl-2) acétamido-7 oxa-5 aza-1 bicyclo[4.2.0] octène-2 sous la forme d'une poudre marron.

Spectre de RMN du proton (350 MHz, $CDCl_3$, $\delta$ en ppm, J en Hz)

2,10 (s, 3H, $>N-CO-CH_3$); 3,90 (AB limite, $-CH_2-CO-N<$); 3,80 et 4,20 (mt, $-CH_2-O-$);

5,04 (d, J = 3,5, 1H, –H en 6); 5,94 (dd, J = 3,5 et 10, 1H, –H en 7); 6,73 (s, 1H, $-COO-C\underline{H}(C_6H_5)_2$); 6,8 à 6,95 (mt, =CH–CH= du thiophène); 6,9 à 7,6 (mt, aromatiques, –H du thiazole et –S–CH= du thiophène); 7,77 (mf, 1H, –CO–NH–); 13,15 (mf, 1H, $-N\underline{H}-CO-CH_3$).

Une solution de 0,1 g d'(acétylamino-2 thiazolyl-5)-3 benzhydryloxycarbonyl-2 oxo-8 (thiényl-2) acétamido-7 oxa-5 aza-1 bicyclo[4.2.0] octène-2 dans 5 $cm^3$ d'acide formique est chauffée à 50°C avec agitation pendant 30 minutes. Le mélange est concentré à sec sous pression réduite (30 mm de mercure; 4 kPa) à 50°C et le résidu est dissous dans 5 $cm^3$ d'éthanol. La suspension obtenu est agitée à 50°C pendant 5 minutes et le solvant évaporé sous pression réduite (30 mm de mercure; 4 kPa) à 50°C; cette opération est répétée 1 fois et le résidu ainsi obtenu est repris dans 2 $cm^3$ d'éthanol. Le solide en suspension donne, après filtration, 12 mg d'(acétylamino-2 thiazolyl-5)-3 carboxy-2 oxo-8 (thiényl-2) acétamido-7 oxa-5 aza-1 bicyclo[4.2.0] octène-2 sous la forme d'une poudre beige.

Spectre infra-rouge (KBr), bandes caractéristiques ($cm^{-1}$) 3280, 3100, 2220, 1790, 1690, 1655, 1540, 1515, 700.

Spectre de RMN du proton (350 MHz, DMSO $d_6$, $\delta$ en ppm, J en Hz)

2,14 (s, 3H, $>N-CO-CH_3$); 3,76 (s, 2H, $-CH_2-CO-N<$);

4,53 et 4,93 (2d, J = 18, 2H, $-CH_2-O-$); 5,23 (d, J = 3,5, 1H, –H en 6); 5,58 (dd, J = 3,5et 9,5, 1H, –H en 7); 6,85 à 7 (mt, 2H, =CH–CH= du thiophène); 7,35 (dd, =CH–S–du thiophène); 7,61 (s, 1H, –H du thiazole); 8,97 (d, J = 9,5, 1H, –CO–NH–).

## Exemple de reference 8

Le produit obtenu à l'exemple 9 peut être utilisé de la manière suivante:

A une solution refroidie à 0°C de 10 g d'(amino-2 thiazolyl-5)-3 benzhydryloxycarbonyl-2 t.butoxycarbonylamino-7 oxo-8 thia-5 aza-1 bicyclo[4.2.0] octène-2 dans 150 $cm^3$ de tétrahydrofuranne sec on ajoute goutte à goutte, en 5 minutes, une solution de 1,51 $cm^3$ de chlorure d'acéthyle dans 10 $cm^3$ de tétrahydrofuranne sec puis une solution de 2,8 $cm^3$ de triéthylamine dans 5 $cm^3$ de tétrahydrofuranne sec. Le mélange réactionnel est agité pendant 1 heure 30 minutes vers 0—5°C puis traite de nouveau par 1,51 $cm^3$ de chlorure d'acétyle dans 10 $cm^3$ de tétrahydrofuranne. Après 1 heure

50 à 5°C le mélange réactionnel est filtré, le filtrat concentré à 50 $cm^3$ et dilué par 250 $cm^3$ d'acétate d'éthyle. La phase organique est lavée par 100 $cm^3$ de solution saturée bicarbonate de sodium, 100 $cm^3$ d'eau distillée et 100 $cm^3$ de solution demi-saturée de chlorure de sodium puis séchée sur sulfate de magnésium et concentrée à sec. On obtient 10 g d'(acétylamino-2 thiazolyl-5)-3 benzhydryloxycarbonyl-2 t.butoxycarbonylamino-7 oxo-8 thia-5 aza-1 bicyclo[4.2.0] octène-2 brut sous la forme d'une meringue brune. Par cristallisation dans 25 $cm^3$ d'acétonitrile on obtient 5,9 g d'(acétylamino-2 thiazolyl-5)-3 benzhydryloxycarbonyl-2 t.butoxycarbonylamino-7 oxo-8 thia-5 aza-1 bicyclo[4.2.0] octène-2 pur, sous la forme d'une poudre cristalline blanchâtre dont les caractéristiques sont identiques à celles du produit obtenu dans l'exemple de référence 1.

Une solution de 6,06 g d'(acétamido-2 thiazolyl-5)-3 benzhydryloxycarbonyl-2 t.butoxycarbonylamino-7 oxo-8 thia-5 aza-1 bicyclo[4.2.0] octène-2 dans 100 $cm^3$ d'acétonitrile est agitée avec 6 $cm^3$ d'acide méthanesulfonique pendant 30 minutes à 20°C puids diluée par 200 $cm^3$ d'acétate d'éthyle et agitée avec 300 $cm^3$ de solution saturée de bicarbonate de sodium. La couche aqueuse est extraite par 100 $cm^3$ d'acétate d'éthyle et les solutions organiques jointes sont lavées par 2 fois 250 $cm^3$ de solution demi-saturée de chlorure de sodium puis séchées sur sulfate de magnésium et concentrées à sec sous pression réduite (30 mm de mercure; 4 kPa) à 40°C. On obtient 4,9 g d'(acétamido-2 thiazolyl-5)-3 amino-7 benzhydryloxycarbonyl-2 oxo-8 thia-5 aza-1 bicyclo[4.2.0] octène-2 sous la forme d'une meringue jaune. Ce produit est redissous dans 75 $cm^3$ de tétrahydrofuranne sec. La solution refroidie vers 0°C est traitée successivement par 1,54 g de chlorure de phénylacétyle et par 1,4 $cm^3$ de triéthylamine puis agitée pendant 1 heure entre 0 et 4°C. Le mélange réactionnel est dilué par 100 $cm^3$ d'acétate d'éthyle et 200 $cm^3$ de solution demi-saturée de bicarbonate de sodium. La couche organique est décantée, lavée par 100 $cm^3$ d'eau puis par 100 $cm^3$ de solution demi-saturée de chlorure de sodium, séchée sur sulfate de magnésium et concentrée à sec sous pression réduite (30 mm de mercure; 4 kPa) à 40°C. Le résidu est repris par 25 $cm^3$ de chlorure de méthylène. Le précipité est essoré puis repris par 80 $cm^3$ d'acétonitrile bouillant. Après refroidissement le précipité est essoré, lavè par 100 $cm^3$ d'acétonitrile et séché sous pression réduite (2 mm de mercure; 0,27 kPa) à 25°C. On obtient ainsi 2,65 g d'(acétamido-2 thiazolyl-5)-3 benzhydryloxycarbonyl-2 oxo-8 phénylacétamido-7 thia-5 aza-1 bicyclo[4.2.0] octène-2 sous la forme d'un solide blanc.

Spectre infra-rouge (KBr), bandes caractéristiques ($cm^{-1}$) 3410, 3370, 3170, 1780, 1730, 1720, 1700, 1680, 1655, 1540, 1525, 1515, 1495, 1455, 1230, 760, 740, 700.

Spectre de RMN du proton (350 MHz, $CDCl_3$-DMSO $d_6$, $\delta$ en ppm, J en Hz)

2,25 (s, 3H, $>N-CO-CH_3$); 3,57 et 3,68 (2d, J = 14, 2H, $-CH_2CO\ N<$);

3,63 et 3,75 (2d, J = 18, 2H, $-CH_2-S-$); 5,14 (d, J = 4,5, 1H, —H en 6);
5,84 (dd, J = 4,5 et 9, 1H, —H en 7); 6,88 (s, 1H, $-COO-\underline{CH}(C_6H_5)_2$);
7,12 (s, 1H, —H du thiazole); 7 à 7,5 (mt, 15H, aromatiques); 9,08 (d, J = 9, 1H, —CO—NH—);
11,95 (s large, 1H, $-\underline{NH}-CO-CH_3$).

De façon similaire à l'exemple de référence 1 on traite 2,65 g d'(acétamido-2 thiazolyl-5)-3 benzhydryloxycarbonyl-2 oxo-8 phénylacétamido-7 thia-5 aza-1 bicyclo[4.2.0] octène-2 et obtient 1,7 g de l'acide correspondant, qui est purifie de la façon suivante: on dissout le produit dans une solution à 5% de bicarbonate de sodium. La solution aqueuse est lavée par de l'acétate d'éthyle puis acidifiée à pH = 4 par de l'acide chlorhydrique 1N. Le précipité est essoré, lavé par 10 $cm^3$ d'eau, 10 $cm^3$ d'éthanol et 10 $cm^3$ d'éther éthylique et séché. On obtient ainsi 1,55 g d'(acétamido-2 thiazolyl-5)-3 carboxy-2 oxo-8 phénylacétamido-7 thia-5 aza-1 bicyclo[4.2.0] octène-2 sous la forme d'un solide blanc.

Spectre infra-rouge (KBr), bandes caractéristiques ($cm^{-1}$) 3280, 3200, 3100—2300, 1785, 1710, 1695, 1650, 1540, 1520, 1495, 1450.

Spectre de RMN du proton (350 MHz, DMSO $d_6$, $\delta$ en ppm, J en Hz)

2,18 (s, 3H, $>N-CO-CH_3$); 3,53 et 3,63 (2d, J = 14, 2H, $-CH_2-CO-N<$);

3,78 et 3,93 (2d, J = 18, 2H, $-CH_2-S-$); 5,17 (d, J = 4,5, 1H, —H en 6);
5,74 (dd, J = 4,5 et 8, 1H, —H en 7); 7,15 à 7,45 (mt, 5H, aromatiques);
7,52 (s, 1H, —H du thiazole); 9,17 (d, J = 9, 1H, —CO—NH—); 12,19 (s large, 1H, $-NH-CO-CH_3$);
13,54 (mf, 1H, —COOH).

## Exemple de reference 9

Le produit obtenu à l'exemple 9 peut être utilisé de la manière suivante:

En traitant 4.5 g d'(amino-2 thiazolyl-5)-3 benzhydryloxycarbonyl-2 t.butoxycarbonylamino-7 oxo-8 thia-5 aza-1 bicyclo[4.2.0] octène-2 par 1,11 $cm^3$ de chlorure de benzoyle dans 55 $cm^3$ de tétrahydrofuranne sec en présence de 1,23 $cm^3$ de triéthylamine selon le mode opératoire de l'exemple de référence 8, on obtient 1,8 g de (benzamido-2 thiazolyl-5)-3 benzhydryloxycarbonyl-2 t.butoxy-

carbonylamino-7 oxo-8 thia-5 aza-1 bicyclo[4.2.0] octène-2 sous la forme de cristaux de couleur crème.

Spectre infra-rouge (KBr), bandes caractéristiques ($cm^{-1}$) 3420, 3240, 1790, 1730, 1705, 1675, 1600, 1580, 1545, 1510, 1500, 1455, 1370, 1160, 760, 700.

Spectre de RMN du proton (350 MHz, DMSO $d_6$, $\delta$ en ppm, J en Hz)
1,45 (s, 9H, $-C(CH_3)_3$); 3,83 et 3,97 (2d, J = 18, 2H, $-CH_2-S-$); 5,24 (d, J = 5, 1H, −H en 6); 5,67 (dd, J = 5 et 9, 1H, −H en 7); 6,91 (s, 1H, $-COO-C\underline{H}(C_6H_5)_2$);
7,05 à 7,45 (mt, 10H, aromatiques); 7,49 (s, 1H, −H thiazole);
7,63 (t, J = 7,5, 2H, aromatiques en méta du benzamido);
7,72 (t, J = 7,5, 1H, aromatiques en para du benzamido); 8,13 (d, J = 9, 1H, −CO−NH−);
8,16 (d, J = 7,5, 2H, aromatiques en ortho du benzamido); 12,72 (s, 1H, $-N\underline{H}-CO-C_6H_5$).

Une solution de 2,9 g de (benzamido-2 thiazolyl-5)-3 benzhydryloxycarbonyl-2 t.butoxycarbonylamino-7 oxo-8 thia-5 aza-1 bicyclo[4.2.0] octène-2 dans 29 $cm^3$ d'acétonitrile est traitée par 2,9 $cm^3$ d'acide méthanesulfonique selon le mode opératoire de l'exemple de référence 8. On obtient 2 g d'amino-7 (benzamido-2 thiazolyl-5)-3 benzhydryloxycarbonyl-2 oxo-8 thia-5 aza-1 bicyclo[4.2.0] octène-2 brut sous la forme d'une meringue jaune.

Rf = 0,22 (chromatoplaque de gel de silice, éluant: mélange de cyclohexane et d'acétate d'éthyle 30—70 en volumes).

Ce produit est repris dans 50 $cm^3$ de tétrahydrofuranne sec et acylé par 0,53 $cm^3$ de chlorure de (thiényl-2)acétyle en présence de 0,6 $cm^3$ de triéthylamine en opérant selon le mode opératoire décrit dans l'exemple de référence 8. Le produit brut est chromatographié sur une colonne (hauteur: 22 cm; diamètre: 4,4 cm) de gel de silice (0,04—0,06 mm) en éluant sous une pression de 0,4 bar (40 kPa) par un mélange de cyclohexane et d'acétate d'éthyle (50—50 en volumes) et en recueillant des fractions de 125 $cm^3$. Les fractions 7 à 16 contenant le produit pur sont concentrées à sec sous pression réduite (30 mm de mercure; 4 kPa) à 30°C. On obtient 1,5 g de (benzamido-2 thiazolyl-5)-3 benzhydryloxycarbonyl-2 oxo-8 (thiényl-2 acétamido)-7 thia-5 aza-1 bicyclo[4.2.0] octène-2 sous la forme d'une meringue jaune.

Spectre infra-rouge ($CHBr_3$), bandes caractéristiques ($cm^{-1}$) 3400, 3300—2500, 1785, 1725, 1675, 1600, 1580, 1535, 1505, 1490, 1450, 755, 740.

Spectre de RMN du proton (350 MHz, DMSO $d_6$, $\delta$ en ppm, J en Hz)

3,74 et 3,81 (2d, J = 14, 2H, $-CH_2-CO-N\langle$); 3,81 et 3,95 (2d, J = 18, 2H, $-CH_2-S-$);

5,24 (d, J = 5, 1H, −H en 6); 5,82 (dd, J = 5 et 8, −H en 7); 6,84 (s, 1H, $-COO-C\underline{H}(C_6H_5)_2$);
6,90 à 7 (mt, 2H, =CH—CH= du thiophène); 7 à 7,4 (mt, 10H, aromatiques);
7,35 (dd, J = 4 et 1, 1H, =CH—S— du thiophène); 7,42 (s, 1H, −H thiazole);
7,55 (t, J = 7,5, 2H, aromatiques en méta du benzamido);
7,66 (t, J = 7,5, 1H, aromatique en para de benzamido);
8,08 (d, J = 7,5, 2H, aromatiques en ortho du benzamido); 9,23 (d, J = 8, 1H, −CO−NH−);
12,60 (s, 1H, $-N\underline{H}CO-C_6H_5$).

Une solution de 1,5 g de (benzamido-2 thiazolyl-5)-3 benzhydryloxycarbonyl-2 oxo-8 (thiényl-2 acétamido)-7 thia-5 aza-1 bicyclo[4.2.0] octène-2 dans un mélange de 43 $cm^3$ d'acide formique et 8 $cm^3$ d'eau distillée est chauffée pendant 30 minutes à 60°C puis diluée par 35 $cm^3$ d'eau distillée. Le précipité est essoré, lavé par 2 fois 10 $cm^3$ d'acide formique à 50% (en volumes) puis par 3 fois 75 $cm^3$ d'oxyde d'isopropyle et séché; on obtient 1 g de (benzamido-2 thiazolyl-5)-3 carboxy-2 oxo-8 (thienyl-2 acétamido)-7 thia-5 aza-1 bicyclo[4.2.0] octène-2 sous la forme d'un solide blanc.

Spectre infra-rouge (KBr), bandes caractéristiques ($cm^{-1}$) 3400, 3260, 3100—2100, 1785, 1665, 1605, 1585, 1550, 1495, 1450, 705.

Spectre de RMN du proton (350 MHz, DMSO $d_6$, $\delta$ en ppm, J en Hz)

3,73 et 3,82 (2d, J = 14, 2H, $-CH_2-CO-N\langle$); 3,80 et 3,95 (2d, J = 18, 2H, $-CH_2-S-$);

5,20 (d, J = 5, 1H, −H en 6); 5,75 (dd, J = 5 et 9, −H en 7);
6,9 à 7 (mt, 2H, =CH—CH= du thiophène); 7,35 (dd, J = 4 et 1, 1H, =CH—S— du thiophène);
7,53 (t, J = 7,5, 2H, aromatiques en méta du benzamido); 7,63 (s, 1H, −H thiazole);
7,64 (t, J = 7,5, 1H, aromatique en para du benzamido);
8,10 (d, J = 7,5, 2H, aromatiques en ortho du benzamido); 9,18 (d, J = 9, 1H, −CO−NH−);
11,60 (mf, 1H, −COOH); 12,45 (mf, 1H, $-N\underline{H}-CO-C_6H_5$).

Exemple de reference 10

Le produit de l'exemple 10 peut être utilisé de la manière suivante:

A une solution refroidie à 4°C de 10 g d'(amino-2 thiazolyl-5)-3 benzhydryloxycarbonyl-2 [(D)$\alpha$-t.butoxycarbonylamino phénylacétamido]-7 oxo 8 thia-5 aza-1 bicyclo[4.2.0] octène-2 dans 140 $cm^3$

de tétrahydrofuranne sec on ajoute en 5 minutes une solution de 1,22 $cm^3$ de chlorure d'acétyle dans 10 $cm^3$ de tétrahydrofuranne sec puis après 5 minutes une solution de 2,15 $cm^3$ de triéthylamine dans 5 $cm^3$ de tétrahydrofuranne. Le mélange réactionnel est agité 4 heures vers 5°C puis filtré, concentré partiellement, puis versé dans un mèlange de 200 $cm^3$ d'acétate d'éthyle et 200 $cm^3$ de solution demi-saturée de bicarbonate de sodium; la phase organique est lavée 2 fois par 100 $cm^3$ d'eau et par 100 $cm^3$ de solution saturée de chlorure de sodium puis séchée sur sulfate de magnesium. Après évaporation du solvant sous pression réduite (30 mm de mercure; 4 kPa) à 40°C, on cristallise le résidu dans 80 $cm^3$ d'acétonitrile pour obtenir 2,6 g d'(acétylamino-2 thiazolyl-5)-3 benzhydryloxycarbonyl-2 [(D) $\alpha$-t.butoxycarbonylamino-2 phénylacétamido]-7 oxo-8 thia-5 aza-1 bicyclo[4.2.0] octène-2 sous la forme d'une poudre cristalline beige clair.

Spectre infra-rouge ($CHBr_3$), bandes caractéristiques ($cm^{-1}$) 3400, 3260, 3160, 1780, 1715, 1690, 1560, 1500, 1490, 1450, 1390, 1370, 760, 740.

Spectre de RMN du proton (350 MHz, DMSO $d_6$, $\delta$ en ppm, J en Hz)
1,4 (s, 9H, $-C(CH_3)_3$); 2,13 (s, 3H, $-CO-CH_3$); 3,67 et 3,81 (2d, J = 18, 2H, $-S-CH_2-$);
5,12 (d, J = 5, 1H, —H en 6); 5,35 (d, J = 8, 1H, >N—CH—CO—N<);
5,54 (d, J = 8, 0,5H, —NH—CO—O— en partie échangée); 5,83 (dd, J = 5 et 9, 1H, —H en 7);
6,82 (s, 1H, $-COO-\underline{CH}(C_6H_5)_2$); 7 à 7,5 (mt, 16H, aromatiques et —H du thiazole);
9,26 (d, J = 9, 1H, —CO—NH—); 12,04 (s, 1H, $-\underline{NH}-CO-CH_3$).

2,25 g d'(acétylamino-2 thiazolyl-5)-3 benzhydryloxycarbonyl-2 [(D) $\alpha$-t.butoxycarbonylamino-phénylacétamido]-7 oxo-8 thia-5 aza-1 bicyclo[4.2.0] octène-2 sont dissous dans 20 $cm^3$ d'acide trifluoroácetique. La solution est agitée à 25°C pendant 20 minutes puis concentré à sec sous pression réduite (5 mm de mercure; 0,7 kPa) à 40°C. Le résidu est repris par 30 $cm^3$ d'oxyde d'isopropyle. On évapore à sec de nouveau sous pression réduite (30 mm de mercure; 4 kPa) à 40°C. Cette opération est répétée 4 fois puis le résidu est trituré avec 100 $cm^3$ d'éther éthylique puis essoré, lavé par 3 fois 30 $cm^3$ d'éther éthylique puis séché. On obtient 1,45 g de trifluoroacétate d'(acétylamino-2 thiazolyl-5)-3 [(D) $\alpha$-aminophénylacétamido]-7 carboxy-2 oxo-8 thia-5 aza-1 bicyclo[4.2.0] octène-2 sous la forme d'une poudre beige.

Spectre infra-rouge (KBr), bandes caractéristiques ($cm^{-1}$) 3180, 3100, 2150, 1775, 1680, 1455, 1200, 800, 720, 700.

Spectre de RMN du proton (350 MHz, DMSO $d_6$, $\delta$ en ppm, J en Hz)
2,13 (s, 3H, $-CO-CH_3$); 3,66 et 3,78 (2d, J = 18, 2H, $-S-CH_2-$);
5,01 (s, 1H, >N—CH—CO—N<); 5,11 (d, J = 4,5, 1H, —H en 6); 5,81 (mf, 1H, —H en 7);
7,35 à 7,65 (mt, 6H, aromatiques et —H thiazole); 8 à 10 (mf, étalé, 1H, —COOH);
9,58 (d, J = 8, 1H, —CO—NH—); 12,14 (mf, 1H, $-\underline{NH}-CO-CH_3$).

On redissout 1,2 g de ce trifluoroacétate dans 100 $cm^3$ d'eau et la solution est lavée par 3 fois 20 $cm^3$ d'acétate d'éthyle puis décantée, traitée par 0,5 g de noir décolorant, filtrée et agitée avec 20 $cm^3$ de résine Amberlite IR 45 ($OH^-$) (préalablement lavée à l'eau à jusqu'à neutralité) jusqu'à ce que le pH de la solution aqueuse atteigne 5,5. Après élimination de la résine par filtration, la solution aqueuse est lyophilisée. On obtient 0,58 g d'(acétylamino-2 thiazolyl-5)-3 [(D) $\alpha$-aminophénylacétamido]-7 carboxy-2 oxo-8 thia-5 aza-1 bicyclo[4.2.0] octène-2 sous la forme d'un lyophilisat blanc.

Spectre infra-rouge (KBr), bandes caractéristiques ($cm^{-1}$) 3240, 3100—2300, 1765, 1685, 1600, 1545, 1510, 1455, 1370, 720, 700.

Spectre de RMN ($CF_3CO_2H$) conforme à celui du trifluoroacétate.

## Exemple de reference 11

Le produit de l'exemple 6 peut être utilisé de la manière suivante:

Selon le mode opératoire de l'exemple de référence 5 on traite 2,6 g de benzhydryloxycarbonyl-2 (bromo-1 oxo-2 éthyl)-3 diéthoxyphosphorylamino-7 oxo-8 thia-5 aza-1 bicyclo[4.2.0] octène-2 (mélange des bromoaldéhydes isomères) par 0,31 g de thioformamide dans 20 $cm^3$ de tétrahydrofuranne sec et l'on chromatographie le produit obtenu sur une colonne (hauteur: 25 cm; diamètre: 2,5 cm) de gel de silice (0,04—0,06 mm) en éluant sous une pression de 0,5 bar par de l'acétate d'éthyle et en recueillant des fractions de 20 $cm^3$. Les fractions 6 à 22 contenant le produit pur sont réunies et évaporées à sec sous pression réduite (30 mm de mercure; 4 kPa) à 30°C. On obtient 1,6 g de benzhydryloxycarbonyl-2 diéthoxyphosphorylamino-7 oxo-8 (thiazolyl-5)-3 thia-5 aza-1 bicyclo[4.2.0] octène-2 sous la forme d'une meringue jaune.

Spectre infra-rouge ($CHBr_3$), bandes caractéristiques ($cm^{-1}$) 3390, 1790, 1730, 1240, 1020, 975, 760, 740.

Spectre de RMN du proton (350 MHz, $CDCl_3$, $\delta$ en ppm, J en Hz)
1,36 et 1,38 (2T, J = 7, 6H, $-CH_3$); 3,56 et 3,75 (2D, J = 18, 2H, $-CH_2-S-$);
3,59 (T, J = 11, 1H, $>P(=O)-NH-$); 4,10 à 4,25 (Mt, 4H, $-CH_2-O-$);
5,06 (D, J = 5, 1H, −H en 6); 5,22 (DT, J = 5 et 11, 1H, −H en 7);
6,89 (s, 1H, $-COOC\underline{H}(C_6H_5)_2$); 7 à 7,4 (Mt, 10H, aromatiques); 7,56 (s, 1H, =CH—N= du thiazole);
8,57 (s, 1H, —S—CH=N— du thiazole).

Un mélange de 1,5 g de benzhydryloxycarbonyl-2 diéthoxyphosphorylamino-7 oxo-8 (thiazolyl-5)-3 thia-5 aza-1 bicyclo[4.2.0] octène-2 et de 5 $cm^3$ d'acide phosphotique à 85% (poids) est agité pendant 16 heures à 20°C puis dilué avec 30 $cm^3$ d'eau distillée à 0°C et filtré. Le solide est lavé par 3 fois 3 $cm^3$ d'eau distillée. Au filtrat (auquel on a joint les lavages) refroidi à 0°C, on ajoute 20,9 g de bicarbonate de sodium puis 40 $cm^3$ d'acétone. Après refroidissement à 0°C on ajoute une solution de 1,2 $cm^3$ de chlorure de thiényl-2 acétyle dans 10 $cm^3$ d'acétone puis agite le mélange réactionnel pendant 3 heures à 0°C, puis 16 heures à 20°C. On dilue par 200 $cm^3$ d'eau distillée et 150 $cm^3$ d'acétate d'éthyle et décante la phase aqueuse, qui est lavée par 3 fois 40 $cm^3$ d'acétate d'éthyle puis refroidie à 0°C et acidifiée à pH = 2 en présence de 100 $cm^3$ d'acétate d'éthyle par addition d'acide chlorhydrique 10N. La phase aqueuse est extraite par 2 fois 50 $cm^3$ d'acétate d'éthyle. Les solutions organiques rassemblées sont lavées par 3 fois 50 $cm^3$ d'eau, séchées sur sulfate de magnésium et concentrées à sec sous pression réduite (30 mm de mercure; 4 kPa) à 30°C. Le résidu est trituré avec 50 $cm^3$ d'oxyde d'isopropyle. On obtient 0,2 g de carboxy-2 oxo-8 (thiazolyl-5)-3 (thiényl-2 acétamido)-7 thia-5 aza-1 bicyclo[4.2.0] octène-2 sous la forme d'une poudre beige dont les caractéristiques sont identiques à celles du produit obtenu dans l'exemple de référence 4.

## Exemple de reference 12

Le produit de l'exemple 7 peut être utilisé de la manière suivante:

Selon le mode opératoire de l'exemple de référence 5 on traite 1,16 g benzhydryloxycarbonyl-2 (chloro-1 oxo-2 éthyl)-3 diéthoxyphosphorylamino-7 oxo-8 thia-5 aza-1 bicyclo[4.2.0] octène-2 (mélange des chloroaldéhydes diastéréoisomères) par 0,15 g de thioformamide dans 10 $cm^3$ de tétrahydrofuranne et l'on chromatographie le produit brut obtenu sur une colonne (hauteur: 25 cm; diamètre: 2,5 cm) de gel de silice (0,04—0,06 mm) en éluant sous une pression de 0,5 bar (50 kPa) par de l'acétate d'éthyle et en recueillant des fractions de 20 $cm^3$. Les fractions 10 à 18 contenant le produit pur sont réunies et évaporées à sec sous pression réduite (30 mm de mercure; 4 kPa) à 30°C. On obtient 0,8 g de benzhydryloxycarbonyl-2 diéthoxyphosphorylamino-7 oxo-8 (thiazolyl-5)-3 thia5 aza-1 bicyclo[4.2.0] octène-2 sous la forme d'une meringue jaune dont les caractéristiques sont identiques à celles du produit obtenu dans l'exemple de référence 11.

## Exemple de reference 13

Le produit de l'exemple 12 peut être mis en oeuvre de la manière suivante:

On ajoute à une solution de 0,5 g de benzhydryloxycarbonyl-2 (bromo-1 oxo-2 éthyl)-3 (dichloro-3,4 phénylthio) acétamido-7 oxo-8 thia-5 aza-1 bicyclo[4.2.0] octène-2 (mélange des diastéréoisomères au niveau du substituant en -3) dans 10 $cm^3$ de tétrahydrofuranne, une solution de 0,054 g de thiourée dans 1 $cm^3$ d'eau distillée puis agite le mélange réactionnel pendant 17 heures à 25°C avant de le verser dans un mélange de 250 $cm^3$ d'eau distillée, 60 $cm^3$ de solution saturée de bicarbonate de sodium et 60 $cm^3$ d'acétate d'éthyle. La phase organique est lavée 3 fois par 30 $cm^3$ d'eau distillée, puis par 30 $cm^3$ de solution saturée de chlorure de sodium et séchée sur sulfate de magnesium. Le résidu obtenu après concentration à sec du solvant sous pression réduite (30 mm de mercure; 4 kPa) à 30°C est chromatographié sur une colonne (hauteur: 28 cm, diamètre: 2 cm) de gel de silice (0,04—0,063 mm) en éluant sous une pression de 0,5 bar (50 kPa) par un mélange de cyclohexane et d'acétate d'éthyle 30/70 en volumes et en recueillant des fractions de 15 $cm^3$. Les fractions 7 à 10 contenant le produit pur sont réunies et concentrées sous pression réduite (30 mm de mercure; 4 kPa) à 30°C pour donner 0,29 g d'(amino-2 thiazolyl-5)-3 benzhydryloxycarbonyl-2 (dichloro-3,4 phénylthio) acétamido-7 oxo-8 thia-5 aza-1 bicyclo[4.2.0] octène-2 sous la forme d'une meringue jaune pâle.

Spectre infra-rouge ($CHCl_3$), bandes caractéristiques ($cm^{-1}$) 3490, 3390, 1780, 1725, 1685, 1600, 1495, 1460.

Spectre de RMN du proton (350 MHz, $CDCl_3$, $\delta$ en ppm, J en Hz)
3,41 et 3,51 (2d, J = 17,5, 2H, $-SCH_2-$ du cyle); 3,60 et 3,68 (2d, J = 16, 2H, $-S-CH_2-CO-$);
5,01 (d, J = 5, 1H, H en -6); 5,37 (s large, 2H, $-NH_2$); 5,77 (dd, J = 5 et 9, 1H, H en -7);

6,85 (s, 1H, H en -4 du thiazole); 6,92 (s, 1H, $-CO_2CH\langle$); 7,10 à 7,50 (m, 13H, aromatiques); 7,73 (d, J = 9, 1H, —CONH—).

En opérant par analogie avec la méthode décrite à l'exemple de référence 10, on prépare l'(acétylamino-2 thiazolyl-5)-3 benzhydryloxycarbonyl-2 (dichloro-3,4 phénylthio) acétamido-7 oxo-8 thia-5 aza-1 bicyclo[4.2.0] octène-2, puis l'(acétylamino-2 thiazolyl-5)-3 carboxy-2 (dichloro-3,4 phénylthio) acétamido-7 oxo-8 thia-5 aza-1 bicyclo[4.2.0] octène-2.

**Revendications pour les Etats contractants: BE, CH, DE, FR, GB, IT, LI, LU, NL, SE**

1. Un dérivé de la céphalosporine de formule générale:

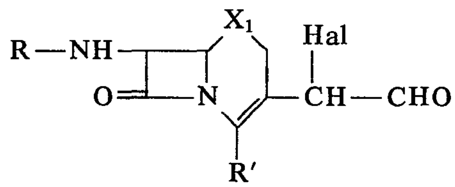

dans laquelle,

— le symbole R' représente un radical carboxy protégé
— le symbole Hal représente un atome d'halogène et

A) le symbole $X_1$ représente un atome de soufre ou d'oxygène ou un radical sulfinyle et le symbole R est un radical de formule générale:

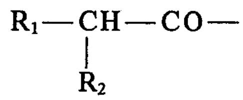

[dans laquelle $R_1$ est un radical hétérocyclyle tel que thiényle, furyle ou dithiol-1,3 one-2 yle-4, ou un radical phényle, p.hydroxyphényle libre ou protégé ou phénoxy et $R_2$ est un atome d'hydrogène, ou $R_1$ est un radical phényle ou p.hydroxyphényle libre ou protégé et $R_2$ est un radical amino protégé] ou
un radical protecteur d'amino, facilement éliminable, ou bien

B) le symbole $X_1$ représente un atome de soufre et
le symbole R représente un radical de formule générale:

$$R''CO-$$

[dans laquelle R'' est choisi parmi les radicaux alcoyle contenant 1 à 7 atomes de carbone, cycloalcoyle, aryle, aralcoyle, hétéroaryle ou hétéroaralcoyle, qui peuvent être substitués par un ou plusieurs atomes d'halogène ou radicaux alcoyle, alcoyloxy, alcoylthio, haloalcoyle, azido, cyano, amino protégé éventuellement substitué, carboxy protégé ou carboxyalcoyle protégé], ou
un radical de formule générale:

$$R''-Y-(CH_2)_m-CO-$$

[dans laquelle m est un nombre entier de 0 à 4, Y est un atome d'oxygène ou de soufre et R'' est défini comme ci-dessus], ou un radical de formule générale:

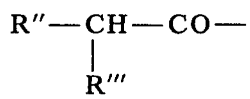

[dans laquelle R'' est défini comme ci-dessus et R''' est un atome d'halogène ou un radical hydroxy, hydroxy protégé, azido, acyloxy, amino protégé ou carboxy protégé], ou
R—NH représente un groupe sulfonamido,
étant etendu que les portions et radicaux alcoyle et acyle cités ci-dessus sont droits ou ramifiés et contiennent 1 à 4 atomes de carbone,

leurs formes isomères et leurs mélanges.

2. Un dérivé de la céphalosporine selon la revendication 1, caractérisé en ce que R' est un radical carboxy protégé choisi parmi t.butyle, méthoxyméthyle, trichloro-2,2,2 éthyle, benzhydryle, p.nitrobenzyle ou p.méthoxybenzyle.

3. Un dérivé de la céphalosporine selon la revendication 1, caractérisé en ce que R est un radical protecteur d'amino, choisi parmi

1) benzhydryle ou trityle,
2) un radical acyle de formule générale:

$R_3CO—$

dans laquelle $R_3$ représente

a) un atome d'hydrogène ou un radical alcoyle contenant 1 à 7 atomes de carbone ou méthyle substitué par 1 à 3 atomes d'halogène,
b) un radical phényle (pouvant être jusqu'à 3 fois substitué par des atomes d'halogène ou des radicaux hydroxy, nitro, cyano, trifluorométhyle, alcoyle ou alcoyloxy) ou un radical thiényle-2 ou -3,
c) un radical de formule générale:

$R'_3—Y—CH_2—$

dans laquelle $R'_3$ est un radical phényle (pouvant être substitué par un atome d'halogène ou par un radical alcoyle, alcoyloxy ou hydroxy) et Y est un atome d'oxygène ou de soufre,
d) un radical arylalcoyle de formule générale:

$R''_3CH_2—$

dans laquelle $R''_3$ est un radical phényle pouvant être jusqu'à 3 fois substitué par des radicaux hydroxy, alcoyle ou alcoyloxy, ou un radical hétérocyclyle tel que thiényle-2 ou -3 ou furyle-2 ou -3,

3) un radical amino-5 adipoyle dont les fonctions amine et acide sont protégées par des radicaux protecteurs tels que définis précédemment,
4) un radical de formule générale:

$R_4OCO—$

dans laquelle $R_4$ est un radical alcoyle ramifié non substitué, un radical alcoyle droit ou ramifié portant un ou plusieurs substituants [choisis parmi des atomes d'halogène ou des radicaux cyano, trialcoylsilyle, phényle ou phényle substitué (par un ou plusieurs atomes d'halogène ou radicaux alcoyle, alcoyloxy, nitro ou phényle)] ou un radical quinolyle,
5) un radical de formule générale:

$$\underset{\substack{|\\(O)_n}}{Ar—S—} \quad \text{ou} \quad ArSe—$$

dans lesquelles le reste Ar est un radical phényle éventuellement substitué par un ou plusieurs atomes d'halogène ou radicaux alcoyle ou nitro et n est égal à 0 ou 1,
6) ou bien R NH— est remplacé par un radical dialcoylaminométhylènamino ou par un radical de formule générale:

$Ar'—CH{=}N—$

dans laquelle Ar' est un radical phényle éventuellement substitué par un ou plusieurs radicaux alcoyle, alcoyloxy, hydroxy ou nitro,
7) ou encore R est un radical diphénylphosphinoyle ou un radical de formule générale:

$$(ZO)_2P\rightarrow O$$

dans laquelle Z est alcoyle, trichloro-2,2,2 éthyle, phényle ou benzyle, ces deux derniers étant éventuellement substitués par un atome d'halogène ou par un radical alcoyle, alcoyloxy ou

nitro, ou les symboles Z forment ensemble un radical alcoylène contenant 2 ou 3 atomes de carbone.

4. Un procédé de préparation d'un produit selon la revendication 1, pour lequel le symbole $X_1$ est autre que sulfinyle, caractérisé en ce que l'on fait agir un agent d'halogénation sur une énamine de formule généralé:

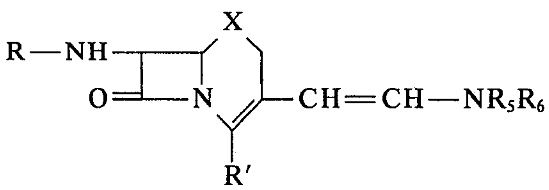

dans laquelle R et R' sont définis comme dans la revendication 1, et $R_5$ et $R_6$ identiques ou différents représentent des radicaux alcoyle (éventuellement substitués par un radical alcoyloxy ou dialcoylamino) ou phényle ou forment ensemble, avec l'atome d'azote auquel ils sont rattachés, un hétérocycle à 5 ou 6 chaînons contenant éventuellement un autre hétéroatome choisi parmi l'azote; l'oxygène ou le soufre et éventuellement substitué par un radical alcoyle, et X est un atome d'oxygène ou de soufre] puis on hydrolyse le produit formé.

5. Un procédé de préparation d'un produit selon la revendication 1 pour lequel le symbole $X_1$ est un radical sulfinyle, caractérisé en ce que l'on oxyde un produit selon la revendication 1 pour lequel le symbole $X_1$ est un atome de soufre.

**Revendication pour l'Etat contractant AT**

Procédé de préparation d'un dérivé de la céphalosporine de formule générale:

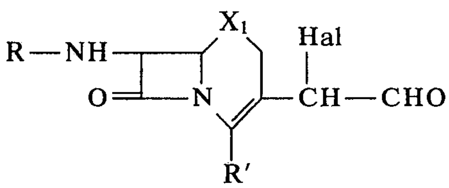

dans laquelle,

- le symbole R' représente un radical carboxy protégé
- le symbole Hal représente un atome d'halogène et

A/ le symbole $X_1$ représente un atome de soufre ou d'oxygène ou un radical sulfinyle et le symbole R est un radical de formule générale:

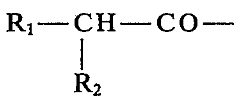

[dans laquelle $R_1$ est un radical hétérocyclyle tel que thiényle, furyle ou dithiol-1,3 one-2 yle-4, ou un radical phényle, p.hydroxyphényle libre ou protégé ou phénoxy et $R_2$ est un atome d'hydrogène, ou $R_1$ est un radical phényle ou p.hydroxyphényle libre ou protégé et $R_2$ est un radical amino protégé] ou
un radical protecteur d'amino, facilement éliminable, ou bien

B/ le symbole $X_1$ représente un atome de soufre et
le symbole R représente un radical de formule générale:

$R''CO-$

[dans laquelle R'' est choisi parmi les radicaux alcoyle contenant 1 à 7 atomes de carbone, cycloalcoyle, aryle, aralcoyle, hétéroaryle ou hétéroaralcoyle, qui peuvent être substitués par un ou plusieurs atomes d'halogène ou radicaux alcoyle, alcoyloxy, alcoylthio, haloalcoyle, azido, cyano, amino protégé éventuellement substitué, carboxy protégé ou carboxyalcoyle protégé], ou
un radical de formule générale:

$R''—Y—(CH_2)_m—CO—$

[dans laquelle m est un nombre entier de 0 à 4, Y est un atome d'oxygène ou de soufre et R'' est défini comme ci-dessus], ou un radical de formule générale:

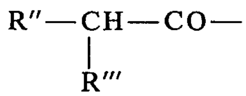

[dans laquelle R'' est défini comme ci-dessus et R''' est un atome d'halogène ou un radical hydroxy, hydroxy protégé, azido, acyloxy, amino protégé ou carboxy protégé], ou R–NH représente un groupe sulfonamido, étant entendu que les portions et radicaux alcoyle et acyle cités ci-dessus sont droits ou ramifiés et contiennent 1 à 4 atomes de carbone, leurs formes isomères et leurs mélanges, caractérisé en ce que, lorsque le symbole $X_1$ est autre que sulfinyle, on fait agir un agent d'halogénation sur une énamine de formule générale:

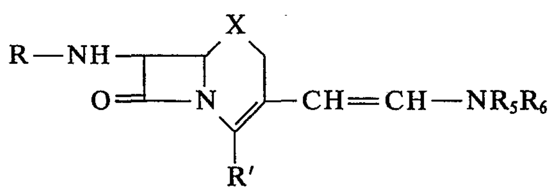

[dans laquelle R et R' sont définis comme dans la revendication 1, et $R_5$ et $R_6$ identiques ou différents représentent des radicaux alcoyle (éventuellement substitués par un radical alcoyloxy ou dialcoylamino) ou phényle ou forment ensemble, avec l'atome d'azote auquel ils sont rattachés, un hétérocycle à 5 ou 6 chaînons contenant éventuellement un autre hétéroatome choisi parmi l'azote; l'oxygène ou le soufre et éventuellement substitué par un radical alcoyle, et X est un atome d'oxygène ou de soufre] puis on hydrolyse le produit formé, ou bien lorsque le symbole $X_1$ est un radical sulfinyle, on oxyde le produit correspondant pour lequel le symbole $X_1$ est un atome de soufre, puis sépare éventuellement le produit obtenu en ses isomères.

**Patentansprüche für die Vertragsstaaten BE, CH, DE, FR, GB, IT, LI, LU, NL, SE**

1. Ein Cephalosporinderivat der allgemeinen Formel

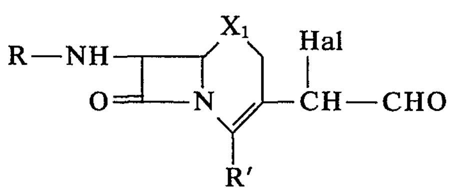

worin

– das Symbol R' einen geschützten Carboxyrest bedeutet,
– das Symbol Hal ein Halogenatom bedeutet und

A) das Symbol $X_1$ ein Schwefel- oder Sauerstoffatom oder einen Sulfinylrest bedeutet und das Symbol R ist ein Rest der allgemeinen Formel

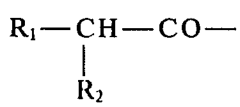

[worin $R_1$ ein Heterocyclylrest, wie Thienyl, Furyl oder 1,3-Dithiol-2-on-4-yl, oder ein Phenylrest, ein freier oder geschützter p-Hydroxyphenylrest oder ein Phenoxyrest ist und $R_2$ ist ein Wasserstoffatom, oder $R_1$ ist ein Phenyl- oder einer freier oder geschützter p-Hydroxyphenylrest und $R_2$ ist ein geschützter Aminorest] oder ein leicht entfernbarer Aminoschutzrest, oder aber

B) das Symbol $X_1$ bedeutet ein Schwefelatom und
das Symbol R bedeutet einen Rest der allgemeinen Formel

$R''CO—$

[worin R'' ausgewählt ist unter den Alkylresten mit 1 bis 7 Kohlenstoffatomen, den Cycloalkyl-, Aryl-, Aralkyl-, Heteroaryl- oder Heteroaralkylresten, die substituiert sein können durch ein oder mehrere Halogenatome oder Alkyl-, Alkyloxy-, Alkylthio-, Haloalkyl-, Azido-, Cyano-, geschützte gegebenenfalls substituierte Amino-, geschütztes Carboxy oder geschützte Carboxyalkylreste], oder
einen Rest der allgemeinen Formel

$R''—Y—(CH_2)_m—CO—$

worin m eine ganze Zahl von 0 bis 4 ist, Y ein Sauerstoff- oder Schwefelatom bedeutet und R'' wie vorstehend definiert ist], oder
einen Rest der allgemeinen Formel

$$R''—\underset{\displaystyle R'''}{\underset{|}{CH}}—CO—$$

[worin R'' wie vorstehend definiert ist und R''' ein Halogenatom oder einen Hydroxy-, geschützten Hydroxy-, Azido-, Acyloxy-, geschützten Amino- oder geschützten Carboxyrest bedeutet], oder
R—NH bedeutet eine Sulfonamidgruppe,
wobei die vorstehend erwähnten Alkyl- und Acylteile und -reste gerade oder verzweigt sind und 1 bis 4 Kohlenstoffatome enthalten,

ihre isomeren Formen und ihre Mischungen.

2. Ein Cephalosporinderivat gemäß Anspruch 1, dadurch gekennzeichnet, daß R' ein geschützter Carboxyrest ist ausgewählt unter tert.-Butyl, Methoxymethyl, 2,2,2-Trichlorethyl, Benzhydryl, p-Nitrobenzyl oder p-Methoxybenzyl.

3. Ein Cephalosporinderivat gemäß Anspruch 1, dadurch gekennzeichnet, daß R ein Aminoschutzrest ist ausgewählt unter

1) Benzhydryl oder Trityl,
2) einem Acylrest der allgemeinen Formel

$R_3CO—$

worin $R_3$ die folgenden Bedeutungen besitzt:

a) ein Wasserstoffatom oder ein Alkylrest mit 1 bis 7 Kohlenstoffatomen oder Methyl substituiert durch 1 bis 3 Halogenatome,
b) ein Phenylrest (der bis zu dreimal durch Halogenatome oder Hydroxy-, Nitro-, Cyano-, Trifluormethyl-, Alkyl- oder Alkyloxyreste substituiert sein kann) oder ein Thienyl-2- oder -3-Rest,
c) ein Rest der allgemeinen Formel

$R_3'—Y—CH_2—$

worin $R_3'$ ein Phenylrest ist (der durch ein Halogenatom oder durch einen Alkyl-, Alkyloxy- oder Hydroxyrest substituiert sein kann) und Y bedeutet ein Sauerstoff- oder Schwefelatom,
d) ein Aralkylrest der allgemeinen Formel

$R_3''CH_2—$

worin $R_3''$ ein Phenylrest ist, der bis zu dreimal durch Hydroxy-, Alkyl- oder Alkyloxyreste substituiert sein kann, oder ein Heterocyclylrest wie Thienyl-2 oder -3 oder Furyl-2 oder -3,

3) einem 5-Aminoadipoylrest, dessen Amin- und Säurefunktionen durch Schutzreste, wie sie vorstehend definiert sind, geschützt ist,
4) einem Rest der allgemeinen Formel

$R_4OCO—$

worin $R_4$ ein verzweigter nicht substituierter Alkylrest, ein gerader oder verzweigter Alkylrest mit einem oder mehreren Substituenten [ausgewählt unter den Halogenatomen oder den Cyano-, Trialkylsilyl-, Phenyl- oder (durch ein oder mehrere Halogenatome oder Alkyl-, Alkyloxy-, Nitro- oder Phenylreste) substituierten Phenylresten] oder ein Chinolylrest ist,

5) einem Rest der allgemeinen Formel

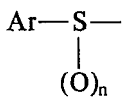 oder ArSe—

worin der Rest Ar einen Phenylrest gegebenenfalls substituiert durch ein oder mehrere Halogenatome oder Alkyl- oder Nitroreste bedeutet und n gleich 0 oder 1 ist,

6) oder auch R NH— ist ersetzt durch einen Dialkylaminomethylenaminorest oder durch einen Rest der allgemeinen Formel

Ar'—CH=N—

worin Ar' ein Phenylrest gegebenenfalls substituiert durch ein oder mehrere Alkyl-, Alkyloxy-, Hydroxy- oder Nitroreste ist,

7) oder aber R ist ein Diphenylphosphinoylrest oder ein Rest der allgemeinen Formel

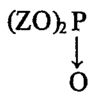

worin Z Alkyl, 2,2,2-Trichlorethyl, Phenyl oder Benzyl bedeutet, wobei die beiden letzteren gegebenenfalls substituiert sind durch ein Halogenatom oder durch einen Alkyl-, Alkyloxy- oder Nitrorest, oder die Symbole Z bilden zusammen einen Alkylenrest mit 2 bis 3 Kohlenstoffatomen.

4. Verfahren zur Herstellung eines Produkts gemäß Anspruch 1, worin das Symbol $X_1$ anders als Sulfinyl ist, dadurch gekennzeichnet, daß man ein Halogenierungsmittel auf ein Enamin der allgemeinen Formel

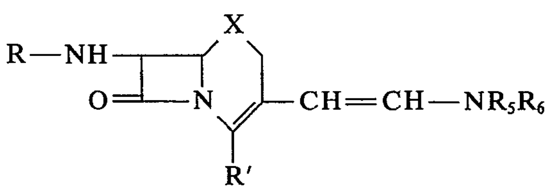

[worin R und R' wie in Anspruch 1 definiert sind und $R_5$ und $R_6$, welches identisch oder verschieden sind, Alkylreste (gegebenenfalls substituiert durch einen Alkyloxy- oder Dialkylaminorest) oder Phenylreste bedeuten oder zusammen mit dem Stickstoffatom, an das sie gebunden sind, einen Heterocyclus mit 5 oder 6 Kettengliedern bilden, enthaltend gegebenenfalls ein anderes Heteroatom ausgewählt unter dem Stickstoff, dem Sauerstoff oder dem Schwefel und gegebenenfalls substituiert durch einen Alkylrest, und X ein Sauerstoff- oder Schwefelatom ist] einwirken läßt und daß man dann das gebildete Produkt hydrolysiert.

5. Verfahren zur Herstellung eines Produkts gemäß Anspruch 1, worin das Symbol $X_1$ ein Sulfinylrest ist, dadurch gekennzeichnet, daß man ein Produkt gemäß Anspruch 1, worin das Symbol $X_1$ ein Schwefelatom ist, oxydiert.

## Patentanspruch für den Vertragsstaat: AT

Verfahren zur Herstellung eines Cephalosporinderivats der allgemeinen Formel:

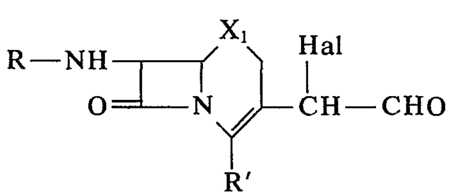

in welcher

- das Symbol R' einen geschützten Carboxyrest darstellt,
- das Symbol Hal ein Halogenatom darstellt und

A/ das Symbol $X_1$ ein Schwefel- oder Sauerstoffatom oder einen Sulfinylrest darstellt und das Symbol R ein Rest der allgemeinen Formel:

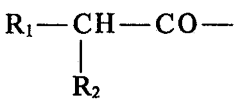

[in welcher $R_1$ ein Heterocyclylrest, wie Thienyl, Furyl oder 1,3-Dithiol-2-on-4-yl, oder ein Phenylrest, ein freier oder geschützter p-Hydroxyphenylrest oder Phenoxyrest ist und $R_2$ ein Wasserstoffatom ist oder $R_1$ ein Phenylrest oder einer freier oder geschützter p-Hydroxyphenylrest ist und $R_2$ ein geschützter Aminorest ist] oder
eine leicht entfernbare Aminoschutzgruppe ist oder

B/ das Symbol $X_1$ ein Schwefelatom darstellt und das Symbol R einen Rest der allgemeinen Formel:

$$R''CO—$$

[in welcher R'' ausgewählt ist aus den Alkylresten mit 1 bis 7 Kohlenstoffatomen, den Cycloalkyl-, Aryl-, Aralkyl-, Heteroaryl- oder Heteroaralkylresten, die durch ein oder mehrere Halogenatome oder Alkyl-, Alkyloxy-, Alkylthio-, Halogenalkyl-, Azido-, Cyano-, geschützte und gegebenenfalls substituierte Amino-, geschützte Carboxy- oder geschützte Carboxyalkylreste substituiert sein können]
oder einen Rest der allgemeinen Formel:

$$R''—Y—(CH_2)_m—CO—$$

[in welcher m eine ganze Zahl von 0 bis 4 ist, Y ein Sauerstoff- oder Schwefelatom ist und R'' die obige Bedeutung hat]
oder einen Rest der allgemeinen Formel:

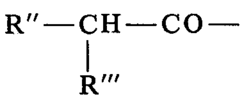

[in welcher R'' die obige Bedeutung hat und R''' ein Halogenatom oder ein Hydroxy-, geschützter Hydroxy-, Azido-, Acyloxy-, geschützter Amino- oder geschützter Carboxyrest ist] darstellt oder R—NH stellt eine Sulfoamidogruppe dar,
wobei die oben genannten Alkyl- und Acylteile und -reste selbstverständlich geradkettig oder verzweigt sind und 1 bis 4 Kohlenstoffatome enthalten, seiner isomeren Formen und ihrer Mischungen, dadurch gekennzeichnet, daß man,
wenn das Symbol $X_1$ von Sulfinyl verschieden ist, ein Halogenierungsmittel auf ein Enamin der allgemeinen Formel:

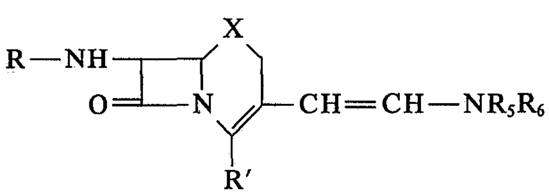

[in welcher R und R' die im Anspruch 1 angegebene Bedeutung haben und $R_5$ und $R_6$ unabhängig voneinander Alkylreste (gegebenenfalls substituiert durch einen Alkyloxy- oder Dialkylaminorest) oder Phenylreste darstellen oder zusammen mit dem Stickstoffatom, an das sie gebunden sind, einen Heterocyclus mit 5 oder 6 Gliedern bilden, der gegebenenfalls ein weiteres Heteroatom, ausgewählt aus Stickstoff, Sauerstoff oder Schwefel, enthält und gegebenenfalls substituiert ist durch einen Alkylrest, und X ein Sauerstoff- oder Schwefelatom ist] einwirken läßt und dann das gebildete Produkt hydrolysiert oder daß man, wenn das Symbol $X_1$ ein Sulfinylrest ist, die entsprechende Verbindung, worin das Symbol $X_1$ ein Schwefelatom ist, oxidiert und dann gegebenenfalls das erhaltene Produkt in seine Isomeren spaltet.

**Claims for the contracting states BE, CH, DE, FR, GB, IT, LI, LU, NL, SE**

1. A cephalosporin derivate of general formula:

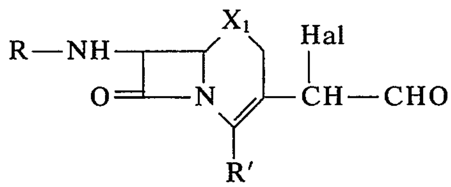

in which,

- the symbol R′ denotes a protected carboxy radical
- the symbol Hal denotes a halogen atom and

A) the symbol $X_1$ denotes a sulphur or oxygen atom or a sulphinyl radical and the symbol R is a radical of general formula:

$R_1$—CH—CO—
 |
 $R_2$

[in which $R_1$ is a heterocyclic radical such as thienyl, furyl or 1,3-dithiol-2-on-4-yl, or a phenyl, free or protected p-hydroxyphenyl or phenoxy radical and $R_2$ is a hydrogen atom, or $R_1$ is a phenyl or free or protected p-hydroxyphenyl radical and $R_2$ is a protected amino radical] or a readily removable amino-protecting radical, or alternatively

B) the symbol $X_1$ denotes a sulphur atom and the symbol R denotes a radical of general formula:

R″CO—

[in which R″ is chosen from alkyl radicals containing 1 to 7 carbon atoms and cycloalkyl, aryl, aralkyl, heteroaryl or heteroaralkyl radicals, which can be substituted with one or more halogen atoms, or alkyl, alkyloxy, alkylthio, haloalkyl, azido and cyano radicals, optionally substituted protected amino radicals, and protected carboxy or protected carboxyalkyl radicals], or a radical of general formula:

$R''—Y—(CH_2)_m—CO—$

[in which m is an integer from 0 to 4, Y is an oxygen or sulphur atom and R″ is defined as above], or a radical of general formula:

R″—CH—CO—
 |
 R‴

[in which R″ is defined as above and R‴ is a halogen atom or a hydroxy, protected hydroxy, azido, acyloxy, protected amino or protected carboxy radical], or R—NH denotes a sulphonamido group, on the understanding that the constituents and the alkyl and acyl radicals mentioned above are linear or branched and contain 1 to 4 carbon atoms,

isomeric forms thereof and mixtures thereof.

2. A cephalosporin derivate according to Claim 1, characterised in that R′ is a protected carboxy radical chosen from t-butyl, methoxymethyl, 2,2,2-trichlorethyl, benzhydryl-, p-nitrobenzyl or p-methoxybenzyl.

3. A cephalosporin derivative according to Claim 1, characterised in that R is an amino-protecting radical chosen from

1) benzhydryl or trityl,

2) an acyl radical of general formula:

$R_3CO-$

in which $R_3$ denotes

a) a hydrogen atom or an alkyl radical containing 1 to 7 carbon atoms or a methyl radical substituted with 1 to 3 halogen atoms,
b) a phenyl radical (which can be substituted up to 3 times with halogen atoms or hydroxy, nitro, cyano, trifluoromethyl, alkyl or alkyloxy radicals) or a 2- or 3-thienyl radical,
c) a radical of general formula:

$R_3'-Y-CH_2-$

in which $R_3'$ is a phenyl radical (which can be substituted with a halogen atom or with an alkyl, alkyloxy or hydroxy radical) and Y is an oxygen or sulphur atom,
d) an arylalkyl radical of general formula:

$R_3''CH_2-$

in which $R_3''$ is a phenyl radical which can be substituted up to 3 times with hydroxy, alkyl or alkyloxy radicals, or a heterocyclic radical such as 2- or 3-thienyl or 2- or 3-furyl,

3) a 5-aminoadipoyl radical in which the amine and acid functions are protected by protective radicals such as those defined above,
4) a radical of general formula:

$R_4OCO-$

in which $R_4$ is an unsubstituted branched alkyl radical, a linear or branched alkyl radical bearing one or more substituents [chosen from halogen atoms or cyano, trialkylsilyl and phenyl radicals, or phenyl radicals substituted with one or more halogen atoms or with one or more alkyl, alkyloxy, nitro or phenyl radicals] or a quinolyl radical,
5) a radical of general formula:

$Ar-\underset{(O)_n}{\underset{|}{S}}-$ or $ArSe-$

in which the Ar residue is a phenyl radical optionally substituted with one or more halogen atoms or alkyl or nitro radicals and n equals 0 or 1,
6) or alternatively R NH– is replaced by a dialkylaminomethyleneamino radical or by a radical of general formula:

$Ar'-CH{=}N-$

in which Ar′ is a phenyl radical optionally substituted with one or more alkyl, alkyloxy, hydroxy or nitro radicals.
7) or R is a diphenylphosphinoyl radical or a radical of general formula:

$(ZO)_2\underset{O}{\underset{\downarrow}{P}}$

in which Z is alkyl, 2,2,2-trichloroethyl, phenyl or benzyl, the two latter radicals being optionally substituted with a halogen atom or with an alkyl, alkyloxy or nitro radical, or the symbols Z together form an alkylene radical containing 2 or 3 carbon atoms.

4. A process for preparing a product according to Claim 1, for which the symbol $X_1$ is other than sulphinyl, characterised in that a halogenating agent is reacted with an enamine of general formula:

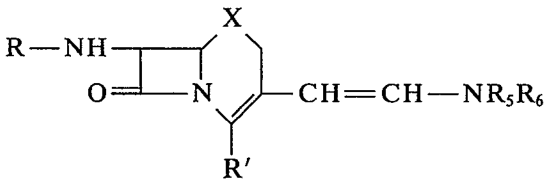

[in which R and R'are defined as in Claim 1, and $R_5$ and $R_6$, which may be identical or different, denote alkyl radicals (optionally substituted with an alkyloxy or dialkylamino radical) or phenyl radicals or together form, with the nitrogen atom to which they are attached, à 5- or 6-membered heterocycle optionally containing another hetero atom chosen from nitrogen, oxygen or sulphur, and optionally substituted with an alkyl radical, and X is an oxygen or sulphur atom] and the product formed is then hydrolysed.

5. A process for preparing a product according to Claim 1, for which the symbol $X_1$ is a sulphinyl radical, characterised in that a product according to Claim 1, for which the symbol $X_1$ is a sulphur atom, is oxidised.

**Claim for the contracting state AT**

Process for preparing a cephalosporin derivate of general formula:

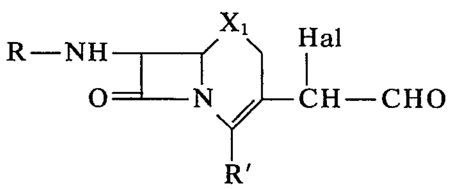

in which,

- the symbol R' denotes a protected carboxy radical
- the symbol Hal denotes a halogen atom and

A) the symbol $X_1$ denotes a sulphur or oxygen atom or a sulphinyl radical and the symbol R is a radical of general formula:

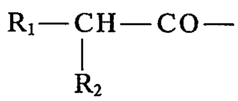

[in which $R_1$ is a heterocyclic radical such as thienyl, furyl or 1,3-dithiol-2-on-4-yl, or a phenyl, free or protected p-hydroxyphenyl or phenoxy radical and $R_2$ is a hydrogen atom, or $R_1$ is a phenyl or free or protected p-hydroxyphenyl radical and $R_2$ is a protected amino radical] or a readily removable amino-protecting radical, or alternatively

B) the symbol $X_1$ denotes a sulphur atom and the symbol R denotes a radical of general formula:

R″CO—

[in which R″ is chosen from alkyl radicals containing 1 to 7 carbon atoms and cycloalkyl, aryl, aralkyl, heteroaryl or heteroaralkyl radicals, which can be substituted with one or more halogen atoms, or alkyl, alkyloxy, alkylthio, haloalkyl, azido and cyano radicals, optionally substituted protected amino radicals, and protected carboxy or protected carboxyalkyl radicals], or a radical of general formula:

$$R''—Y—(CH_2)_m—CO—$$

[in which m is an integer from 0 to 4, Y is an oxygen or sulphur atom and R″ is defined as above], or a radical of general formula:

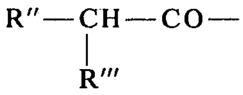

[in which R″ is defined as above and R‴ is a halogen atom or a hydroxy, protected hydroxy, azido, acyloxy, protected amino or protected carboxy radical], or
R—NH denotes a sulphonamido group,
on the understanding that the constituents and the alkyl and acyl radicals mentioned above are linear or branched and contain 1 to 4 carbon atoms, isomeric forms thereof and mixtures thereof, characterised in that,
when the symbol $X_1$ is other than sulphinyl, a halogenating agent is reacted with an enamine of general formula:

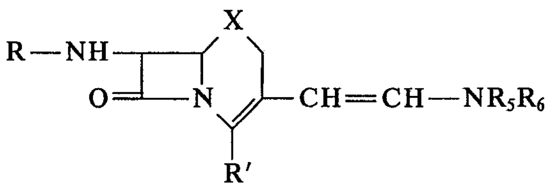

[in which R and R′ are defined as in Claim 1, and $R_5$ and $R_6$, which may be identical or different, denote alkyl radicals (optionally substituted with an alkyloxy or dialkylamino radical) or phenyl radicals or together form, with the nitrogen atom to which they are attached, a 5- or 6-membered heterocycle optionally containing another hetero atom chosen from nitrogen, oxygen or sulphur, and optionally substituted with an alkyl radical, and X is an oxygen or sulphur atom] and the product formed is then hydrolysed, or alternatively when the symbol $X_1$ is a sulphinyl radical, the corresponding product, for which the symbol $X_1$ is a sulphur atom, is oxidised, and the product obtained is then optionally separated into its isomers.